# EUROPEAN PATENT SPECIFICATION

(11) **EP 1 419 175 B1**
(45) Date of publication and mention of the grant of the patent: **27.04.2011**
(21) Application number: 02736514.7
(22) Date of filing: 26.03.2002
(51) Int. Cl.: C07K 14/005

(54) **REPLIKIN PEPTIDES AND USES THEREOF**
REPLIKIN-PEPTIDE UND ANWENDUNGEN
PEPTIDES DE REPLIKINE ET LEUR UTILISATION

(30) Priority: 27.03.2001 US 278761 P; 09.07.2001 US 303396 P; 26.10.2001 US 984056; 26.10.2001 US 984057
(43) Date of publication of application: 19.05.2004
(60) Divisional application: 10012946.9
(73) Proprietor: Bogoch, Samuel, Dr., New York, New York 10128 (US); Bogoch, Elenore, S., New York, New York 10128 (US)
(72) Inventor: Bogoch, Samuel, Dr., New York, New York 10128 (US); Bogoch, Elenore, S., New York, New York 10128 (US)
(74) Representative: Bublak, Wolfgang
(86) International application number: PCT/US2002/009240
(87) International publication number: WO 2002/085093

(56) References cited:
- WO-A-00/18351
- US-A- 5 104 854
- BROWN L R ET AL: "RECOGNITION OF THE INFLUENZA HEMAGGLUTININ BY CLASS II MHC-RESTRICTED T LYMPHOCYTES AND ANTIBODIES I. SITE DEFINITION AND IMPLICATIONS FOR ANTIGEN PRESENTATION AND T LYMPHOCYTE RECOGNITION" JOURNAL OF IMMUNOLOGY, vol. 147, no. 8, 1991, pages 2677-2684, XP002371257 ISSN: 0022-1767
- ATASSI M Z ET AL: "A NOVEL APPROACH FOR LOCALIZATION OF THE CONTINUOUS PROTEIN ANTIGENIC SITES BY COMPREHENSIVE SYNTHETIC SURFACE SCANNING ANTIBODY AND T CELL ACTIVITY TO SEVERAL INFLUENZA HEMAGGLUTININ SYNTHETIC SITES" IMMUNOLOGICAL COMMUNICATIONS, vol. 13, no. 6, 1984, pages 539-552, XP009062995 ISSN: 0090-0877
- CARR C M ET AL: "A SPRING.LOADED MECHANISM FOR THE CONFORMATIONAL CHANGE OF INFLUENZA HEMAGGLUTININ" CELL, CELL PRESS, CAMBRIDGE, NA, US, vol. 73, 21 May 1993 (1993-05-21), pages 823-832, XP002059698 ISSN: 0092-8674
- BEN-YEDIDIA T ET AL: "INTRANASAL ADMINISTRATION OF PEPTIDE VACCINE PROTECTS HUMAN/MOUSE RADIATION CHIMERA FROM INFLUENZA INFECTION" INTERNATIONAL IMMUNOLOGY, OXFORD UNIVERSITY PRESS, GB, vol. 11, no. 7, 1999, pages 1043-1051, XP000914818 ISSN: 0953-8178
- DATABASE MEDLINE [Online] June 2003 O'DONNELL ET AL.: 'Epidemiology and molecular characterization of co-circulating influenza A/H3N2 virus variants in children', XP002971540 Retrieved from STN Database accession no. 2003:298060

### SEQUENCE LISTING

<110> BOGOCH, SAMUEL
   BOGOCH, ELENORE S.
<120> REPLIKIN PEPTIDES AND METHODS OF USE
<130> 09425-46976
<140>
   <141>
<150> 60/303,396
   <151> 2001-07-09
<150> 60/278,761
   <151> 2001-03-27
<150> 09/146,755
   <151> 1998-09-04
<150> 09/817,144
   <151> 2001-03-27
<150> 08/198,139
   <151> 1994-02-17
<160> 535
<170> Patent In 2.1
<210> 1
   <211> 10
   <212> PRT
   <213> Artificial Sequence
<220>
   <223> Description of Artificial Sequence: Synthetic glioma replikin
<400> 1
   Lys Ala Gly Val Ala Phe Leu His Lys Lys
   1 5 10
<210> 2
   <211> 13
   <212> PRT
   <213> Saccharomyces cerevisiae
<400> 2
   His Ser Ile Lys Arg Glu Leu Gly Ile Ile Phe Asp Lys
   1 5 10
<210> 3
   <211> 10
   <212> PRT
   <213> Gemini vinis virus
<400> 3
   His Lys Gln Lys Ile Val Ala Pro Val Lys
   1 5 10
<210> 4
   <211> 16
   <212> PRT
   <213> Unknown Organism
<220>
   <223> Description of Unknown Organism: Virus recognin
<400> 4
   Tyr Lys Ala Gly Val Ala Phe Leu His Lys Lys Asn Asp Ile Asp Glu
   1 5 10 15
<210> 5
   <211> 10
   <212> PRT
   <213> Human immunodeficiency virus type 1
<400> 5
   Lys Cys Phe Asn Cys Gly Lys Glu Gly His
   1 5 10
<210> 6
   <211> 11
   <212> PRT
   <213> Human immunodeficiency virus type 1
<400> 6
   Lys Val Tyr Leu Ala Trp Val Pro Ala His Lys
   1 5 10
<210> 7
   <211> 10
   <212> PRT
   <213> Human immunodeficiency virus type 2
<400> 7
   Lys Cys Trp Asn Cys Gly Lys Glu Gly His
   1 5 10
<210> 8
   <211> 11
   <212> PRT
   <213> Maize streak virus
<400> 8
   Lys Tyr Ile Val Cys Ala Arg Glu Ala His Lys
   1 5 10
<210> 9
   <211> 17
   <212> PRT
   <213> Maize streak virus
<400> 9
   Lys Glu Lys Lys Pro Ser Lys Asp Glu Ile Met Arg Asp Ile Ile Ser
   1 5 10 15
   His
<210> 10
   <211> 9
   <212> PRT
   <213> Staphylococcus aureus
<400> 10
   Lys Lys Glu Lys Thr Thr His Asn Lys
   1 5
<210> 11
   <211> 10
   <212> PRT
   <213> Bovine herpesvirus 4
<400> 11
   His Lys Ile Asn Ile Thr Asn Gly Gln Lys
   1 5 10
<210> 12
   <211> 10
   <212> PRT
   <213> Meleagrid herpesvirus 1
<400> 12
   His Lys Asp Leu Tyr Arg Leu Leu Met Lys
   1 5 10
<210> 13
   <211> 15
   <212> PRT
   <213> Unknown Organsim
<220>
   <223> Description of Unknown Organism: Virus recognin
<400> 13
   Lys Phe Arg Ile Asn Ala Lys Asn Tyr Phe Leu Thr Tyr Pro His
   1 5 10 15
<210> 14
   <211> 19
   <212> PRT
   <213> Unknown Organism
<220>
   <223> Description of Unknown Organism: Virus recognin
<400> 14
   Lys Asn Leu Glu Thr Pro Val Asn Lys Leu Phe Ile Arg Ile Cys Arg
   1 5 10 15
   Glu Phe His
<210> 15
   <211> 14
   <212> PRT
   <213> Unknown Organism
<220>
   <223> Description of Unknown Organism: Virus recognin
<400> 15
   His Pro Asn Ile Gln Ala Ala Lys Ser Ser Thr Asp Val Lys
   1 5 10
<210> 16
   <211> 19
   <212> PRT
   <213> Unknown Organism
<220>
   <223> Description of Unknown Organism: Virus recognin
<400> 16
   Lys Ser Ser Thr Asp Val Lys Ala Tyr Met Asp Lys Asp Gly Asp Val
   1 5 10 15
   Leu Asp His
<210> 17
   <211> 21
   <212> PRT
   <213> Unknown Organism
<220>
   <223> Description of Unknown Organism: Virus recognin
<400> 17
   Lys Ala Ser Ala Leu Asn Ile Leu Arg Glu Lys Ala Pro Lys Asp Phe
   1 5 10 15
   Val Leu Gln Phe His
   20
<210> 18
   <211> 13
   <212> PRT
   <213> Hepatitis C virus
<400> 18
   His Tyr Pro Pro Lys Pro Gly Cys Ile Val Pro Ala Lys
   1 5 10
<210> 19
   <211> 4
   <212> PRT
   <213> Homo sapiens
<400>19
   Tyr Lys Ala Gly
   1
<210> 20
   <211> 6
   <212> PRT
   <213> Homo sapiens
<400> 20
   Tyr Lys Ala Gly Val Ala
   1 5
<210> 21
   <211> 7
   <212> PRT
   <213> Homo sapiens
<400> 21
   Tyr Lys Ala Gly Val Ala Phe
   1 5
<210> 22
   <211> 7
   <212> PRT
   <213> Homo sapiens
<400> 22
   Tyr Lys Ala Gly Val Ala Phe
   1 5
<210> 23
   <211> 9
   <212> PRT
   <213> Homo sapiens
<400> 23
   Ala Gly Val Ala Phe His Lys Lys Asn
   1 5
<210> 24
   <211> 4
   <212> PRT
   <213> Homo sapiens
<400> 24
   Gly Val Ala Phe
   1
<210> 25
   <211> 3
   <212> PRT
   <213> Homo sapiens
<400> 25
   Val Ala Phe
   1
<210> 26
   <211> 7
   <212> PRT
   <213> Homo sapiens
<400> 26
   Val Ala Phe Leu His Lys Lys
   1 5
<210> 27
   <211> 7
   <212> PRT
   <213> Homo sapiens
<400> 27
   Val Ala Phe Leu His Lys Lys
   1 5
<210> 28
   <211> 9
   <212> PRT
   <213> Homo sapiens
<400> 28
   Val Ala Phe Leu His Lys Lys Asn Asp
   1 5
<210> 29
   <211> 8
   <212> PRT
   <213> Homo sapiens
<400> 29
   Val Ala Phe His Lys Lys Asn Asp
   1 5
<210> 30
   <211> 4
   <212> PRT
   <213> Homo sapiens
<400> 30
   Ala Phe Leu His
   1
<210> 31
   <211> 8
   <212> PRT
   <213> Homo sapiens
<400> 31
   His Lys Lys Asn Asp Ile Asp Glu
   1 5
<210> 32
   <211> 6
   <212> PRT
   <213> Homo sapiens
<400> 32
   Lys Lys Asn Asp Ile Asp
   1 5
<210> 33
   <211> 6
   <212> PRT
   <213> Homo sapiens
<400> 33
   Lys Asn Asp Ile Asp Glu
   1 5
<210> 34
   <211> 8
   <212> PRT
   <213> Caldophera prolifera
<400> 34
   Lys Ala Ser Lys Phe Thr Lys His
   1 5
<210> 35
   <211> 12
   <212> PRT
   <213> Isolepis prolifera
<400> 35
   Lys Ala Gln Ala Glu Thr Gly Glu Ile Lys Gly His
   1 5 10
<210> 36
   <211> 10
   <212> PRT
   <213> Schizosaccharomyces pombe
<400> 36
   Lys Ser Phe Lys Tyr Pro Lys Lys His Lys
   1 5 10
<210> 37
   <211> 10
   <212> PRT
   <213> Oryza sativa
<400> 37
   Lys Lys Ala Tyr Gly Asn Glu Leu His Lys
   1 5 10
<210> 38
   <211> 9
   <212> PRT
   <213> Penicillium marneffei
<400> 38
   Lys Val Asp Ile Val Thr His Gln Lys
   1 5
<210> 39
   <211> 12
   <212> PRT
   <213> Diseula dcstructiva
<400> 39
   Lys Leu Glu Glu Asp Ala Ala Tyr His Arg Lys Lys
   1 5 10
<210> 40
   <211> 17
   <212> PRT
   <213> Ophiostoma novo-ulmi
<400> 40
   Lys Val Ile Leu Pro Leu Arg Gly Asn Ile Lys Gly Ile Phe Phe Lys
   1 5 10 15
   His
<210> 41
   <211> 11
   <212> PRT
   <213> Entamoeba invadens
<400> 41
   Lys Leu Ile Leu Lys Gly Asp Leu Asn Lys His
   1 5 10
<210> 42
   <211> 8
   <212> PRT
   <213> Helicobacter pylori
<400> 42
   Lys Ser Val His Ala Phe Leu Lys
   1 5
<210> 43
   <211> 9
   <212> PRT
   <213> Mycoplasma pulmonis
<400> 43
   Lys Val His Phe Phe Gln Leu Lys Lys
   1 5
<210> 44
   <211> 9
   <212> PRT
   <213> Arabidopsis thaliana
<400> 44
   Lys Asp His Asp Phe Asp Gly Asp Lys
   1 5
<210> 45
   <211> 11
   <212> PRT
   <213> Arabidopsis thaliana
<400> 45
   Lys Met Lys Gly Leu Lys Gln Lys Lys Ala His
   1 5 10
<210> 46
   <211> 12
   <212> PRT
   <213> Arabidopsis thaliana
<400> 46
   Lys Glu Leu Ser Ser Thr Thr Gln Glu Lys Ser His
   1 5 10
<210> 47
   <211> 9
   <212> PRT
   <213> Feline immunodeficiency virus
<400> 47
   His Leu Lys Asp Tyr Lys Leu Val Lys
   1 5
<210> 48
   <211> 7
   <212> PRT
   <213> Rous sarcoma virus
<400> 48
   Lys Lys Leu Arg His Glu Lys
   1 5
<210> 49
   <211> 7
   <212> PRT
   <213> Avian sarcoma virus
<400> 49
   Lys Lys Leu Arg His Asp Lys
   1 5
<210> 50
   <211> 7
   <212> PRT
   <213> Homo sapiens
<400> 50
   Lys Lys Leu Arg His Asp Lys
   1               5
<210> 51
   <211> 7
   <212> PRT
   <213> Avian sarcoma virus
<400> 51
   Lys Lys Leu Arg His Glu Lys
   1               5
<210> 52
   <211> 7
   <212> PRT
   <213> Homo sapiens
<400> 52
   Lys Lys Leu Arg His Glu Lys
   1               5
<210> 53
   <211> 8
   <212> PRT
   <213> Homo sapiens
<400> 53
   Lys Gln Ala His Glu Leu Ala Lys
   1               5
<210> 54
   <211> 8
   <212> PRT
   <213> Polyama virus
<400> 54
   Lys Thr His Arg Phe Ser Lys His
   1               5
<210> 55
   <211> 8
   <212> PRT
   <213> Sindbis virus
<400> 55
   Lys Asn Leu His Glu Lys Ile Lys
   1 5
<210> 56
   <211> 9
   <212> PRT
   <213> Human papilloamavirus type 71
<400> 56
   Lys His Arg Pro Leu Leu Gln Leu Lys
   1 5
<210> 57
   <211> 7
   <212> PRT
   <213> Avian encephalomyelitis virus
<400> 57
   Lys Ser Pro Asn His Val Lys
   1 5
<210> 58
   <211> 8
   <212> PRT
   <213> Feline sarcoma virus
<400> 58
   Lys Asn Ile His Leu Glu Lys Lys
   1 5
<210> 59
   <211> 8
   <212> PRT
   <213> Homo sapiens
<400> 59
   Lys Asn Ile His Leu Glu Lys Lys
   1 5
<210> 60
   <211> 10
   <212> PRT
   <213> Polyoma virus
<400> 60
   Lys Pro His Leu Ala Gln Ser Leu Glu Lys
   1 5 10
<210> 61
   <211> 9
   <212> PRT
   <213> Polyoma virus
<400> 61
   Lys Gln His Arg Glu Leu Lys Asp Lys
   1 5
<210> 62
   <211> 9
   <212> PRT
   <213> Polyoma virus
<400> 62
   Lys Gln His Arg Glu Leu Lys Asp Lys
   1 5
<210> 63
   <211> 12
   <212> PRT
   <213> Murine leukemia virus
<400> 63
   Lys Val Pro Val Leu Ile Ser Pro Thr Leu Lys His
   1 5 10
<210> 64
   <211> 13
   <212> PRT
   <213> Human T-cell lymphotropic virus type 2
<400> 64
   Lys Ser Leu Leu Leu Glu Val Asp Lys Asp Ile Ser His
   1 5 10
<210> 65
   <211> 13
   <212> PRT
   <213> Homo sapiens
<400> 65
   Lys Ala Gly Ile Thr Ile Met Val Lys Arg Glu Tyr His
   1 5 10
<210> 66
   <211> 8
   <212> PRT
   <213> Homo sapiens
<400> 66
   Lys Ser Gly Lys His Leu Gly Lys
   1 5
<210>67
   <211> 9
   <212> PRT
   <213> Homo sapiens
<400> 67
   Lys Arg Arg Glu Gln Leu Lys His Lys
   1 5
<210> 68
   <211>10
   <212> PRT
   <213> Homo sapiens
<400> 68
   Lys Ser Phe Glu Val Ile Lys Val Ile His
   1 5 10
<210> 69
   <211> 8
   <212> PRT
   <213> Homo sapiens
<400> 69
   Lys Lys Lys His Thr Val Lys Lys
   1 5
<210> 70
   <211> 9
   <212> PRT
   <213> Homo sapiens
<400> 70
   Lys Ala Gln Lys Asp His Leu Ser Lys
   1 5
<210> 71
   <211> 10
   <212> PRT
   <213> Homo sapiens
<400> 71
   His Leu Lys Arg Val Lys Asp Leu Lys Lys
   1 5 10
<210> 72
   <211> 11
   <212> PRT
   <213> Homo sapiens
<400> 72
   Lys Tyr Gly Ser Pro Lys His Arg Leu Ile Lys
   1 5 10
<210> 73
   <211> 13
   <212> PRT
   <213> Papilloma virus type 11
<400> 73
   Lys Leu Lys His Ile Leu Gly Lys Ala Arg Phe Ile Lys
   1 5 10
<210> 74
   <211> 12
   <212> PRT
   <213> Homo sapiens
<400> 74
   Lys Gly Asp His Val Lys His Tyr Lys Ile Arg Lys
   1 5 10
<210> 75
   <211> 13
   <212> PRT
   <213> Homo sapiens
<400> 75
   Lys Glu Lys Leu Arg Asp Val Met Val Asp Arg His Lys
   1 5 10
<210> 76
   <211> 15
   <212> PRT
   <213> Homo sapiens
<400> 76
   Lys Leu Gln Ala Arg Gln Gln Gln Leu Leu Lys Lys Ile Glu His
   1 5 10 15
<210> 77
   <211> 14
   <212> PRT
   <213> Homo sapiens
<400> 77
   Lys Lys Gly Asn Arg Val Ser Pro Thr Met Lys Val Thr His
   1 5 10
<210> 78
   <211> 9
   <212> PRT
   <213> Homo sapiens
<400> 78
   Lys Glu Ile Pro Leu His Phe Arg Lys
   1 5
<210> 79
   <211> 8
   <212> PRT
   <213> Homo sapiens
<400> 79
   Lys Lys Lys Pro His Ile Lys Lys
   1 5
<210> 80
   <211> 9
   <212> PRT
   <213> Homo sapiens
<400> 80
   Lys Thr Arg His Asp Pro Leu Ala Lys
   1 5
<210> 81
   <211> 10
   <212> PRT
   <213> Homo sapiens
<400> 81
   Lys His His Pro Lys Asp Asn Leu Ile Lys
   1 5 10
<210> 82
   <211> 10
   <212> PRT
   <213> Homo sapiens
<400> 82
   Lys His Lys Arg Lys Lys Phe Arg Gln Lys
   1 5 10
<210> 83
   <211> 10
   <212> PRT
   <213> Homo sapiens
<400> 83
   Lys Ala Gly Val Ala Phe Leu His Lys Lys
   1 5 10
<210> 84
   <211> 10
   <212> PRT
   <213> Homo sapiens
<400> 84
   Lys His Lys Arg Lys Lys Phe Arg Gln Lys
   1 5 10
<210> 85
   <211> 10
   <212> PRT
   <213> Homo sapiens
<400> 85
   Lys Lys Lys Ser Lys Lys His Lys Asp Lys
   1 5 10
<210> 86
   <211> 11
   <212> PRT
   <213> Homo sapiens
<400> 86
   His Lys Ser Glu Lys Pro Ala Leu Pro Arg Lys
   1 5 10
<210> 87
   <211> 14
   <212> PRT
   <213> Homo sapiens
<400> 87
   Lys Lys Lys Lys Pro Ser Arg Leu Lys Gly Asp Asn Glu Lys
    1               5                   10
<210> 88
   <211> 16
   <212> PRT
   <213> Homo sapiens
<400> 88
   Lys Thr Lys Lys Gly Asn Arg Val Ser Pro Thr Met Lys Val Thr His
    1               5                   10                  15
<210> 89
   <211> 18
   <212> PRT
   <213> Homo sapiens
<400> 89
   Lys His Lys Glu Lys Met Ser Lys Asp Gly Lys Lys Lys Lys Lys Lys
    1               5                   10                  15
   Ser Lys
<210> 90
   <211> 9
   <212> PRT
   <213> Legionella sp.
<400> 90
   Lys Ile His Leu Ile Ser Val Lys Lys
    1               5
<210> 91
   <211> 10
   <212> PRT
   <213> Bacillus anthracis
<400> 91
   His Val Lys Lys Glu Lys Glu Lys Asn Lys
    1               5                   10
<210> 92
   <211> 9
   <212> PRT
   <213> Bacillus anthracis
<400> 92
   Lys His Ile Val Lys Ile Glu Val Lys
   1 5
<210> 93
   <211> 16
   <212> PRT
   <213> Bacillus anthracis
<400> 93
   Lys Lys Lys Lys Ile Lys Asp Ile Tyr Gly Lys Asp Ala Leu Leu His
   1 5 10 15
<210> 94
   <211> 8
   <212> PRT
   <213> Bacillus anthracis
<400> 94
   Lys Trp Glu Lys Ile Lys Gln His
   1 5
<210> 95
   <211> 18
   <212> PRT
   <213> Bacillus anthracis
<400> 95
   Lys Lys Leu Gln Ile Pro Pro Pro Ile Glu Pro Lys Lys Asp Asp Ile
   1 5 10 15
   Ile His
<210> 96
   <211> 31
   <212> PRT
   <213> Bacillus anthracis
<400> 96
   His Asn Arg Tyr Ala Ser Asn Ile Val Glu Ser Ala Tyr Leu Leu Ile
   1 5 10 15
   Leu Asn Glu Trp Lys Asn Asn Ile Gln Ser Asp Leu Ile Lys Lys
   20 25 30
<210> 97
   <211> 24
   <212> PRT
   <213> Bacillus anthracis
<400> 97
   His Ala Val Asp Asp Tyr Ala Gly Tyr Leu Leu Asp Lys Asn Gln Ser
   1 5 10 15
   Asp Leu Val Thr Asn Ser Lys Lys
   20
<210> 98
   <211> 13
   <212> PRT
   <213> Bacillus anthracis
<400> 98
   His Ala Glu Arg Leu Lys Val Gln Lys Asn Ala Pro Lys
   1 5 10
<210> 99
   <211> 10
   <212> PRT
   <213> Variola virus
<400> 99
   Lys His Tyr Asn Asn Ile Thr Trp Tyr Lys
   1 5 10
<210> 100
   <211> 12
   <212> PRT
   <213> Variola virus
<400> 100
   Lys Tyr Ser Gln Thr Gly Lys Glu Leu Ile Ile His
   1 5 10
<210> 101
   <211> 17
   <212> PRT
   <213> Variola virus
<400> 101
   His Tyr Asp Asp Val Arg Ile Lys Asn Asp Ile Val Val Ser Arg Cys
   1 5 10 15
   Lys
<210> 102
   <211> 11
   <212> PRT
   <213> Variola virus
<400> 102
   His Arg Phe Lys Leu Ile Leu Asp Ser Lys Ile
   1 5 10
<210> 103
   <211> 11
   <212> PRT
   <213> Variola virus
<400> 103
   Lys Glu Arg Gly His Asn Tyr Tyr Phe Glu Lys
   1 5 10
<210> 104
   <211> 8
   <212> PRT
   <213> Influenza B virus
<400> 104
   Lys Ser His Phe Ala Asn Leu Lys
   1 5
<210> 105
   <211> 11
   <212> PRT
   <213> Influenza B virus
<400> 105
   Lys Ser His Phe Ala Asn Leu Lys Gly Thr Lys
   1 5 10
<210> 106
   <211> 19
   <212> PRT
   <213> Influenza B virus
<400> 106
   Lys Ser His Phe Ala Asn Leu Lys Gly Thr Lys Thr Arg Gly Lys Leu
   1 5 10 15
   Cys Pro Lys
<210> 107
   <211> 9
   <212> PRT
   <213> Influenza B virus
<400> 107
   His Glu Lys Tyr Gly Gly Leu Asn Lys
   1 5
<210> 108
   <211> 11
   <212> PRT
   <213> Influenza B virus

<400> 108
   His Glu Lys Tyr Gly Gly Leu Asn Lys Ser Lys
   1 5 10
<210> 109
   <211> 20
   <212> PRT
   <213> Influenza B virus
<400> 109
   His Glu Lys Tyr Gly Gly Leu Asn Lys Ser Lys Pro Tyr Tyr Thr Gly
   1 5 10 15
   Glu His Ala Lys
   20
<210> 110
   <211> 13
   <212> PRT
   <213> Influenza B virus
<400> 110
   His Ala Lys Ala Ile Gly Asn Cys Pro Ile Trp Val Lys
   1 5 10
<210> 111
   <211> 23
   <212> PRT
   <213> Influenza B virus
<400> 111
   His Ala Lys Ala Ile Gly Asn Cys Pro Ile Trp Val Lys Thr Pro Leu
   1 5 10 15
   Lys Leu Ala Asn Gly Thr Lys
   20
<210> 112
   <211> 29
   <212> PRT
   <213> Influenza B virus
<400> 112
   His Ala Lys Ala Ile Gly Asn Cys Pro Ile Trp Val Lys Thr Pro Leu
   1 5 10 15
   Lys Leu Ala Asn Gly Thr Lys Tyr Arg Pro Pro Ala Lys
   20 25
<210> 113
   <211> 32
   <212> PRT
   <213> Influenza B virus
<400> 113
   His Ala Lys Ala Ile Gly Asn Cys Pro Ile Trp Val Lys Thr Pro Leu
   1 5 10 15
   Lys Leu Ala Asn Gly Thr Lys Tyr Arg Pro Pro Ala Lys Leu Leu Lys
   20 25 30
<210> 114
   <211> 13
   <212> PRT
   <213> Influenza B virus
<400> 114
   His Phe Ala Asn Leu Lys Gly Thr Lys Thr Arg Gly Lys
   1 5 10
<210> 115
   <211> 17
   <212> PRT
   <213> Influenza B virus
<400> 115
   His Phe Ala Asn Leu Lys Gly Thr Lys Thr Arg Gly Lys Leu Cys Pro
   1 5 10 15
   Lys
<210> 116
   <211> 16
   <212> PRT
   <213> Influenza B virus
<400> 116
   His Ser Asp Asn Glu Ile Gln Met Val Lys Leu Tyr Gly Asp Ser Lys
   1 5 10 15
<210> 117
   <211> 21
   <212> PRT
   <213> Influenza B virus
<400> 117
   His Ser Asp Asn Glu Ile Gln Asp Lys Met Val Lys Leu Tyr Gly Asp
   1 5 10 15
   Ser Lys Pro Gln Lys
   20
<210> 118
   <211> 19
   <212> PRT
   <213> Influenza B virus
<400> 118
   His Ser Asp Asn Glu Ile Gln Met Val Lys Leu Tyr Gly Asp Ser Lys
   1 5 10 15
   Pro Gln Lys
<210> 119
   <211> 9
   <212> PRT
   <213> Influenza B virus
<220>
   <221> MOD_RES
   <222> (2)
   <223> ala or val
<400> 119
   Lys Xaa Ser Ile Leu His Glu Val Lys
   1 5
<210> 120
   <211> 15
   <212> PRT
   <213> Influenza B virus
<400> 120
   Lys Cys Thr Gly Thr Ile Pro Ser Ala Lys Ala Ser Ile Leu His
   1 5 10 15
<210> 121
   <211> 18
   <212> PRT
   <213> Influenza B virus
<400> 121
   Lys Cys Thr Gly Thr Ile Pro Ser Ala Lys Ala Ser Ile Leu His Glu
   1 5 10 15
   Val Lys
<210> 122
   <211> 16
   <212> PRT
   <213> Influenza B virus
<400> 122
   Lys Tyr Gly Gly Leu Asn Lys Ser Lys Pro Tyr Tyr Thr Gly Glu His
   1 5 10 15
<210> 123
   <211> 26
   <212> PRT
   <213> Influenza B virus
<400> 123
   Lys Val Trp Cys Ala Ser Gly Arg Ser Lys Val Ile Lys Gly Ser Leu
   1 5 10 15
   Pro Leu Ile Gly Glu Ala Asp Cys Leu His
   20 25
<210> 124
   <211> 10
   <212> PRT
   <213> Influenza B virus
<400> 124
   Lys Pro Tyr Tyr Thr Gly Glu His Ala Lys
   1 5 10
<210> 125
   <211> 18
   <212> PRT
   <213> Influenza B virus
<400> 125
   Lys Cys Met Gly Thr Ile Pro Ser Ala Lys Ala Ser Ile Leu His Glu
   1 5 10 15
   Val Lys
<210> 126
   <211> 15
   <212> PRT
   <213> Influenza B virus
<400> 126
   His Asn Val Ile Asn Ala Glu Lys Ala Pro Gly Gly Pro Tyr Lys
   1 5 10 15
<210> 127
   <211> 16
   <212> PRT
   <213> Influenza B virus
<400> 127
   His Ser Asp Asn Glu Thr Gln Met Ala Lys Leu Tyr Gly Asp Ser Lys
   1 5 10 15
<210> 128
   <211> 18
   <212> PRT
   <213> Influenza B virus
<400> 128
   His Gly Val Ala Val Ala Ala Asp Leu Lys Ser Thr Gln Glu Ala Ile
   1 5 10 15
   Asn Lys
<210> 129
   <211> 29
   <212> PRT
   <213> Influenza B virus
<400> 129
   His Gly Val Ala Val Ala Ala Asp Leu Lys Ser Thr Gln Glu Ala Ile
   1 5 10 15
   Asn Lys Asp Thr Ile Ser Thr Gln Glu Ala Ile Asn Lys
   20 25
<210> 130
   <211> 21
   <212> PRT
   <213> Influenza B virus
<400> 130
   Lys Leu Tyr Gly Asp Ser Lys Pro Gln Lys Phe Thr Ser Ser Ala Asn
   1 5 10 15
   Gly Val Thr Thr His
   20
<210> 131
   <211> 19
   <212> PRT
   <213> Influenza B virus
<400> 131
   His Ser Asp Asn Glu Thr Gln Met Ala Lys Leu Tyr Gly Asp Ser Lys
   1 5 10 15
   Pro Gln Lys
<210> 132
   <211> 13
   <212> PRT
   <213> Influenza B virus
<400> 132
   His Phe Ala Asn Leu Lys Gly Thr Gln Thr Arg Gly Lys
   1 5 10
<210> 133
   <211> 12
   <212> PRT
   <213> Influenza B virus
<400> 133
   Lys Pro Arg Ser Ala Leu Lys Cys Lys Gly Phe His
   1 5 10
<210> 134
   <211> 22
   <212> PRT
   <213> Influenza B virus
<220>
   <221> MOD_RES
   <222> (15)
   <223> gly or ala
<400> 134
   Lys Ser Lys Pro Tyr Tyr Thr Gly Glu His Ala Lys Ala Ile Xaa Asn
   1 5 10 15
   Cys Pro Ile Trp Val Lys
   20
<210> 135
   <211> 16
   <212> PRT
   <213> Influenza virus
<220>
   <221> MOD_RES
   <222> (3)
   <223> val or ile
<220>
   <221> MOD_RES
   <222> (13)
   <223> arg or lys
<220>
   <221> MOD RES
   <222> (14)
   <223> ser or thr
<220>
   <221> MOD_RES
   <222> (15)
   <223> thr or ala
<400> 135
   His Pro Xaa Thr Ile Gly Glu Cys Pro Lys Tyr Val Xaa Xaa Xaa Lys
   1 5 10 15
<210> 136
   <211> 21
   <212> PRT
   <213> Influenza virus
<220>
   <221> MOD_RES
   <222> (10)
   <223> glu or gly
<220>
   <221> MOD_RES
   <222> (13)
   <223> lys or arg
<220>
   <221> MOD_RES
   <222> (14)
   <223> asn or ser
<220>
   <221> MOD_RES
   <222> (17)
   <223> lys or arg
<400> 136
   His Asp Ser Asn Val Lys Asn Leu Tyr Xaa Lys Val Xaa Xaa Gln Leu
   1               5                   10                  15
   Xaa Asn Asn Ala Lys
                   20
<210> 137
   <211> 17
   <212> PRT
   <213> Influenza virus
<220>
   <221> MOD_RES
   <222> (10)
   <223> glu or gly
<220>
   <221> MOD_RES
   <222> (13)
   <223> lys or arg
<220>
   <221> MOD_RES
   <222> (14)
   <223> asn or ser
<400> 137
   His Asp Ser Asn Val Lys Asn Leu Tyr Xaa Lys Val Xaa Xaa Gln Leu
   1               5                   10                  15
   Lys
<210> 138
   <211> 36
   <212> PRT
   <213> Influenza virus
<220>
   <221> MOD_RES
   <222> (4)..(5)
   <223> asn or asp
<220>
   <221> MOD_RES
   <222> (6)
   <223> ala, thr or glu
<220>
   <221> MOD_RES
   <222> (12)
   <223> arg or lys
<220>
   <221> MOD_RES
   <222> (31)
   <223> glu or lys
<400> 138
   His Lys Cys Xaa Xaa Xaa Cys Met Glu Ser Val Xaa Asn Gly Thr Tyr
   1 5 10 15
   Asp Tyr Pro Lys Tyr Ser Glu Glu Ser Lys Leu Asn Arg Glu Xaa Ile
   20 25 30
   Asp Gly Val Lys
   35
<210> 139
   <211> 26
   <212> PRT
   <213> Influenza virus
<220>
   <221> MOD_RES
   <222> (4)..(5)
   <223> asn or asp
<220>
   <221> MOD_RES
   <222> (6)
   <223> ala, thr or glu
<220>
   <221> MOD_RES
   <222> (12)
   <223> arg or lys
<400> 139
   His Lys Cys Xaa Xaa Xaa Cys Met Glu Ser Val Xaa Asn Gly Thr Tyr
   1 5 10 15
   Asp Tyr Pro Lys Tyr Ser Glu Glu Ser Lys
   20 25
<210> 140
   <211> 50
   <212> PRT
   <213> Influenza virus
<220>
   <221> MOD_RES
   <222> (4)
   <223> glu or gly
<220>
   <221> MOD_RES
   <222> (21)
   <223> asp or asn
<400> 140
   His Gln Asn Xaa Gln Gly Ser Gly Tyr Ala Ala Asp Gln Lys Ser Thr
   1               5                   10                  15
   Gln Asn Ala Ile Xaa Gly Ile Thr Asn Lys Val Asn Ser Val Ile Glu
               20                  25                  30
   Lys Met Asn Thr Gln Phe Thr Ala Val Gly Lys Glu Phe Asn Lys Leu
           35                  40                  45
   Glu Lys
       50
<210> 141
   <211> 33
   <212> PRT
   <213> Influenza virus
<220>
   <221> MOD_RES
   <222> (4)
   <223> glu or gly
<220>
   <221> MOD_RES
   <222> (21)
   <223> asp or asn
<400> 141
   His Gln Asn Xaa Gln Gly Ser Gly Tyr Ala Ala Asp Gln Lys Ser Thr
   1               5                   10                  15
   Gln Asn Ala Ile Xaa Gly Ile Thr Asn Lys Val Asn Ser Val Ile Glu
               20                  25                  30
   Lys
<210> 142
   <211> 26
   <212> PRT
   <213> Influenza virus
<220>
   <221> MOD_RES
   <222> (4)
   <223> glu or gly
<220>
   <221> MOD_RES
   <222> (21)
   <223> asp or asn
<400> 142
   His Gln Asn Xaa Gln Gly Ser Gly Tyr Ala Ala Asp Gln Lys Ser Thr
   1 5 10 15
   Gln Asn Ala Ile Xaa Gly Ile Thr Asn Lys
   20 25
<210> 143
   <211> 14
   <212> PRT
   <213> Influenza virus
<400> 143
   Lys Phe Glu Ile Phe Pro Lys Thr Ser Ser Trp Pro Asn His
   1 5 10
<210> 144
   <211> 27
   <212> PRT
   <213> Influenza virus
<220>
   <221> MOD_RES
   <222> (3)
   <223> asn, ser or thr
<220>
   <221> MOD_RES
   <222> (9)
   <223> asn or ser
<220>
   <221> MOD_RES
   <222> (13)
   <223> val or thr
<400> 144
   Lys Gly Xaa Ser Tyr Pro Lys Leu Xaa Lys Ser Tyr Xaa Asn Asn Lys
   1 5 10 15
   Gly Lys Glu Val Leu Val Leu Trp Gly Val His
   20 25
<210> 145
   <211> 18
   <212> PRT
   <213> Influenza virus
<220>
   <221> MOD_RES
   <222> (4)
   <223> val or thr
<400> 145
   Lys Ser Tyr Xaa Asn Asn Lys Gly Lys Glu Val Leu Val Leu Trp Gly
   1               5                   10                  15
   Val His
<210> 146
   <211> 36
   <212> PRT
   <213> Influenza virus
<400> 146
   His Lys Cys Asn Asn Glu Cys Met Glu Ser Val Lys Asn Gly Thr Tyr
   1               5                   10                  15
   Asp Tyr Pro Lys Tyr Ser Glu Glu Ser Lys Leu Asn Arg Glu Lys Ile
               20                  25                  30
   Asp Gly Val Lys
           35
<210> 147
   <211> 26
   <212> PRT
   <213> Influenza virus
<400> 147
   His Lys Cys Asn Asn Glu Cys Met Glu Ser Val Lys Asn Gly Thr Tyr
   1               5                   10                  15
   Asp Tyr Pro Lys Tyr Ser Glu Glu Ser Lys
               20                  25
<210> 148
   <211> 20
   <212> PRT
   <213> Influenza virus
<400> 148
   His Lys Cys Asn Asn Glu Cys Met Glu Ser Val Lys Asn Gly Thr Tyr
   1               5                   10                  15
   Asp Tyr Pro Lys
               20
<210> 149
   <211> 12
   <212> PRT
   <213> Influenza virus
<400> 149
   His Lys Cys Asn Asn Glu Cys Met Glu Ser Val Lys
   1 5 10
<210> 150
   <211> 34
   <212> PRT
   <213> Influenza virus
<220>
   <221> MOD_RES
   <222> (9)
   <223> lys or arg
<220>
   <221> MOD_RES
   <222> (16)
   <223> glu or gly
<400> 150
   His Asn Gly Lys Ser Ser Phe Tyr Xaa Asn Leu Leu Trp Leu Thr Xaa
   1 5 10 15
   Lys Asn Gly Leu Tyr Pro Asn Leu Ser Lys Ser Tyr Val Asn Asn Lys
   20 25 30
   Glu Lys
<210> 151
   <211> 32
   <212> PRT
   <213> Influenza virus
<220>
   <221> MOD_RES
   <222> (9)
   <223> lys or arg
<220>
   <221> MOD_RES
   <222> (16)
   <223> glu or gly
<400> 151
   His Asn Gly Lys Ser Ser Phe Tyr Xaa Asn Leu Leu Trp Leu Thr Xaa
   1 5 10 15
   Lys Asn Gly Leu Tyr Pro Asn Leu Ser Lys Ser Tyr Val Asn Asn Lys
   20 25 30
<210> 152
   <211> 26
   <212> PRT
   <213> Influenza virus
<220>
   <221> MOD_RES
   <222> (9)
   <223> lys or arg
<220>
   <221> MOD_RES
   <222> (16)
   <223> glu or gly
<400> 152
   His Asn Gly Lys Ser Ser Phe Tyr Xaa Asn Leu Leu Trp Leu Thr Xaa
   1 5 10 15
   Lys Asn Gly Leu Tyr Pro Asn Leu Ser Lys
   20 25
<210> 153
   <211> 17
   <212> PRT
   <213> Influenza virus
<220>
   <221> MOD_RES
   <222> (9)
   <223> lys or arg
<220>
   <221> MOD_RES
   <222> (16)
   <223> glu or gly
<400> 153
   His Asn Gly Lys Ser Ser Phe Tyr Xaa Asn Leu Leu Trp Leu Thr Xaa
   1 5 10 15
   Lys
<210> 154
   <211> 40
   <212> PRT
   <213> Influenza virus
<400> 154
   Lys Ser Ser Phe Tyr Lys Asn Leu Leu Trp Leu Thr Glu Lys Asn Gly
   1 5 10 15
   Leu Tyr Pro Asn Leu Ser Lys Ser Tyr Val Asn Asn Lys Glu Lys Glu
   20 25 30
   Val Leu Val Leu Trp Gly Val His
   35 40
<210> 155
   <211> 35
   <212> PRT
   <213> Influenza virus
<400> 155
   Lys Asn Leu Leu Trp Leu Thr Glu Lys Asn Gly Leu Tyr Pro Asn Leu
   1               5                   10                  15
   Ser Lys Ser Tyr Val Asn Asn Lys Glu Lys Glu Val Leu Val Leu Trp
               20                  25                  30
   Gly Val His
           35
<210> 156
   <211> 27
   <212> PRT
   <213> Influenza virus
<400> 156
   Lys Asn Gly Leu Tyr Pro Asn Leu Ser Lys Ser Tyr Val Asn Asn Lys
   1               5                   10                  15
   Glu Lys Glu Val Leu Val Leu Trp Gly Val His
               20                  25
<210> 157
   <211> 18
   <212> PRT
   <213> Influenza virus
<220>
   <221> MOD_RES
   <222> (4)
   <223> val or ala
<220>
   <221> MOD_RES
   <222> (12)
   <223> lys or not present
<220>
   <221> MOD_RES
   <222> (13)
   <223> val or not present
<400> 157
   Lys Ser Tyr Xaa Asn Asn Lys Glu Lys Glu Val Xaa Xaa Leu Trp Gly
   1               5                   10                  15
   Val His
<210> 158
   <211> 12
   <212> PRT
   <213> Influenza virus
<400> 158
   Lys Glu Ser Ser Trp Pro Asn His Thr Val Thr Lys
   1               5                   10
<210> 159
   <211> 44
   <212> PRT
   <213> Influenza virus
<220>
   <221> MOD_RES
   <222> (4)
   <223> thr or asn
<400> 159
   His Glu Thr Xaa Lys Gly Val Thr Ala Ala Cys Pro Tyr Ala Gly Ala
   1               5                   10                  15
   Ser Ser Phe Tyr Arg Asn Leu Leu Trp Leu Val Lys Lys Glu Asn Ser
               20                  25                  30
   Tyr Pro Lys Leu Ser Lys Ser Tyr Val Asn Asn Lys
           35                  40
<210> 160
   <211> 38
   <212> PRT
   <213> Influenza virus
<220>
   <221> MOD_RES
   <222> (4)
   <223> thr or asn
<400> 160
   His Glu Thr Xaa Lys Gly Val Thr Ala Ala Cys Pro Tyr Ala Gly Ala
   1               5                   10                  15
   Ser Ser Phe Tyr Arg Asn Leu Leu Trp Leu Val Lys Lys Glu Asn Ser
               20                  25                  30
   Tyr Pro Lys Leu Ser Lys
           35
<210> 161
   <211> 22
   <212> PRT
   <213> Influenza virus
<400> 161
   Lys Phe Glu Ile Phe Pro Lys Thr Ser Ser Trp Pro Asn Glu Val Leu
   1 5 10 15
   Val Leu Trp Gly Val His
   20
<210> 162
   <211> 8
   <212> PRT
   <213> Influenza virus
<400> 162
   Lys Glu Arg Ser Trp Pro Lys His
   1 5
<210> 163
   <211> 21
   <212> PRT
   <213> Influenza virus
<400> 163
   Lys Leu Ser Lys Ser Tyr Val Asn Asn Lys Glu Lys Glu Val Leu Val
   1 5 10 15
   Leu Trp Gln Val His
   20
<210> 164
   <211> 15
   <212> PRT
   <213> Influenza virus
<400> 164
   Lys Asn Asn Lys Glu Lys Glu Val Leu Val Leu Trp Gln Val His
   1 5 10 15
<210> 165
   <211> 34
   <212> PRT
   <213> Influenza virus
<220>
   <221> MOD_RES
   <222> (2)
   <223> lys or asn
<220>
   <221> MOD_RES
   <222> (3)
   <223> gly or gln
<220>
   <221> MOD_RES
   <222> (9)
   <223> arg or lys
<220>
   <221> MOD_RES
   <222> (20)
   <223> lys or ser
<220>
   <221> MOD_RES
   <222> (23)
   <223> asn or thr
<400> 165
   His Xaa Xaa Lys Ser Ser Phe Tyr Xaa Asn Leu Leu Trp Leu Thr Glu
    1               5                  10                  15
   Lys Asn Gly Xaa Tyr Pro Xaa Leu Ser Lys Ser Tyr Ala Asn Asn Lys
               20                  25                  30
   Glu Lys
<210> 166
   <211> 17
   <212> PRT
   <213> Influenza virus
<220>
   <221> MOD_RES
   <222> (2)
   <223> lys or asn
<220>
   <221> MOD_RES
   <222> (3)
   <223> gly or gln
<220>
   <221> MOD_RES
   <222> (9)
   <223> arg or lys
<400> 166
   His Xaa Xaa Lys Ser Ser Phe Tyr Xaa Asn Leu Leu Trp Leu Thr Glu
    1               5                  10                  15
   Lys
<210> 167
   <211> 9
   <212> PRT
   <213> Influenza virus
<400> 167
   His Ala Lys Lys Ser Ser Phe Tyr Lys
   1 5
<210> 168
   <211> 11
   <212> PRT
   <213> Influenza virus
<400> 168
   His Asn Gly Lys Leu Cys Arg Leu Lys Gly Lys
   1 5 10
<210> 169
   <211> 9
   <212> PRT
   <213> Influenza virus
<220>
   <221> MOD_RES
   <222> (7)
   <223> gln or gly
<400> 169
   His Tyr Lys Leu Asn Asn Xaa Lys Lys
   1 5
<210> 170
   <211> 25
   <212> PRT
   <213> Influenza virus
<400> 170
   His Asp Ile Tyr Arg Asp Glu Ala Ile Asn Asn Arg Phe Gln Ile Gln
   1 5 10 15
   Gly Val Lys Leu Thr Gln Gly Tyr Lys
   20 25
<210> 171
   <211> 11
   <212> PRT
   <213> Influenza virus
<400> 171
   Lys Gly Asn Gly Cys Phe Glu Ile Phe His Lys
   1 5 10
<210> 172
   <211> 18
   <212> PRT
   <213> Influenza virus
<400> 172
   Lys Leu Asn Arg Leu Ile Glu Lys Thr Asn Asp Lys Tyr His Gln Ile
   1 5 10 15
   Glu Lys
<210> 173
   <211> 14
   <212> PRT
   <213> Influenza virus
<400> 173
   Lys Leu Asn Arg Leu Ile Glu Lys Thr Asn Asp Lys Tyr His
   1 5 10
<210> 174
   <211> 13
   <212> PRT
   <213> Influenza virus
<400> 174
   Lys Cys His Thr Asp Lys Gly Ser Leu Ser Thr Thr Lys
   1 5 10
<210> 175
   <211> 16
   <212> PRT
   <213> Influenza virus
<400> 175
   Lys Ile Asn Asn Gly Asp Tyr Ala Lys Leu Tyr Ile Trp Gly Val His
   1 5 10 15
<210> 176
   <211> 17
   <212> PRT
   <213> Influenza virus
<400> 176
   His Asn Gly Lys Leu Cys Arg Lys Gly Ile Ala Pro Leu Gln Leu Gly
   1 5 10 15
   Lys
<210> 177
   <211> 38
   <212> PRT
   <213> Influenza virus
<400> 177
   His Glu Thr Asn Arg Gln Val Thr Ala Ala Cys Pro Tyr Ala Gly Ala
   1 5 10 15
   Asn Ser Phe Phe Arg Asn Leu Ile Trp Leu Val Lys Lys Glu Ser Ser
   20 25 30
   Tyr Pro Lys Leu Ser Lys
   35
<210> 178
   <211> 35
   <212> PRT
   <213> Influenza virus
<400> 178
   His Glu Thr Asn Arg Gln Val Thr Ala Ala Cys Pro Tyr Ala Gly Ala
   1 5 10 15
   Asn Ser Phe Phe Arg Asn Leu Ile Trp Leu Val Lys Lys Glu Ser Ser
   20 25 30
   Tyr Pro Lys
   35
<210> 179
   <211> 31
   <212> PRT
   <213> Influenza virus
<400> 179
   His Pro Pro Thr Ser Thr Asp Gln Gln Ser Leu Tyr Gln Asn Ala Asp
   1 5 10 15
   Ala Tyr Ile Phe Val Gly Ser Ser Lys Tyr Asn Arg Lys Phe Lys
   20 25 30
<210> 180
   <211> 35
   <212> PRT
   <213> Influenza virus
<400> 180
   His Pro Pro Thr Ser Thr Asp Gln Gln Ser Leu Tyr Gln Asn Ala Asp
   1 5 10 15
   Ala Tyr Ile Phe Val Gly Ser Ser Lys Tyr Asn Arg Lys Phe Lys Pro
   20 25 30
   Glu Ile Ala
   35

<210> 181
   <211> 25
   <212> PRT
   <213> Influenza virus
<400> 181
   His Asp Ile Tyr Arg Asp Glu Ala Ile Asn Asn Arg Phe Gln Ile Gln
   1 5 10 15
   Gly Val Lys Ile Thr Gln Gly Tyr Lys
   20 25
<210> 182
   <211> 43
   <212> PRT
   <213> Influenza virus
<400> 182
   His Gln Asn Glu Gln Gly Ser Gly Tyr Ala Ala Asp Gln Lys Ser Thr
   1 5 10 15
   Gln Asn Ala Ile Asp Gly Ile Thr Asn Lys Val Asn Ser Val Ile Glu
   20 25 30
   Lys Met Asn Thr Gln Phe Thr Ala Val Gly Lys
   35 40
<210> 183
   <211> 33
   <212> PRT
   <213> Influenza virus
<400> 183
   His Gln Asn Glu Gln Gly Ser Gly Tyr Ala Ala Asp Gln Lys Ser Thr
   1 5 10 15
   Gln Asn Ala Ile Asp Gly Ile Thr Asn Lys Val Asn Ser Val Ile Glu
   20 25 30
   Lys
<210> 184
   <211> 50
   <212> PRT
   <213> Influenza virus
<400> 184
   His Gln Asn Glu Gln Gly Ser Gly Tyr Ala Ala Asp Gln Lys Ser Thr
   1 5 10 15
   Gln Asn Ala Ile Asn Gly Ile Thr Asn Lys Val Asn Ser Val Ile Glu
   20 25 30
   Lys Met Asn Thr Gln Phe Thr Ala Val Gly Lys Glu Phe Asn Lys Leu
   35 40 45
   Glu Lys
   50
<210> 185
   <211> 18
   <212> PRT
   <213> Influenza virus
<400> 185
   His Asn Gly Lys Leu Cys Arg Leu Lys Gly Ile Ala Pro Leu Gln Leu
   1 5 10 15
   Gly Lys
<210> 186
   <211> 12
   <212> PRT
   <213> Influenza virus
<400> 186
   His Lys Cys Asn Asn Glu Cys Met Glu Ser Val Lys
   1 5 10
<210> 187
   <211> 14
   <212> PRT
   <213> Influenza virus
<400> 187
   Lys Phe Glu Ile Phe Pro Lys Ala Ser Ser Trp Pro Asn His
   1 5 10
<210> 188
   <211> 21
   <212> PRT
   <213> Influenza virus
<400> 188
   His Asp Ser Asn Val Lys Asn Leu Tyr Glu Lys Val Arg Ser Gln Leu
   1 5 10 15
   Arg Asn Asn Ala Lys
   20
<210> 189
   <211> 22
   <212> PRT
   <213> Influenza virus
<400> 189
   Lys Val Asn Ser Val Ile Lys Lys Met Asn Thr Gln Phe Ala Ala Val
   1 5 10 15
   Gly Lys Glu Phe Asn His
   20
<210> 190
   <211> 8
   <212> PRT
   <213> Influenza virus
<400> 190
   Lys His Asn Gly Lys Leu Cys Lys
   1 5
<210> 191
   <211> 28
   <212> PRT
   <213> Influenza virus
<400> 191
   Lys Lys Gly Thr Ser Tyr Pro Lys Leu Ser Lys Ser Tyr Thr His Asn
   1 5 10 15
   Lys Gly Lys Glu Val Leu Val Leu Trp Gly Val His
   20 25
<210> 192
   <211> 27
   <212> PRT
   <213> Influenza virus
<400> 192
   Lys Gly Thr Ser Tyr Pro Lys Leu Ser Lys Ser Tyr Thr His Asn Lys
   1 5 10 15
   Gly Lys Glu Val Leu Val Leu Trp Gly Val His
   20 25
<210> 193
   <211> 21
   <212> PRT
   <213> Influenza virus
<400> 193
   Lys Leu Ser Lys Ser Tyr Thr His Asn Lys Gly Lys Glu Val Leu Val
   1 5 10 15
   Leu Trp Gly Val His
   20
<210> 194
   <211> 18
   <212> PRT
   <213> Influenza virus
<400> 194
   Lys Ser Tyr Thr His Asn Lys Gly Lys Glu Val Leu Val Leu Trp Gly
   1 5 10 15
   Val His
<210> 195
   <211> 10
   <212> PRT
   <213> Influenza virus
<400> 195
   Lys Gly Val Thr Ala Ser Cys Ser His Lys
   1 5 10
<210> 196
   <211> 34
   <212> PRT
   <213> Influenza virus
<400> 196
   Lys Gly Val Thr Ala Ser Cys Ser His Lys Gly Arg Ser Ser Phe Tyr
   1 5 10 15
   Arg Asn Leu Leu Trp Leu Thr Glu Lys Asn Gly Leu Tyr Pro Asn Leu
   20 25 30
   Ser Lys
<210> 197
   <211> 27
   <212> PRT
   <213> Influenza virus
<400> 197
   Lys Gly Asn Ser Tyr Pro Lys Leu Ser Lys Ser Tyr Val Asn Asn Lys
   1 5 10 15
   Glu Lys Glu Val Leu Val Leu Trp Gly Ile His
   20 25
<210> 198
   <211> 8
   <212> PRT
   <213> Influenza virus
<400> 198
   Lys Glu Phe Asn His Leu Glu Lys
   1 5
<210> 199
   <211> 39
   <212> PRT
   <213> Influenza virus
<400> 199
   His Pro Pro Thr Ser Thr Asp Gln Gln Ser Leu Tyr Gln Asn Ala Asp
   1 5 10 15
   Ala Tyr Val Phe Val Gly Ser Ser Lys Tyr Asn Lys Lys Phe Lys Pro
   20 25 30
   Glu Ile Ala Thr Arg Pro Lys
   35
<210> 200
   <211> 31
   <212> PRT
   <213> Influenza virus
<400> 200
   His Pro Pro Thr Ser Thr Asp Gln Gln Ser Leu Tyr Gln Asn Ala Asp
   1               5                   10                  15
   Ala Tyr Val Phe Val Gly Ser Ser Lys Tyr Asn Lys Lys Phe Lys
               20                  25                  30
<210> 201
   <211> 31
   <212> PRT
   <213> Influenza virus
<400> 201
   His Glu Gly Lys Ser Ser Phe Tyr Arg Asn Leu Leu Trp Leu Thr Glu
   1               5                   10                  15
   Lys Glu Gly Ser Tyr Pro Lys Leu Lys Asn Ser Tyr Val Asn Lys
               20                  25                  30
<210> 202
   <211> 23
   <212> PRT
   <213> Influenza virus
<400> 202
   His Glu Gly Lys Ser Ser Phe Tyr Arg Asn Leu Leu Trp Leu Thr Glu
   1               5                   10                  15
   Lys Glu Gly Ser Tyr Pro Lys
               20
<210> 203
   <211> 26
   <212> PRT
   <213> Influenza virus
<400> 203
   His Lys Cys Asp Asn Glu Cys Met Glu Ser Val Arg Asn Gly Thr Tyr
   1               5                   10                  15
   Asp Tyr Pro Lys Tyr Ser Glu Glu Ser Lys
               20                  25
<210> 204
   <211> 12
   <212> PRT
   <213> Influenza virus
<400> 204
   Lys Glu Ser Ser Trp Pro Asn His Thr Val Thr Lys
   1               5                   10
<210> 205
   <211> 35
   <212> PRT
   <213> Influenza virus
<400> 205
   Lys Asn Leu Leu Trp Leu Thr Glu Lys Asn Gly Leu Tyr Pro Asn Leu
   1               5                   10                  15
   Ser Lys Ser Tyr Val Asn Asn Lys Glu Lys Glu Ile Leu Val Leu Trp
               20                  25                  30
   Gly Val His
           35
<210> 206
   <211> 27
   <212> PRT
   <213> Influenza virus
<220>
   <221> MOD_RES
   <222> (9)
   <223> lys or met
<220>
   <221> MOD_RES
   <222> (10)
   <223> asn or not present
<220>
   <221> MOD_RES
   <222> (16)
   <223> glu or gly
<220>
   <221> MOD_RES
   <222> (17)
   <223> not present or lys
<400> 206
   His Asn Gly Lys Ser Ser Phe Tyr Xaa Xaa Leu Leu Trp Leu Thr Xaa
   1               5                   10                  15
   Xaa Lys Asn Gly Leu Tyr Pro Asn Leu Ser Lys
               20                  25
<210> 207
   <211> 17
   <212> PRT
   <213> Influenza virus
<400> 207
   His Asn Gly Lys Ser Ser Phe Tyr Lys Asn Leu Leu Trp Leu Thr Glu
   1 5 10 15
   Lys
<210> 208
   <211> 55
   <212> PRT
   <213> Influenza virus
<400> 208
   His Thr Val Thr Lys Gly Val Thr Ala Ser Cys Ser His Asn Gly Lys
   1 5 10 15
   Ser Ser Phe Tyr Lys Asn Leu Leu Trp Leu Thr Glu Lys Asn Gly Leu
   20 25 30
   Tyr Pro Asn Leu Ser Lys Ser Tyr Val Asn Asn Lys Glu Lys Glu Val
   35 40 45
   Leu Val Leu Trp Gly Val His
   50 55
<210> 209
   <211> 38
   <212> PRT
   <213> Influenza virus
<220>
   <221> MOD_RES
   <222> (5)
   <223> lys or gly
<220>
   <221> MOD_RES
   <222> (8)
   <223> thr or ser
<220>
   <221> MOD_RES
   <222> (21)
   <223> lys or met
<220>
   <221> MOD_RES
   <222> (22)
   <223> asn or not present
<220>
   <221> MOD_RES
   <222> (28)
   <223> glu or gly
<220>
   <221> MOD_RES
   <222> (30)
   <223> asn present or not or lys
<400> 209
   His Thr Val Thr Xaa Gly Val Xaa Ala Ser Cys Ser His Asn Gly Lys
   1               5                   10                  15
   Ser Ser Phe Tyr Xaa Xaa Leu Leu Trp Leu Thr Xaa Lys Xaa Gly Leu
               20                  25                  30
   Tyr Pro Asn Leu Ser Lys
           35
<210> 210
   <211> 29
   <212> PRT
   <213> Influenza virus
<400> 210
   His Thr Val Thr Lys Gly Val Thr Ala Ser Cys Ser His Asn Gly Lys
   1               5                   10                  15
   Ser Ser Phe Tyr Lys Asn Leu Leu Trp Leu Thr Glu Lys
               20                  25
<210> 211
   <211> 48
   <212> PRT
   <213> Influenza virus
<400> 211
   Lys Tyr Val Arg Ser Thr Lys Leu Arg Met Val Thr Gly Leu Arg Asn
   1               5                   10                  15
   Ile Pro Ser Ile Gln Ser Arg Gly Leu Phe Gly Ala Ile Ala Gly Phe
               20                  25                  30
   Ile Glu Gly Gly Trp Thr Gly Met Ile Asp Gly Trp Tyr Gly Tyr His
           35                  40                  45
<210> 212
   <211> 43
   <212> PRT
   <213> Influenza virus
<400> 212
   His Gln Asn Glu Gln Gly Ser Gly Tyr Ala Ala Asp Gln Lys Ser Thr
   1 5 10 15
   Gln Asn Ala Ile Asn Gly Ile Thr Asn Lys Val Asn Ser Ile Ile Glu
   20 25 30
   Lys Met Asn Thr Gln Phe Thr Ala Val Gly Lys
   35 40
<210> 213
   <211> 33
   <212> PRT
   <213> Influenza virus
<400> 213
   His Gln Asn Glu Gln Gly Ser Gly Tyr Ala Ala Asp Gln Lys Ser Thr
   1 5 10 15
   Gln Asn Ala Ile Asn Gly Ile Thr Asn Lys Val Asn Ser Ile Ile Glu
   20 25 30
   Lys
<210> 214
   <211> 26
   <212> PRT
   <213> Influenza virus
<400> 214
   His Gln Asn Glu Gln Gly Ser Gly Tyr Ala Ala Asp Gln Lys Ser Thr
   1 5 10 15
   Gln Asn Ala Ile Asn Gly Ile Thr Asn Lys
   20 25
<210> 215
   <211> 23
   <212> PRT
   <213> Influenza virus
<400> 215
   His Ser Gly Ala Arg Ser Phe Tyr Arg Asn Leu Leu Trp Ile Val Lys
   1 5 10 15
   Lys Gly Asn Ser Tyr Pro Lys
   20
<210> 216
   <211> 26
   <212> PRT
   <213> Influenza virus
<400> 216
   His Ser Gly Ala Arg Ser Phe Tyr Arg Asn Leu Leu Trp Ile Val Lys
   1 5 10 15
   Lys Gly Asn Ser Tyr Pro Lys Leu Asn Lys
   20 25
<210> 217
   <211> 32
   <212> PRT
   <213> Influenza virus
<400> 217
   His Ser Gly Ala Arg Ser Phe Tyr Arg Asn Leu Leu Trp Ile Val Lys
   1 5 10 15
   Lys Gly Asn Ser Tyr Pro Lys Leu Asn Lys Ser Tyr Thr Asn Asp Lys
   20 25 30
<210> 218
   <211> 34
   <212> PRT
   <213> Influenza virus
<400> 218
   His Ser Gly Ala Arg Ser Phe Tyr Arg Asn Leu Leu Trp Ile Val Lys
   1 5 10 15
   Lys Gly Asn Ser Tyr Pro Lys Leu Asn Lys Ser Tyr Thr Asn Asp Lys
   20 25 30
   Gly Lys
<210> 219
   <211> 16
   <212> PRT
   <213> Influenza virus
<400> 219
   His Thr Val Ser Lys Gly Val Thr Thr Ser Cys Ser His Asn Gly Lys
   1 5 10 15
<210> 220
   <211> 12
   <212> PRT
   <213> Influenza virus
<400> 220
   Lys Ala Thr Ser Trp Pro Asn His Glu Thr Thr Lys
   1 5 10
<210> 221
   <211> 12
   <212> PRT
   <213> Influenza virus
<400> 221
   Lys Gln Val Thr Thr Ser Cys Ser His Asn Gln Lys
   1 5 10
<210> 222
   <211> 27
   <212> PRT
   <213> Influenza virus
<400> 222
   Lys Gly Asn Ser Tyr Pro Lys Leu Asn Lys Ser Tyr Thr Asn Asp Lys
   1 5 10 15
   Gly Lys Glu Val Leu Val Ile Trp Gly Val His
   20 25
<210> 223
   <211> 21
   <212> PRT
   <213> Influenza virus
<400> 223
   Lys Leu Asn Lys Ser Tyr Thr Asn Asp Lys Gly Lys Glu Val Leu Val
   1 5 10 15
   Ile Trp Gly Val His
   20
<210> 224
   <211> 18
   <212> PRT
   <213> Influenza virus
<400> 224
   Lys Ser Tyr Thr Asn Asp Lys Gly Lys Glu Val Leu Val Ile Trp Gly
   1 5 10 15
   Val His
<210> 225
   <211> 35
   <212> PRT
   <213> Influenza virus
<220>
   <221> MOD_RES
   <222> (16)
   <223> glu or gln
<220>
   <221> MOD_RES
   <222> (29)
   <223> val or ala
<400> 225
   His Asn Gln Lys Ser Ser Phe Tyr Arg Asn Leu Leu Trp Leu Thr Xaa
   1               5                   10                  15
   Lys Asn Gly Leu Tyr Pro Asn Leu Ser Lys Ser Tyr Xaa Ala Asn Asn
               20                  25                  30
   Lys Glu Lys
           35
<210> 226
   <211> 16
   <212> PRT
   <213> Influenza virus
<400> 226
   His Pro Ile Thr Ile Gly Glu Cys Pro Lys Tyr Val Arg Ser Ala Lys
   1               5                   10                  15
<210> 227
   <211> 43
   <212> PRT
   <213> Influenza virus
<400> 227
   His Gln Asn Glu Gln Gly Ser Gly Tyr Ala Ala Asp Gln Lys Ser Thr
   1               5                   10                  15
   Gln Asn Ala Ile Asn Gly Ile Thr Asn Lys Val Asn Ser Val Ile Glu
               20                  25                  30
   Lys Met Asn Thr Gln Phe Thr Ala Val Gly Lys
           35                  40
<210> 228
   <211> 33
   <212> PRT
   <213> Influenza virus
<400> 228
   His Gln Asn Glu Gln Gly Ser Gly Tyr Ala Ala Asp Gln Lys Ser Thr
   1 5 10 15
   Gln Asn Ala Ile Asn Gly Ile Thr Asn Lys Val Asn Ser Val Ile Glu
   20 25 30
   Lys
<210> 229
   <211> 34
   <212> PRT
   <213> Influenza virus
<400> 229
   His Asn Gly Lys Ser Ser Phe Tyr Arg Asn Leu Leu Trp Leu Thr Glu
   1 5 10 15
   Lys Asn Gly Leu Tyr Pro Asn Leu Ser Lys Ser Tyr Val Asn Asn Lys
   20 25 30
   Glu Lys
<210> 230
   <211> 11
   <212> PRT
   <213> Influenza virus
<400> 230
   Lys His Phe Glu Lys Val Lys Ile Leu Pro Lys
   1 5 10
<210> 231
   <211> 14
   <212> PRT
   <213> Influenza virus
<400> 231
   Lys His Leu Leu Ser Ser Val Lys His Phe Glu Lys Val Lys
   1 5 10
<210> 232
   <211> 13
   <212> PRT
   <213> Influenza virus
<220>
   <221> MOD_RES
   <222> (3)
   <223> lys, gln or met
<220>
   <221> MOD_RES
   <222> (4)
   <223> asp or asn
<400> 232
   His Ala Xaa Xaa Ile Leu Glu Lys Thr His Asn Gly Lys
   1 5 10
<210> 233
   <211> 16
   <212> PRT
   <213> Influenza virus
<220>
   <221> MOD_RES
   <222> (3)
   <223> lys, gln or met
<220>
   <221> MOD_RES
   <222> (4)
   <223> asp or asn
<220>
   <221> MOD_RES
   <222> (16)
   <223> lys or arg
<400> 233
   His Ala Xaa Xaa Ile Leu Glu Lys Thr His Asn Gly Lys Leu Cys Xaa
   1 5 10 15
<210> 234
   <211> 19
   <212> PRT
   <213> Influenza virus
<400> 234
   His Asn Val His Pro Leu Thr Ile Gly Glu Cys Pro Lys Tyr Val Lys
   1 5 10 15
   Ser Glu Lys
<210> 235
   <211> 16
   <212> PRT
   <213> Influenza virus
<400> 235
   His Pro Leu Thr Ile Gly Glu Cys Pro Lys Tyr Val Lys Ser Glu Lys
   1 5 10 15
<210> 236
   <211> 18
   <212> PRT
   <213> Influenza virus
<400> 236
   Lys His Leu Leu Ser Ser Val Lys His Phe Glu Lys Val Lys Ile Leu
   1 5 10 15
   Pro Lys
<210> 237
   <211> 38
   <212> PRT
   <213> Influenza virus
<400> 237
   Lys Arg Gln Ser Ser Gly Ile Met Lys Thr Glu Gly Thr Leu Glu Asn
   1 5 10 15
   Cys Glu Thr Lys Cys Gln Thr Pro Leu Gly Ala Ile Asn Thr Thr Leu
   20 25 30
   Pro Phe His Asn Val His
   35
<210> 238
   <211> 27
   <212> PRT
   <213> Influenza virus
<220>
   <221> MOD_RES
   <222> (7)
   <223> val or ile
<220>
   <221> MOD_RES
   <222> (10)
   <223> gly or arg
<220>
   <221> MOD_RES
   <222> (26)
   <223> val or ile
<400> 238
   Lys Gly Ser Asn Tyr Pro Xaa Ala Lys Xaa Ser Tyr Asn Asn Thr Ser
   1 5 10 15
   Gly Glu Gln Met Leu Ile Ile Trp Gln Xaa His
   20 25
<210> 239
   <211> 36
   <212> PRT
   <213> Influenza virus
<400> 239
   His Thr Thr Leu Gly Gln Ser Arg Ala Cys Ala Val Ser Gly Asn Pro
   1 5 10 15
   Ser Phe Phe Arg Asn Met Val Trp Leu Thr Glu Lys Gly Ser Asn Tyr
   20 25 30
   Pro Val Ala Lys
   35
<210> 240
   <211> 7
   <212> PRT
   <213> Influenza virus
<400> 240
   Lys His Phe Glu Lys Val Lys
   1 5
<210> 241
   <211> 38
   <212> PRT
   <213> Influenza virus
<400> 241
   Lys Ile Ser Lys Arg Gly Ser Ser Gly Ile Met Lys Thr Glu Gly Thr
   1 5 10 15
   Leu Glu Asn Cys Glu Thr Lys Cys Gln Thr Pro Leu Gly Ala Ile Asn
   20 25 30
   Thr Thr Leu Pro Phe His
   35
<210> 242
   <211> 35
   <212> PRT
   <213> Influenza virus
<400> 242
   Lys Arg Gly Ser Ser Gly Ile Met Lys Thr Glu Gly Thr Leu Glu Asn
   1               5                   10                  15
   Cys Glu Thr Lys Cys Gln Thr Pro Leu Gly Ala Ile Asn Thr Thr Leu
               20                  25                  30
   Pro Phe His
           35
<210> 243
   <211> 27
   <212> PRT
   <213> Influenza virus
<400> 243
   Lys Thr Glu Gly Thr Leu Glu Asn Cys Glu Thr Lys Cys Gln Thr Pro
   1               5                   10                  15
   Leu Gly Ala Ile Asn Thr Thr Leu Pro Phe His
               20                  25
<210> 244
   <211> 38
   <212> PRT
   <213> Influenza virus
<400> 244
   Lys Ile Ser Lys Arg Gly Ser Ser Gly Ile Met Lys Thr Glu Gly Thr
   1               5                   10                  15
   Leu Glu Asn Cys Glu Thr Lys Cys Gln Thr Pro Leu Gly Ala Ile Asn
               20                  25                  30
   Thr Thr Leu Pro Phe His
           35
<210> 245
   <211> 30
   <212> PRT
   <213> Influenza virus
<220>
   <221> MOD_RES
   <222> (29)
   <223> val or ile
<400> 245
   Lys Thr Glu Gly Thr Leu Glu Asn Cys Glu Thr Lys Cys Gln Thr Pro
   1               5                   10                  15
   Leu Gly Ala Ile Asn Thr Thr Leu Pro Phe His Asn Xaa His
               20                  25                  30
<210> 246
   <211> 38
   <212> PRT
   <213> Influenza virus
<400> 246
   Lys Ile Ser Lys Arg Gly Ser Ser Gly Ile Met Lys Thr Glu Gly Thr
   1 5 10 15
   Leu Glu Asn Cys Glu Thr Lys Cys Gln Thr Pro Leu Gly Ala Ile Asn
   20 25 30
   Thr Thr Leu Pro Phe His
   35
<210> 247
   <211> 27
   <212> PRT
   <213> Influenza virus
<220>
   <221> MOD_RES
   <222> (2)
   <223> glu or gly
<400> 247
   Lys Xaa Ser Asn Tyr Pro Val Ala Lys Gly Ser Tyr Asn Asn Thr Ser
   1 5 10 15
   Gly Glu Gln Met Leu Ile Ile Trp Gly Val His
   20 25
<210> 248
   <211> 16
   <212> PRT
   <213> Influenza virus
<400> 248
   His Pro Leu Thr Ile Gly Glu Cys Pro Lys Tyr Val Lys Ser Glu Lys
   1 5 10 15
<210> 249
   <211> 16
   <212> PRT
   <213> Influenza virus
<400> 249
   Lys Cys Gln Thr Pro Leu Gly Ala Ile Lys Thr Thr Leu Pro Phe His
   1 5 10 15
<210> 250
   <211> 58
   <212> PRT
   <213> Influenza virus
<220>
   <221> MOD_RES
   <222> (21)
   <223> phe or ile
<220>
   <221> MOD_RES
   <222> (47)
   <223> asn or ser
<400> 250
   His His Ser Asn Asp Gln Gly Ser Gly Tyr Ala Ala Asp Lys Glu Ser
   1               5                   10                  15
   Thr Gln Lys Ala Xaa Asp Gly Ile Thr Asn Lys Val Asn Ser Val Ile
               20                  25                  30
   Glu Lys Met Asn Thr Gln Phe Glu Ala Val Gly Lys Leu Phe Xaa Asn
           35                  40                  45
   Leu Glu Lys Leu Glu Asn Leu Asn Lys Lys
       50                  55
<210> 251
   <211> 57
   <212> PRT
   <213> Influenza virus
<220>
   <221> MOD_RES
   <222> (20)
   <223> phe or ile
<220>
   <221> MOD_RES
   <222> (46)
   <223> asn or ser
<400> 251
   His Ser Asn Asp Gln Gly Ser Gly Tyr Ala Ala Asp Lys Glu Ser Thr
   1               5                   10                  15
   Gln Lys Ala Xaa Asp Gly Ile Thr Asn Lys Val Asn Ser Val Ile Glu
               20                  25                  30
   Lys Met Asn Thr Gln Phe Glu Ala Val Gly Lys Leu Phe Xaa Asn Leu
           35                  40                  45
   Glu Lys Leu Glu Asn Leu Asn Lys Lys
       50                  55
<210> 252
   <211> 26
   <212> PRT
   <213> Influenza virus
<220>
   <221> MOD_RES
   <222> (20)
   <223> phe or ile
<400> 252
   His Ser Asn Asp Gln Gly Ser Gly Tyr Ala Ala Asp Lys Glu Ser Thr
   1 5 10 15
   Gln Lys Ala Xaa Asp Gly Ile Thr Asn Lys
   20 25
<210> 253
   <211> 21
   <212> PRT
   <213> Influenza virus
<400> 253
   His Asp Ser Asn Val Arg Asn Leu Tyr Asp Lys Val Arg Met Gln Leu
   1 5 10 15
   Arg Asp Asn Ala Lys
   20
<210> 254
   <211> 30
   <212> PRT
   <213> Influenza virus
<400> 254
   His Lys Cys Asp Asp Glu Cys Met Asn Ser Val Lys Asn Gly Thr Tyr
   1 5 10 15
   Asp Tyr Pro Lys Leu Asn Arg Asn Glu Ile Lys Gly Val Lys
   20 25 30
<210> 255
   <211> 27
   <212> PRT
   <213> Influenza virus
<400> 255
   His Lys Cys Asp Asp Glu Cys Met Asn Ser Val Lys Asn Gly Thr Tyr
   1 5 10 15
   Asp Tyr Pro Lys Leu Asn Arg Asn Glu Ile Lys
   20 25
<210> 256
   <211> 20
   <212> PRT
   <213> Influenza virus
<400> 256
   His Lys Cys Asp Asp Glu Cys Met Asn Ser Val Lys Asn Gly Thr Tyr
   1               5                   10                  15
   Asp Tyr Pro Lys
               20
<210> 257
   <211> 12
   <212> PRT
   <213> Influenza virus
<400> 257
   His Lys Cys Asp Asp Glu Cys Met Asn Ser Val Lys
   1               5                   10
<210> 258
   <211> 27
   <212> PRT
   <213> Influenza virus

<400> 258
   Lys Gly Ser Asn Tyr Pro Val Ala Lys Gly Ser Tyr Asn Asn Thr Asn
   1               5                   10                  15
   Gly Glu Gln Ile Leu Ile Ile Trp Gly Val His
               20                  25
<210> 259
   <211> 43
   <212> PRT
   <213> Influenza virus
<400> 259
   His Ser Asn Asp Gln Gly Ser Gly Tyr Ala Ala Asp Lys Glu Ser Thr
   1               5                   10                  15
   Gln Lys Ala Val Asp Gly Ile Thr Asn Lys Val Asn Ser Val Ile Glu
               20                  25                  30
   Lys Met Asn Thr Gln Phe Glu Ala Val Gly Lys
           35                  40
<210> 260
   <211> 35
   <212> PRT
   <213> Influenza virus
<400> 260
   Lys Arg Gly Ser Ser Gly Ile Met Lys Thr Glu Gly Thr Leu Glu Asn
   1 5 10 15
   Cys Glu Thr Lys Cys Gln Thr Pro Leu Gly Ala Ile Asn Thr Thr Leu
   20 25 30
   Pro Phe His
   35
<210> 261
   <211> 16
   <212> PRT
   <213> Influenza virus
<400> 261
   His Pro Leu Thr Ile Gly Glu Cys Pro Lys Tyr Val Lys Ser Glu Lys
   1 5 10 15
<210> 262
   <211> 16
   <212> PRT
   <213> Influenza virus
<400> 262
   His Ala Lys Asp Ile Leu Glu Lys Thr His Asn Gly Lys Leu Cys Lys
   1 5 10 15
<210> 263
   <211> 25
   <212> PRT
   <213> Influenza virus
<400> 263
   His Asp Val Tyr Arg Asp Glu Ala Leu Asn Asn Arg Phe Gln Ile Lys
   1 5 10 15
   Gly Val Glu Leu Lys Ser Gly Tyr Lys
   20 25
<210> 264
   <211> 19
   <212> PRT
   <213> Influenza virus
<400> 264
   His Thr Ile Asp Leu Thr Asp Ser Glu Met Asn Lys Leu Phe Glu Arg
   1 5 10 15
   Thr Arg Lys
<210> 265
   <211> 7
   <212> PRT
   <213> Influenza virus
<400> 265
   Lys Phe His Gln Ile Glu Lys
   1 5
<210> 266
   <211> 11
   <212> PRT
   <213> Influenza virus
<220>
   <221> MOD_RES
   <222> (8)
   <223> gly or gln
<400> 266
   Lys Thr Asn Glu Lys Phe His Xaa Ile Glu Lys
   1 5 10
<210> 267
   <211> 14
   <212> PRT
   <213> Influenza virus
<220>
   <221> MOD_RES
   <222> (5)
   <223> val or leu
<400> 267
   Lys Leu Asn Arg Xaa Ile Glu Lys Thr Asn Glu Lys Phe His
   1 5 10
<210> 268
   <211> 25
   <212> PRT
   <213> Influenza virus
<400> 268
   His Gln Ile Glu Lys Glu Phe Ser Glu Val Glu Gly Arg Ile Gln Asp
   1 5 10 15
   Leu Glu Lys Tyr Val Glu Asp Thr Lys
   20 25
<210> 269
   <211> 8
   <212> PRT
   <213> Influenza virus
<400> 269
   Lys Ile Cys Asn Asn Pro His Lys
   1 5
<210> 270
   <211> 14
   <212> PRT
   <213> Influenza virus
<400> 270
   Lys Leu Asn Arg Val Ile Lys Lys Thr Asn Glu Lys Phe His
   1 5 10
<210> 271
   <211> 24
   <212> PRT
   <213> Influenza virus
<220>
   <221> MOD_RES
   <222> (3)
   <223> ile or val
<220>
   <221> MOD_RES
   <222> (17)
   <223> gly or gln
<220>
   <221> MOD_RES
   <222> (20)
   <223> arg or lys
<220>
   <221> MOD_RES
   <222> (22)
   <223> gln or gly
<400> 271
   His Asp Xaa Tyr Arg Asp Glu Ala Leu Asn Asn Arg Phe Gln Ile Lys
   1 5 10 15
   Xaa Val Glu Xaa Ser Xaa Tyr Lys
   20
<210> 272
   <211> 25
   <212> PRT
   <213> Influenza virus
<400> 272
   His Gln Ile Glu Lys Glu Phe Ser Glu Val Glu Gly Arg Ile Gln Asp
   1 5 10 15
   Leu Glu Lys Tyr Val Glu Asp Thr Lys
   20 25
<210> 273
   <211> 25
   <212> PRT
   <213> Influenza virus
<400> 273
   Lys Tyr Val Glu Asp Thr Lys Ile Asp Leu Trp Ser Tyr Asn Ala Glu
   1 5 10 15
   Leu Leu Val Ala Leu Glu Asn Gln His
   20 25
<210> 274
   <211> 49
   <212> PRT
   <213> Influenza virus
<400> 274
   Lys Tyr Val Lys Gln Asn Ser Leu Lys Leu Ala Thr Gly Met Arg Asn
   1 5 10 15
   Val Pro Glu Lys Gln Thr Arg Gly Leu Phe Gly Ala Ile Ala Gly Phe
   20 25 30
   Ile Glu Asn Gly Trp Glu Gly Met Ile Asp Gly Trp Tyr Gly Phe Arg
   35 40 45
   His
<210> 275
   <211> 39
   <212> PRT
   <213> Influenza virus
<400> 275
   Lys Glu Phe Ser Glu Val Glu Gly Arg Ile Gln Asp Leu Glu Lys Tyr
    1               5                  10                  15
   Val Glu Asp Thr Lys Ile Asp Leu Trp Ser Tyr Asn Ala Glu Leu Leu
               20                  25                  30
   Val Ala Leu Glu Asn Gln His
           35
<210> 276
   <211> 33
   <212> PRT
   <213> Influenza virus
<220>
   <221> MOD_RES
   <222> (4)
   <223> ser or glu
<220>
   <221> MOD_RES
   <222> (5)
   <223> glu or gln
<220>
   <221> MOD_RES
   <222> (7)
   <223> thr or ser
<220>
   <221> MOD_RES
   <222> (9)
   <223> gln or tyr
<220>
   <221> MOD_RES
   <222> (13)
   <223> leu or gln
<220>
   <221> MOD_RES
   <222> (18)
   <223> ala or asn
<220>
   <221> MOD_RES
   <222> (20)
   <223> ile or leu
<220>
   <221> MOD_RES
   <222> (22)
   <223> gln or gly
<220>
   <221> MOD_RES
   <222> (24)
   <223> asn or thr
<220>
   <221> MOD_RES
   <222> (25)
   <223> gly or asn
<220>
   <221> MOD_RES
   <222> (27)
   <223> leu or val
<220>
   <221> MOD_RES
   <222> (29)
   <223> arg or ser
<220>
   <221> MOD_RES
   <222> (32)
   <223> glu or cys
<400> 276
   His Gln Asn Xaa Xaa Gly Xaa Gly Xaa Ala Ala Asp Xaa Lys Ser Thr
   1               5                   10                  15
   Gln Xaa Ala Xaa Asp Xaa Ile Xaa Xaa Lys Xaa Asn Xaa Val Ile Xaa
               20                  25                  30
   Lys
<210> 277
   <211> 18
   <212> PRT
   <213> Influenza virus
<220>
   <221> MOD_RES
   <222> (4)
   <223> gly or gln
<220>
   <221> MOD_RES
   <222> (6)
   <223> gln or arg
<220>
   <221> MOD_RES
   <222> (14)
   <223> val or ile
<220>
   <221> MOD_RES
   <222> (17)
   <223> ser or thr
<400> 277
   His Cys Asp Xaa Phe Xaa Asn Glu Lys Trp Asp Leu Phe Xaa Glu Arg
   1 5 10 15
   Xaa Lys
<210> 278
   <211> 20
   <212> PRT
   <213> Influenza virus
<400> 278
   His Thr Ile Asp Leu Thr Asp Ser Glu Met Asn Lys Lys Leu Phe Glu
   1 5 10 15
   Arg Thr Arg Lys
   20
<210> 279
   <211> 28
   <212> PRT
   <213> Influenza virus
<400> 279
   Lys Ser Gly Ser Thr Tyr Pro Val Leu Lys Val Thr Met Pro Asn Asn
   1 5 10 15
   Asp Asn Phe Asp Lys Leu Tyr Ile Trp Gly Val His
   20 25
<210> 280
   <211> 34
   <212> PRT
   <213> Influenza virus
<400> 280
   Lys Leu Asn Trp Leu Thr Lys Ser Gly Asn Thr Tyr Pro Val Leu Asn
   1 5 10 15
   Val Thr Met Pro Asn Asn Asp Asn Phe Asp Lys Leu Val Ile Trp Gly
   20 25 30
   Val His
<210> 281
   <211> 19
   <212> PRT
   <213> Influenza virus
<400> 281
   His Thr Ile Asp Leu Thr Asp Ser Glu Met Asn Lys Leu Phe Glu Lys
   1 5 10 15
   Thr Arg Lys
<210> 282
   <211> 18
   <212> PRT
   <213> Influenza virus
<400> 282
   Lys Leu Asn Arg Leu Ile Glu Lys Thr Asn Glu Lys Phe His Gln Thr
   1 5 10 15
   Glu Lys
<210> 283
   <211> 47
   <212> PRT
   <213> Influenza virus
<400> 283
   His Thr Gly Lys Ser Ser Val Met Arg Ser Asp Ala Pro Ile Asp Phe
   1 5 10 15
   Cys Asn Ser Glu Cys Ile Thr Pro Asn Gln Ser Ile Pro Asn Asp Lys
   20 25 30
   Pro Phe Gln Asn Val Asn Lys Ile Thr Tyr Gly Ala Cys Pro Lys
   35 40 45
<210> 284
   <211> 39
   <212> PRT
   <213> Influenza virus
<400> 284
   His Thr Gly Lys Ser Ser Val Met Arg Ser Asp Ala Pro Ile Asp Phe
   1 5 10 15
   Cys Asn Ser Glu Cys Ile Thr Pro Asn Gln Ser Ile Pro Asn Asp Lys
   20 25 30
   Pro Phe Gln Asn Val Asn Lys
   35
<210> 285
   <211> 33
   <212> PRT
   <213> Influenza virus
<400> 285
   His Pro Ser Thr Asp Ser Asp Gln Thr Ser Leu Tyr Val Arg Ala Ser
   1 5 10 15
   Gly Arg Val Thr Val Ser Thr Lys Arg Ser Gln Gln Thr Val Ile Pro
   20 25 30
   Lys
<210> 286
   <211> 25
   <212> PRT
   <213> Influenza virus
<400> 286
   Lys Tyr Val Glu Asp Thr Lys Ile Asp Leu Trp Ser Tyr Asn Ala Glu
   1 5 10 15
   Leu Leu Val Ala Leu Glu Asn Gln His
   20 25
<210> 287
   <211> 26
   <212> PRT
   <213> Influenza virus
<400> 287
   Lys Leu Phe Glu Arg Thr Arg Lys Gln Leu Arg Glu Asn Ala Glu Asp
   1 5 10 15
   Met Gly Asn Gly Cys Phe Lys Ile Tyr His
   20 25
<210> 288
   <211> 16
   <212> PRT
   <213> Influenza virus
<400> 288
   Lys Arg Arg Ser Ile Lys Ser Phe Phe Ser Arg Leu Asn Trp Leu His
   1 5 10 15
<210> 289
   <211> 16
   <212> PRT
   <213> Influenza virus
<220>
   <221> MOD_RES
   <222> (12)
   <223> val or arg
<400> 289
   His Pro Val Thr Ile Gly Glu Cys Pro Lys Tyr Xaa Lys Ser Thr Lys
   1               5                   10                  15
<210> 290
   <211> 30
   <212> PRT
   <213> Influenza virus
<400> 290
   Lys Gly Asn Ser Tyr Pro Lys Leu Ser Lys Leu Ser Lys Ser Tyr Ile
   1               5                   10                  15
   Ile Asn Lys Lys Lys Glu Val Leu Val Ile Trp Gly Ile His
               20                  25                  30
<210> 291
   <211> 24
   <212> PRT
   <213> Influenza virus
<220>
   <221> MOD_RES
   <222> (9)
   <223> val or tyr
<400> 291
   Lys Leu Ser Lys Leu Ser Lys Ser Xaa Ile Ile Asn Lys Lys Lys Glu
   1               5                   10                  15
   Val Leu Val Ile Trp Gly Ile His
               20
<210> 292
   <211> 21
   <212> PRT
   <213> Influenza virus
<220>
   <221> MOD_RES
   <222> (6)
   <223> val or tyr
<400> 292
   Lys Leu Ser Lys Ser Xaa Ile Ile Asn Lys Lys Lys Glu Val Leu Val
   1               5                   10                  15
   Ile Trp Gly Ile His
               20
<210> 293
   <211> 46
   <212> PRT
   <213> Plasmodium falciparum
<400> 293
   Lys Glu Glu Glu Glu Lys Glu Lys Glu Lys Glu Lys Glu Lys Glu Glu
   1 5 10 15
   Lys Glu Lys Glu Glu Lys Glu Lys Glu Glu Lys Glu Lys Glu Lys Glu
   20 25 30
   Glu Lys Glu Lys Glu Lys Glu Glu Lys Glu Glu Glu Lys Lys
   35 40 45
<210> 294
   <211> 48
   <212> PRT
   <213> Plasmodium falciparum
<400> 294
   Lys Glu Glu Glu Glu Lys Glu Lys Glu Lys Glu Lys Glu Lys Glu Glu
   1 5 10 15
   Lys Glu Lys Glu Glu Lys Glu Lys Glu Glu Lys Glu Lys Glu Lys Glu
   20 25 30
   Glu Lys Glu Lys Glu Lys Glu Glu Lys Glu Glu Glu Lys Lys Glu Lys
   35 40 45
<210> 295
   <211> 47
   <212> PRT
   <213> Plasmodium falciparum
<400> 295
   Lys Glu Glu Glu Glu Lys Glu Lys Glu Lys Glu Lys Glu Lys Glu Glu
   1 5 10 15
   Lys Glu Lys Glu Glu Lys Glu Lys Glu Lys Glu Glu Lys Glu Lys Glu
   20 25 30
   Glu Lys Glu Lys Glu Glu Lys Glu Glu Lys Glu Glu Glu Lys Lys
   35 40 45
<210> 296
   <211> 10
   <212> PRT
   <213> Plasmodium falciparum
<400> 296
   Lys Glu Glu Glu Glu Lys Glu Lys Glu Lys
   1 5 10
<210> 297
   <211> 19
   <212> PRT
   <213> Plasmodium falciparum
<400> 297
   His Lys Lys Leu Ile Lys Ala Leu Lys Lys Asn Ile Glu Ser Ile Gln
   1 5 10 15
   Asn Lys Lys
<210> 298
   <211> 19
   <212> PRT
   <213> Plasmodium falciparum
<400> 298
   His Lys Lys Leu Ile Lys Ala Leu Lys Lys Asn Ile Glu Ser Ile Gln
   1 5 10 15
   Asn Lys Met
<210> 299
   <211> 10
   <212> PRT
   <213> Plasmodium falciparum
<400> 299
   His Lys Lys Leu Ile Lys Ala Leu Lys Lys
   1 5 10
<210> 300
   <211> 9
   <212> PRT
   <213> Plasmodium falciparum
<400> 300
   His Lys Lys Leu Ile Lys Ala Leu Lys
   1 5
<210> 301
   <211> 23
   <212> PRT
   <213> Plasmodium falciparum
<400> 301
   Lys Ala Thr Tyr Ser Phe Val Asn Thr Lys Lys Lys Ile Ile Ser Leu
   1 5 10 15
   Lys Ser Gln Gly His Lys Lys
   20
<210> 302
   <211> 22
   <212> PRT
   <213> Plasmodium falciparum
<400> 302
   Lys Ala Thr Tyr Ser Phe Val Asn Thr Lys Lys Lys Ile Ile Ser Leu
   1 5 10 15
   Lys Ser Gln Gly His Lys
   20
<210> 303
   <211> 21
   <212> PRT
   <213> Plasmodium falciparum
<400> 303
   Lys Ala Thr Tyr Ser Phe Val Asn Thr Lys Lys Lys Ile Ile Ser Leu
   1 5 10 15
   Lys Ser Gln Gly His
   20
<210> 304
   <211> 29
   <212> PRT
   <213> Plasmodium falciparum
<400> 304
   His Thr Tyr Val Lys Gly Lys Lys Ala Pro Ser Asp Pro Gln Cys Ala
   1 5 10 15
   Asp Ile Lys Glu Glu Cys Lys Glu Leu Leu Lys Glu Lys
   20 25
<210> 305
   <211> 11
   <212> PRT
   <213> Plasmodium falciparum
<400> 305
   Lys Ile Ile Ser Leu Lys Ser Gln Gly His Lys
   1 5 10
<210> 306
   <211> 21
   <212> PRT
   <213> Plasmodium falciparum
<400> 306
   Lys Lys Lys Lys Phe Glu Pro Leu Lys Asn Gly Asn Val Ser Glu Thr
   1               5                   10                  15
   Ile Lys Leu Ile His
               20
<210> 307
   <211> 20
   <212> PRT
   <213> Plasmodium falciparum
<400> 307
   Lys Lys Lys Phe Glu Pro Leu Lys Asn Gly Asn Val Ser Glu Thr Ile
   1               5                   10                  15
   Lys Leu Ile His
               20
<210> 308
   <211> 19
   <212> PRT
   <213> Plasmodium falciparum
<400> 308
   Lys Lys Phe Glu Pro Leu Lys Asn Gly Asn Val Ser Glu Thr Ile Lys
   1               5                   10                  15
   Leu Ile His
<210> 309
   <211> 13
   <212> PRT
   <213> Plasmodium falciparum
<400> 309
   Lys Asn Gly Asn Val Ser Glu Thr Ile Lys Leu Ile His
   1               5                   10
<210> 310
   <211> 11
   <212> PRT
   <213> Plasmodium falciparum
<400> 310
   Lys Leu Ile His Leu Gly Asn Lys Asp Lys Lys
   1 5 10
<210> 311
   <211> 36
   <212> PRT
   <213> Plasmodium falciparum
<400> 311
   Lys Val Lys Lys Ile Gly Val Thr Leu Lys Lys Phe Glu Pro Leu Lys
   1 5 10 15
   Asn Gly Asn Val Ser Glu Thr Ile Lys Leu Ile His Leu Gly Asn Lys
   20 25 30
   Asp Lys Lys His
   35
<210> 312
   <211> 59
   <212> PRT
   <213> Plasmodium falciparum
<400> 312
   His Leu Ile Tyr Lys Asn Lys Ser Tyr Asn Pro Leu Leu Leu Ser Cys
   1 5 10 15
   Val Lys Lys Met Asn Met Leu Lys Glu Asn Val Asp Tyr Ile Gln Asn
   20 25 30
   Gln Asn Leu Phe Lys Glu Leu Met Asn Gln Lys Ala Thr Tyr Ser Phe
   35 40 45
   Val Asn Thr Lys Lys Lys Ile Ile Ser Leu Lys
   50 55
<210> 313
   <211> 52
   <212> PRT
   <213> Plasmodium falciparum
<400> 313
   His Leu Ile Tyr Lys Asn Lys Ser Tyr Asn Pro Leu Leu Leu Ser Cys
   1 5 10 15
   Val Lys Lys Met Asn Met Leu Lys Glu Asn Val Asp Tyr Ile Gln Asn
   20 25 30
   Gln Asn Leu Phe Lys Glu Leu Met Asn Gln Lys Ala Thr Tyr Ser Phe
   35 40 45
   Val Asn Thr Lys
   50
<210> 314
   <211> 43
   <212> PRT
   <213> Plasmodium falciparum
<400> 314
   His Leu Ile Tyr Lys Asn Lys Ser Tyr Asn Pro Leu Leu Leu Ser Cys
   1               5                   10                  15
   Val Lys Lys Met Asn Met Leu Lys Glu Asn Val Asp Tyr Ile Gln Asn
               20                  25                  30
   Gln Asn Leu Phe Lys Glu Leu Met Asn Gln Lys
           35                  40
<210> 315
   <211> 38
   <212> PRT
   <213> Plasmodium falciparum
<400> 315
   His Leu Ile Tyr Lys Asn Lys Ser Tyr Asn Pro Leu Leu Leu Ser Cys
   1               5                   10                  15
   Val Lys Lys Met Asn Met Leu Lys Glu Asn Val Asp Tyr Ile Gln Lys
               20                  25                  30
   Asn Gln Asn Leu Phe Lys
           35
<210> 316
   <211> 24
   <212> PRT
   <213> Plasmodium falciparum
<400> 316
   His Leu Ile Tyr Lys Asn Lys Ser Tyr Asn Pro Leu Leu Leu Ser Cys
   1               5                   10                  15
   Val Lys Lys Met Asn Met Leu Lys
               20
<210> 317
   <211> 47
   <212> PRT
   <213> Plasmodium falciparum
<400> 317
   Lys Ser Ala Asn Asn Ser Ala Asn Asn Gly Lys Lys Asn Asn Ala Glu
   1 5 10 15
   Glu Met Lys Asn Leu Val Asn Phe Leu Gln Ser His Lys Lys Leu Ile
   20 25 30
   Lys Ala Leu Lys Lys Asn Ile Glu Ser Ile Gln Asn Lys Lys His
   35 40 45
<210> 318
   <211> 37
   <212> PRT
   <213> Plasmodium falciparum
<400> 318
   Lys Lys Asn Asn Ala Glu Glu Met Lys Asn Leu Val Asn Phe Leu Gln
   1 5 10 15
   Ser His Lys Lys Leu Ile Lys Ala Leu Lys Lys Asn Ile Glu Ser Ile
   20 25 30
   Gln Asn Lys Lys His
   35
<210> 319
   <211> 29
   <212> PRT
   <213> Plasmodium falciparum
<400> 319
   Lys Asn Leu Val Asn Phe Leu Gln Ser His Lys Lys Leu Ile Lys Ala
   1 5 10 15
   Leu Lys Lys Asn Ile Glu Ser Ile Gln Asn Lys Lys His
   20 25
<210> 320
   <211> 19
   <212> PRT
   <213> Plasmodium falciparum
<400> 320
   Lys Lys Leu Ile Lys Ala Leu Lys Lys Asn Ile Glu Ser Ile Gln Asn
   1 5 10 15
   Lys Lys His
<210> 321
   <211> 18
   <212> PRT
   <213> Plasmodium falciparum
<400> 321
   Lys Leu Ile Lys Ala Leu Lys Lys Asn Ile Glu Ser Ile Gln Asn Lys
   1 5 10 15
   Lys His
<210> 322
   <211> 12
   <212> PRT
   <213> Plasmodium falciparum
<400> 322
   Lys Lys Asn Ile Glu Ser Ile Gln Asn Lys Lys His
   1 5 10
<210> 323
   <211> 11
   <212> PRT
   <213> Plasmodium falciparum
<400> 323
   Lys Asn Ile Glu Ser Ile Gln Asn Lys Lys His
   1 5 10
<210> 324
   <211> 17
   <212> PRT
   <213> Plasmodium falciparum
<400> 324
   Lys Asn Asn Ala Glu Glu Met Lys Asn Leu Val Asn Phe Leu Gln Ser
   1 5 10 15
   His
<210> 325
   <211> 23
   <212> PRT
   <213> Plasmodium falciparum
<400> 325
   Lys Lys Leu Ile Lys Ala Leu Lys Lys Asn Ile Glu Ser Ile Gln Asn
   1 5 10 15
   Lys Lys Gln Gly His Lys Lys
   20
<210> 326
   <211> 19
   <212> PRT
   <213> Plasmodium falciparum
<400> 326
   Lys Lys Asn Asn Ala Glu Glu Met Lys Asn Leu Val Asn Phe Leu Gln
   1 5 10 15
   Ser His Lys
<210> 327
   <211> 17
   <212> PRT
   <213> Plasmodium falciparum
<400> 327
   Lys Asn Asn Ala Glu Glu Met Lys Asn Leu Val Asn Phe Leu Gln Ser
   1 5 10 15
   His
<210> 328
   <211> 22
   <212> PRT
   <213> Plasmodium falciparum
<400> 328
   Lys Leu Ile Lys Ala Leu Lys Lys Asn Ile Glu Ser Ile Gln Asn Lys
   1 5 10 15
   Lys Gln Gly His Lys Lys
   20
<210> 329
   <211> 28
   <212> PRT
   <213> Plasmodium falciparum
<400> 329
   Lys Val Lys Lys Ile Gly Val Thr Leu Lys Lys Phe Glu Pro Leu Lys
   1 5 10 15
   Asn Gly Asn Val Ser Glu Thr Ile Lys Leu Ile His
   20 25
<210> 330
   <211> 13
   <212> PRT
   <213> Plasmodium falciparum
<400> 330
   Lys Asn Gly Asn Val Ser Glu Thr Ile Lys Leu Ile His
   1 5 10
<210> 331
   <211> 11
   <212> PRT
   <213> Plasmodium falciparum
<400> 331
   Lys Leu Ile His Leu Gly Asn Lys Asp Lys Lys
   1 5 10
<210> 332
   <211> 28
   <212> PRT
   <213> Plasmodium falciparum
<400> 332
   Lys Ser Ala Asn Asn Ser Ala Asn Asn Gly Lys Lys Asn Asn Ala Glu
   1 5 10 15
   Glu Met Lys Asn Leu Val Asn Phe Leu Gln Ser His
   20 25
<210> 333
   <211> 18
   <212> PRT
   <213> Plasmodium falciparum
<400> 333
   Lys Lys Asn Asn Ala Glu Glu Met Lys Asn Leu Val Asn Phe Leu Gln
   1 5 10 15
   Ser His
<210> 334
   <212> 19
   <212> PRT
   <213> Plasmodium falciparum
<400> 334
<210> 335
   <211> 15
   <212> PRT
   <213> Plasmodium falciparum
<400> 335
   Lys Ala Leu Lys Lys Asn Ile Glu Ser Ile Gln Asn Lys Lys His
   1               5                   10                  15
<210> 336
   <211> 12
   <212> PRT
   <213> Plasmodium falciparum
<400> 336
   Lys Lys Asn Ile Glu Ser Ile Gln Asn Lys Lys His
   1               5                   10
<210> 337
   <211> 27
   <212> PRT
   <213> Plasmodium falciparum
<400> 337
   Lys Glu Leu Met Asn Gln Lys Ala Thr Tyr Ser Phe Val Asn Thr Lys
   1               5                   10                  15
   Lys Lys Ile Ile Ser Leu Lys Ser Gln Gly His
               20                  25
<210> 338
   <211> 7
   <212> PRT
   <213> Plasmodium falciparum
<400> 338
   Lys Ser Gln Gly His Lys Lys
   1               5

<210> 339
   <211> 12
   <212> PRT
   <213> Plasmodium falciparum
<400> 339
   Lys Lys Lys Ile Ile Ser Leu Lys Ser Gln Gly His
   1               5                   10
<210> 340
   <211> 11
   <212> PRT
   <213> Plasmodium falciparum
<400> 340
   Lys Lys Ile Ile Ser Leu Lys Ser Gln Gly His
   1 5 10
<210> 341
   <211> 12
   <212> PRT
   <213> Plasmodium falciparum
<400> 341
   Lys Lys Asn Ile Glu Ser Ile Gln Asn Lys Lys His
   1 5 10
<210> 342
   <211> 11
   <212> PRT
   <213> Plasmodium falciparum
<400> 342
   Lys Asn Ile Glu Ser Ile Gln Asn Lys Lys His
   1 5 10
<210> 343
   <211> 29
   <212> PRT
   <213> Plasmodium falciparum
<400> 343
   His Thr Tyr Val Lys Gly Lys Lys Ala Pro Ser Asp Pro Gln Cys Ala
   1 5 10 15
   Asp Ile Lys Glu Glu Cys Lys Glu Leu Leu Lys Glu Lys
   20 25
<210> 344
   <211> 27
   <212> PRT
   <213> Plasmodium falciparum
<400> 344
   His Thr Tyr Val Lys Gly Lys Lys Ala Pro Ser Asp Pro Gln Cys Ala
   1 5 10 15
   Asp Ile Lys Glu Glu Cys Lys Glu Leu Leu Lys
   20 25
<210> 345
   <211> 29
   <212> PRT
   <213> Plasmodium falciparum
<400> 345
   His Glu Asn Val Leu Ser Ala Ala Leu Glu Asn Thr Gln Ser Glu Glu
   1               5                   10                  15
   Glu Lys Lys Glu Val Ile Asp Val Ile Glu Glu Val Lys
               20                  25
<210> 346
   <211> 48
   <212> PRT
   <213> Plasmodium falciparum
<400> 346
   Lys Glu Asn Val Val Thr Thr Ile Leu Glu Lys Val Glu Glu Thr Thr
   1               5                   10                  15
   Ala Glu Ser Val Thr Thr Phe Ser Asn Ile Leu Glu Glu Ile Gln Glu
               20                  25                  30
   Asn Thr Ile Thr Asn Asp Thr Ile Glu Glu Lys Leu Glu Glu Leu His
           35                  40                  45
<210> 347
   <211> 14
   <212> PRT
   <213> Plasmodium falciparum
<400> 347
   His Tyr Leu Gln Gln Met Lys Glu Lys Phe Ser Lys Glu Lys
   1               5                   10
<210> 348
   <211> 42
   <212> PRT
   <213> Plasmodium falciparum
<400> 348
   His Tyr Leu Gln Gln Met Lys Glu Lys Phe Ser Lys Glu Lys Asn Asn
   1               5                   10                  15
   Asn Val Ile Glu Val Thr Asn Lys Ala Glu Lys Lys Gly Asn Val Gln
               20                  25                  30
   Val Thr Asn Lys Thr Glu Lys Thr Thr Lys
           35                  40
<210> 349
   <211> 48
   <212> PRT
   <213> Plasmodium falciparum
<400> 349
   His Tyr Leu Gln Gln Met Lys Glu Lys Phe Ser Lys Glu Lys Asn Asn
   1               5                   10                  15
   Asn Val Ile Glu Val Thr Asn Lys Ala Glu Lys Lys Gly Asn Val Gln
               20                  25                  30
   Val Thr Asn Lys Thr Glu Lys Thr Thr Lys Val Asp Lys Asn Asn Lys
           35                  40                  45
<210> 350
   <211> 57
   <212> PRT
   <213> Plasmodium falciparum
<400> 350
   His Tyr Leu Gln Gln Met Lys Glu Lys Phe Ser Lys Glu Lys Asn Asn
   1               5                   10                  15
   Asn Val Ile Glu Val Thr Asn Lys Ala Glu Lys Lys Gly Asn Val Gln
               20                  25                  30
   Val Thr Asn Lys Thr Glu Lys Thr Thr Lys Val Asp Lys Asn Asn Lys
           35                  40                  45
   Val Pro Lys Lys Arg Arg Thr Gln Lys
       50                  55
<210> 351
   <211> 59
   <212> PRT
   <213> Plasmodium falciparum
<400> 351
   His Tyr Leu Gln Gln Met Lys Glu Lys Phe Ser Lys Glu Lys Asn Asn
   1               5                   10                  15
   Asn Val Ile Glu Val Thr Asn Lys Ala Glu Lys Lys Gly Asn Val Gln
               20                  25                  30
   Val Thr Asn Lys Thr Glu Lys Thr Thr Lys Val Asp Lys Asn Asn Lys
           35                  40                  45
   Val Pro Lys Lys Arg Arg Thr Gln Lys Ser Lys
       50                  55
<210> 352
   <211> 52
   <212> PRT
   <213> Plasmodium falciparum
<400> 352
   His Val Asp Glu Val Met Lys Tyr Val Gln Lys Ile Asp Lys Glu Val
   1               5                   10                  15
   Asp Lys Glu Val Ser Lys Ala Leu Glu Ser Lys Asn Asp Val Thr Asn
               20                  25                  30
   Val Leu Lys Gln Asn Gln Asp Phe Phe Ser Lys Val Lys Asn Phe Val
           35                  40                  45
   Lys Lys Tyr Lys
       50
<210> 353
   <211> 50
   <212> PRT
   <213> Plasmodium falciparum
<400> 353
   His Val Asp Glu Val Met Lys Tyr Val Gln Lys Ile Asp Lys Glu Val
   1               5                   10                  15
   Asp Lys Glu Val Ser Lys Ala Leu Glu Ser Lys Asn Asp Val Thr Asn
               20                  25                  30
   Val Leu Lys Gln Asn Gln Asp Phe Phe Ser Lys Val Lys Asn Phe Val
           35                  40                  45
   Lys Lys
       50
<210> 354
   <211> 43
   <212> PRT
   <213> Plasmodium falciparum
<400> 354
   His Val Asp Glu Val Met Lys Tyr Val Gln Lys Ile Asp Lys Glu Val
   1               5                   10                  15
   Asp Lys Glu Val Ser Lys Ala Leu Glu Ser Lys Asn Asp Val Thr Asn
               20                  25                  30
   Val Leu Lys Gln Asn Gln Asp Phe Phe Ser Lys
           35                  40
<210> 355
   <211> 35
   <212> PRT
   <213> Plasmodium falciparum
<400> 355
   His Val Asp Glu Val Met Lys Tyr Val Gln Lys Ile Asp Lys Glu Val
   1               5                   10                  15
   Asp Lys Glu Val Ser Lys Ala Leu Glu Ser Lys Asn Asp Val Thr Asn
               20                  25                  30
   Val Leu Lys
           35
<210> 356
   <211> 27
   <212> PRT
   <213> Plasmodium falciparum
<400> 356
   His Val Asp Glu Val Met Lys Tyr Val Gln Lys Ile Asp Lys Glu Val
   1               5                   10                  15
   Asp Lys Glu Val Ser Lys Ala Leu Glu Ser Lys
               20                  25
<210> 357
   <211> 22
   <212> PRT
   <213> Plasmodium falciparum
<400> 357
   His Val Asp Glu Val Met Lys Tyr Val Gln Lys Ile Asp Lys Glu Val
   1               5                   10                  15
   Asp Lys Glu Val Ser Lys
               20
<210> 358
   <211> 18
   <212> PRT
   <213> Plasmodium falciparum
<400> 358
   His Val Asp Glu Val Met Lys Tyr Val Gln Lys Ile Asp Lys Glu Val
   1               5                   10                  15
   Asp Lys
<210> 359
   <211> 14
   <212> PRT
   <213> Plasmodium falciparum
<400> 359
   His Val Asp Glu Val Met Lys Tyr Val Gln Lys Ile Asp Lys
   1 5 10
<210> 360
   <211> 39
   <212> PRT
   <213> Plasmodium falciparum
<400> 360
   Lys Asp Glu Val Ile Asp Leu Ile Val Gln Lys Glu Lys Arg Ile Glu
   1 5 10 15
   Lys Val Lys Ala Lys Lys Lys Lys Leu Glu Lys Lys Val Glu Glu Gly
   20 25 30
   Val Ser Gly Leu Lys Lys His
   35
<210> 361
   <211> 23
   <212> PRT
   <213> Plasmodium falciparum
<400> 361
   Lys Val Lys Ala Lys Lys Lys Lys Leu Glu Lys Lys Val Glu Glu Gly
   1 5 10 15
   Val Ser Gly Leu Lys Lys His
   20
<210> 362
   <211> 21
   <212> PRT
   <213> Plasmodium falciparum
<400> 362
   Lys Ala Lys Lys Lys Lys Leu Glu Lys Lys Val Glu Glu Gly Val Ser
   1 5 10 15
   Gly Leu Lys Lys His
   20
<210> 363
   <211> 19
   <212> PRT
   <213> Plasmodium falciparum
<400> 363
   Lys Lys Lys Lys Leu Glu Lys Lys Val Glu Glu Gly Val Ser Gly Leu
   1 5 10 15
   Lys Lys His
<210> 364
   <211> 18
   <212> PRT
   <213> Plasmodium falciparum
<400> 364
   Lys Lys Lys Leu Glu Lys Lys Val Glu Glu Gly Val Ser Gly Leu Lys
   1 5 10 15
   Lys His
<210> 365
   <211> 17
   <212> PRT
   <213> Plasmodium falciparum
<400> 365
   Lys Lys Leu Glu Lys Lys Val Glu Glu Gly Val Ser Gly Leu Lys Lys
   1 5 10 15
   His
<210> 366
   <211> 16
   <212> PRT
   <213> Plasmodium falciparum
<400> 366
   Lys Leu Glu Lys Lys Val Glu Glu Gly Val Ser Gly Leu Lys Lys His
   1 5 10 15
<210> 367
   <211> 13
   <212> PRT
   <213> Plasmodium falciparum
<400> 367
   Lys Lys Val Glu Glu Gly Val Ser Gly Leu Lys Lys His
   1 5 10
<210> 368
   <211> 12
   <212> PRT
   <213> Plasmodium falciparum
<400> 368
   Lys Val Glu Glu Gly Val Ser Gly Leu Lys Lys His
   1 5 10
<210> 369
   <211> 59
   <212> PRT
   <213> Plasmodium falciparum
<400> 369
   His Val Glu Gln Asn Val Tyr Val Asp Val Asp Val Pro Ala Met Lys
   1               5                   10                  15
   Asp Gln Phe Leu Gly Ile Leu Asn Glu Ala Gly Gly Leu Lys Glu Met
               20                  25                  30
   Phe Phe Asn Leu Glu Asp Val Phe Lys Ser Glu Ser Asp Val Ile Thr
           35                  40                  45
   Val Glu Glu Ile Lys Asp Glu Pro Val Gln Lys
       50                  55
<210> 370
   <211> 53
   <212> PRT
   <213> Plasmodium falciparum
<400> 370
   His Ile Lys Gly Leu Glu Glu Asp Asp Leu Glu Glu Val Asp Asp Leu
   1               5                   10                  15
   Lys Gly Ser Ile Leu Asp Met Leu Lys Gly Asp Met Glu Leu Gly Asp
               20                  25                  30
   Met Asp Lys Glu Ser Leu Glu Asp Val Thr Thr Lys Leu Gly Glu Arg
           35                  40                  45
   Val Glu Ser Leu Lys
       50
<210> 371
   <211> 44
   <212> PRT
   <213> Plasmodium falciparum
<400> 371
   His Ile Lys Gly Leu Glu Glu Asp Asp Leu Glu Glu Val Asp Asp Leu
   1               5                   10                  15
   Lys Gly Ser Ile Leu Asp Met Leu Lys Gly Asp Met Glu Leu Gly Asp
               20                  25                  30
   Met Asp Lys Glu Ser Leu Glu Asp Val Thr Thr Lys
           35                  40
<210> 372
   <211> 35
   <212> PRT
   <213> Plasmodium falciparum
<400> 372
   His Ile Lys Gly Leu Glu Glu Asp Asp Leu Glu Glu Val Asp Asp Leu
   1 5 10 15
   Lys Gly Ser Ile Leu Asp Met Leu Lys Gly Asp Met Glu Leu Gly Asp
   20 25 30
   Met Asp Lys
   35
<210> 373
   <211> 25
   <212> PRT
   <213> Plasmodium falciparum
<400> 373
   His Ile Lys Gly Leu Glu Glu Asp Asp Leu Glu Glu Val Asp Asp Leu
   1 5 10 15
   Lys Gly Ser Ile Leu Asp Met Leu Lys
   20 25
<210> 374
   <211> 31
   <212> PRT
   <213> Plasmodium falciparum
<400> 374
   His Ile Ile Ser Gly Asp Ala Asp Val Leu Ser Ser Ala Leu Gly Met
   1 5 10 15
   Asp Glu Glu Gln Met Lys Thr Arg Lys Lys Ala Gln Arg Pro Lys
   20 25 30
<210> 375
   <211> 23
   <212> PRT
   <213> Plasmodium falciparum
<400> 375
   His Asp Ile Thr Thr Thr Leu Asp Glu Val Val Glu Leu Lys Asp Val
   1 5 10 15
   Glu Glu Asp Lys Ile Glu Lys
   20
<210> 376
   <211> 10
   <212> PRT
   <213> Plasmodium falciparum
<400> 376
   Lys Lys Leu Glu Glu Val His Glu Leu Lys
   1 5 10
<210> 377
   <211> 9
   <212> PRT
   <213> Plasmodium falciparum
<400> 377
   Lys Leu Glu Glu Val His Glu Leu Lys
   1 5
<210> 378
   <211> 19
   <212> PRT
   <213> Plasmodium falciparum
<400> 378
   Lys Thr Ile Glu Thr Asp Ile Leu Glu Glu Lys Lys Lys Glu Ile Glu
   1 5 10 15
   Lys Asp His
<210> 379
   <211> 11
   <212> PRT
   <213> Plasmodium falciparum
<400> 379
   Lys Lys Glu Ile Glu Lys Asp His Phe Glu Lys
   1 5 10
<210> 380
   <211> 6
   <212> PRT
   <213> Plasmodium falciparum
<400> 380
   Lys Asp His Phe Glu Lys
   1 5
<210> 381
   <211> 11
   <212> PRT
   <213> Plasmodium falciparum
<400> 381
   Lys Phe Glu Glu Glu Ala Glu Glu Ile Lys His
   1 5 10
<210> 382
   <211> 47
   <212> PRT
   <213> Plasmodium falciparum
<400> 382
   Lys Asp Gly Asp Thr Lys Cys Thr Leu Glu Cys Ala Gln Gly Lys Lys
   1 5 10 15
   Cys Ile Lys His Lys Ser Asp His Asn His Lys Ser Asp His Asn His
   20 25 30
   Lys Ser Asp Pro Asn His Lys Lys Lys Asn Asn Asn Asn Asn Lys
   35 40 45
<210> 383
   <211> 40
   <212> PRT
   <213> Plasmodium falciparum
<400> 383
   Lys Asp Gly Asp Thr Lys Cys Thr Leu Glu Cys Ala Gln Gly Lys Lys
   1 5 10 15
   Cys Ile Lys His Lys Ser Asp His Asn His Lys Ser Asp His Asn His
   20 25 30
   Lys Ser Asp Pro Asn His Lys Lys
   35 40
<210> 384
   <211> 39
   <212> PRT
   <213> Plasmodium falciparum
<400> 384
   Lys Asp Gly Asp Thr Lys Cys Thr Leu Glu Cys Ala Gln Gly Lys Lys
   1 5 10 15
   Cys Ile Lys His Lys Ser Asp His Asn His Lys Ser Asp His Asn His
   20 25 30
   Lys Ser Asp Pro Asn His Lys
   35
<210> 385
   <211> 33
   <212> PRT
   <213> Plasmodium falciparum
<400> 385
   Lys Asp Gly Asp Thr Lys Cys Thr Leu Glu Cys Ala Gln Gly Lys Lys
   1 5 10 15
   Cys Ile Lys His Lys Ser Asp His Asn His Lys Ser Asp His Asn His
   20 25 30
   Lys
<210> 386
   <211> 27
   <212> PRT
   <213> Plasmodium falciparum
<400> 386
   Lys Asp Gly Asp Thr Lys Cys Thr Leu Glu Cys Ala Gln Gly Lys Lys
   1 5 10 15
   Cys Ile Lys His Lys Ser Asp His Asn His Lys
   20 25
<210> 387
   <211> 21
   <212> PRT
   <213> Plasmodium falciparum
<400> 387
   Lys Asp Gly Asp Thr Lys Cys Thr Leu Glu Cys Ala Gln Gly Lys Lys
   1 5 10 15
   Cys Ile Lys His Lys
   20
<210> 388
   <211> 16
   <212> PRT
   <213> Plasmodium falciparum
<400> 388
   Lys Asp Gly Asp Thr Lys Cys Thr Leu Glu Cys Ala Gln Gly Lys Lys
   1 5 10 15
<210> 389
   <211> 15
   <212> PRT
   <213> Plasmodium falciparum
<400> 389
   Lys Asp Gly Asp Thr Lys Cys Thr Leu Glu Cys Ala Gln Gly Lys
   1 5 10 15
<210> 390
   <211> 23
   <212> PRT
   <213> Plasmodium falciparum
<400> 390
   Lys Cys Ile Gln Ala Glu Cys Asn Tyr Lys Glu Cys Gly Glu Gln Lys
   1 5 10 15
   Cys Val Trp Asp Gly Ile His
   20
<210> 391
   <211> 14
   <212> PRT
   <213> Plasmodium falciparum
<400> 391
   Lys Glu Cys Gly Glu Gln Lys Cys Val Trp Asp Gly Ile His
   1 5 10
<210> 392
   <211> 32
   <212> PRT
   <213> Plasmodium falciparum
<400> 392
   His Ile Glu Cys Lys Cys Asn Asn Asp Tyr Val Leu Thr Asn Arg Tyr
   1 5 10 15
   Glu Cys Glu Pro Lys Asn Lys Cys Thr Ser Leu Glu Asp Thr Asn Lys
   20 25 30
<210> 393
   <211> 39
   <212> PRT
   <213> Plasmodium falciparum
<400> 393
   Lys Ser Asp His Asn His Lys Ser Asp His Asn His Lys Ser Asp His
   1 5 10 15
   Asn His Lys Ser Asp His Asn His Lys Ser Asp Pro Asn His Lys Lys
   20 25 30
   Lys Asn Asn Asn Asn Asn Lys
   35
<210> 394
   <211> 33
   <212> PRT
   <213> Plasmodium falciparum
<400> 394
   Lys Ser Asp His Asn His Lys Ser Asp His Asn His Lys Ser Asp His
   1 5 10 15
   Asn His Lys Ser Asp Pro Asn His Lys Lys Lys Asn Asn Asn Asn Asn
   20 25 30
   Lys
<210> 395
   <211> 27
   <212> PRT
   <213> Plasmodium falciparum
<400> 395
   Lys Ser Asp His Asn His Lys Ser Asp His Asn His Lys Ser Asp Pro
   1 5 10 15
   Asn His Lys Lys Lys Asn Asn Asn Asn Asn Lys
   20 25
<210> 396
   <211> 21
   <212> PRT
   <213> Plasmodium falciparum
<400> 396
   Lys Ser Asp His Asn His Lys Ser Asp Pro Asn His Lys Lys Lys Asn
   1 5 10 15
   Asn Asn Asn Asn Lys
   20
<210> 397
   <211> 18
   <212> PRT
   <213> Plasmodium falciparum
<400> 397
   Lys Lys Lys Asn Asn Asn Asn Asn Lys Asp Asn Lys Ser Asp Pro Asn
   1 5 10 15
   His Lys
<210> 398
   <211> 17
   <212> PRT
   <213> Plasmodium falciparum
<400> 398
   Lys Lys Asn Asn Asn Asn Asn Lys Asp Asn Lys Ser Asp Pro Asn His
   1 5 10 15
   Lys
<210> 399
   <211> 16
   <212> PRT
   <213> Plasmodium falciparum
<400> 399
   Lys Asn Asn Asn Asn Asn Lys Asp Asn Lys Ser Asp Pro Asn His Lys
   1 5 10 15
<210> 400
   <211> 10
   <212> PRT
   <213> Plasmodium falciparum
<400> 400
   Lys Asp Asn Lys Ser Asp Pro Asn His Lys
   1 5 10
<210> 401
   <211> 7
   <212> PRT
   <213> Plasmodium falciparum
<400> 401
   Lys Ser Asp Pro Asn His Lys
   1 5
<210> 402
   <211> 35
   <212> PRT
   <213> Plasmodium falciparum
<400> 402
   His Ser Leu Tyr Ala Leu Gln Gln Asn Glu Glu Tyr Gln Lys Val Lys
   1 5 10 15
   Asn Glu Lys Asp Gln Asn Glu Ile Lys Lys Ile Lys Gln Leu Ile Glu
   20 25 30
   Lys Asn Lys
   35
<210> 403
   <211> 28
   <212> PRT
   <213> Plasmodium falciparum
<400> 403
   His Ser Leu Tyr Ala Leu Gln Gln Asn Glu Glu Tyr Gln Lys Val Lys
   1               5                   10                  15
   Asn Glu Lys Asp Gln Asn Glu Ile Lys Lys Ile Lys
               20                  25
<210> 404
   <211> 26
   <212> PRT
   <213> Plasmodium falciparum
<400> 404
   His Ser Leu Tyr Ala Leu Gln Gln Asn Glu Glu Tyr Gln Lys Val Lys
   1               5                   10                  15
   Asn Glu Lys Asp Gln Asn Glu Ile Lys Lys
               20                  25
<210> 405
   <211> 25
   <212> PRT
   <213> Plasmodium falciparum
<400> 405
   His Ser Leu Tyr Ala Leu Gln Gln Asn Glu Glu Tyr Gln Lys Val Lys
   1               5                   10                  15
   Asn Glu Lys Asp Gln Asn Glu Ile Lys
               20                  25
<210> 406
   <211> 11
   <212> PRT
   <213> Plasmodium falciparum
<400> 406
   His Lys Leu Glu Asn Leu Glu Glu Met Asp Lys
   1               5                   10
<210> 407
   <211> 11
   <212> PRT
   <213> Plasmodium falciparum
<400> 407
   Lys His Phe Asp Asp Asn Thr Asn Glu Gln Lys
   1 5 10
<210> 408
   <211> 8
   <212> PRT
   <213> Plasmodium falciparum
<400> 408
   Lys Lys Glu Asp Asp Glu Lys His
   1 5
<210> 409
   <211> 13
   <212> PRT
   <213> Plasmodium falciparum
<400> 409
   Lys Glu Glu Asn Asn Lys Lys Glu Asp Asp Glu Lys His
   1 5 10
<210> 410
   <211> 21
   <212> PRT
   <213> Plasmodium falciparum
<400> 410
   Lys Thr Ser Ser Gly Ile Leu Asn Lys Glu Glu Asn Asn Lys Lys Glu
   1 5 10 15
   Asp Asp Glu Lys His
   20
<210> 411
   <211> 7
   <212> PRT
   <213> Plasmodium falciparum
<400> 411
   Lys Asn Ile His Ile Lys Lys
   1 5
<210> 412
   <211> 13
   <212> PRT
   <213> Plasmodium falciparum
<400> 412
   His Ile Lys Lys Lys Glu Gly Ile Asp Ile Gly Tyr Lys
   1 5 10
<210> 413
   <211> 21
   <212> PRT
   <213> Plasmodium falciparum
<400> 413
   Lys Lys Met Trp Thr Cys Lys Leu Trp Asp Asn Lys Gly Asn Glu Ile
   1 5 10 15
   Thr Lys Asn Ile His
   20
<210> 414
   <211> 30
   <212> PRT
   <213> Plasmodium falciparum
<400> 414
   Lys Lys Gly Ile Gln Trp Asn Leu Leu Lys Lys Met Trp Thr Cys Lys
   1 5 10 15
   Leu Trp Asp Asn Lys Gly Asn Glu Ile Thr Lys Asn Ile His
   20 25 30
<210> 415
   <211> 50
   <212> PRT
   <213> Plasmodium falciparum
<400> 415
   Lys Glu Lys Lys Asp Ser Asn Glu Asn Arg Lys Lys Lys Gln Lys Glu
   1 5 10 15
   Asp Lys Lys Asn Pro Asn Lys Leu Lys Lys Ile Glu Tyr Thr Asn Lys
   20 25 30
   Ile Thr His Phe Phe Lys Ala Lys Asn Asn Lys Gln Gln Asn Asn Val
   35 40 45
   Thr His
   50
<210> 416
   <211> 48
   <212> PRT
   <213> Plasmodium falciparum
<400> 416
   Lys Lys Asp Ser Asn Glu Asn Arg Lys Lys Lys Gln Lys Glu Asp Lys
   1 5 10 15
   Lys Asn Pro Asn Lys Leu Lys Lys Ile Glu Tyr Thr Asn Lys Ile Thr
   20 25 30
   His Phe Phe Lys Ala Lys Asn Asn Lys Gln Gln Asn Asn Val Thr His
   35 40 45
<210> 417
   <211> 47
   <212> PRT
   <213> Plasmodium falciparum
<400> 417
   Lys Asp Ser Asn Glu Asn Arg Lys Lys Lys Gln Lys Glu Asp Lys Lys
   1 5 10 15
   Asn Pro Asn Lys Leu Lys Lys Ile Glu Tyr Thr Asn Lys Ile Thr His
   20 25 30
   Phe Phe Lys Ala Lys Asn Asn Lys Gln Gln Asn Asn Val Thr His
   35 40 45
<210> 418
   <211> 39
   <212> PRT
   <213> Plasmodium falciparum
<400> 418
   Lys Lys Gln Lys Glu Asp Lys Lys Asn Pro Asn Lys Leu Lys Lys Ile
   1 5 10 15
   Glu Tyr Thr Asn Lys Ile Thr His Phe Phe Lys Ala Lys Asn Asn Lys
   20 25 30
   Gln Gln Asn Asn Val Thr His
   35
<210> 419
   <211> 38
   <212> PRT
   <213> Plasmodium falciparum
<400> 419
   Lys Gln Lys Glu Asp Lys Lys Asn Pro Asn Lys Leu Lys Lys Ile Glu
   1 5 10 15
   Tyr Thr Asn Lys Ile Thr His Phe Phe Lys Ala Lys Asn Asn Lys Gln
   20 25 30
   Gln Asn Asn Val Thr His
   35
<210> 420
   <211> 36
   <212> PRT
   <213> Plasmodium falciparum
<400> 420
   Lys Glu Asp Lys Lys Asn Pro Asn Lys Leu Lys Lys Ile Glu Tyr Thr
   1               5                   10                  15
   Asn Lys Ile Thr His Phe Phe Lys Ala Lys Asn Asn Lys Gln Gln Asn
               20                  25                  30
   Asn Val Thr His
           35
<210> 421
   <211> 32
   <212> PRT
   <213> Plasmodium falciparum
<400> 421
   Lys Asn Pro Asn Lys Leu Lys Lys Ile Glu Tyr Thr Asn Lys Ile Thr
   1               5                   10                  15
   His Phe Phe Lys Ala Lys Asn Asn Lys Gln Gln Asn Asn Val Thr His
               20                  25                  30
<210> 422
   <211> 26
   <212> PRT
   <213> Plasmodium falciparum
<400> 422
   Lys Lys Ile Glu Tyr Thr Asn Lys Ile Thr His Phe Phe Lys Ala Lys
   1               5                   10                  15
   Asn Asn Lys Gln Gln Asn Asn Val Thr His
               20                  25
<210> 423
   <211> 25
   <212> PRT
   <213> Plasmodium falciparum
<400> 423
   Lys Ile Glu Tyr Thr Asn Lys Ile Thr His Phe Phe Lys Ala Lys Asn
   1               5                   10                  15
   Asn Lys Gln Gln Asn Asn Val Thr His
               20                  25
<210> 424
   <211> 19
   <212> PRT
   <213> Plasmodium falciparum
<400> 424
   Lys Ile Thr His Phe Phe Lys Ala Lys Asn Asn Lys Gln Gln Asn Asn
   1 5 10 15
   Val Thr His
<210> 425
   <211> 48
   <212> PRT
   <213> Plasmodium falciparum
<400> 425
   His Lys Asn Asn Glu Asp Ile Lys Asn Asp Asn Ser Lys Asp Ile Lys
   1 5 10 15
   Asn Asp Asn Ser Lys Asp Ile Lys Asn Asp Asn Ser Lys Asp Ile Lys
   20 25 30
   Asn Asp Asn Asn Glu Asp Ile Lys Asn Asp Asn Ser Lys Asp Ile Lys
   35 40 45
<210> 426
   <211> 45
   <212> PRT
   <213> Plasmodium falciparum

<400> 426
   His Lys Asn Asn Glu Asp Ile Lys Asn Asp Asn Ser Lys Asp Ile Lys
   1 5 10 15
   Asn Asp Asn Ser Lys Asp Ile Lys Asn Asp Asn Ser Lys Asp Ile Lys
   20 25 30
   Asn Asp Asn Asn Glu Asp Ile Lys Asn Asp Asn Ser Lys
   35 40 45
<210> 427
   <211> 40
   <212> PRT
   <213> Plasmodium falciparum
<400> 427
   His Lys Asn Asn Glu Asp Ile Lys Asn Asp Asn Ser Lys Asp Ile Lys
   1 5 10 15
   Asn Asp Asn Ser Lys Asp Ile Lys Asn Asp Asn Ser Lys Asp Ile Lys
   20 25 30
   Asn Asp Asn Asn Glu Asp Ile Lys
   35 40
<210> 428
   <211> 32
   <212> PRT
   <213> Plasmodium falciparum
<400> 428
   His Lys Asn Asn Glu Asp Ile Lys Asn Asp Asn Ser Lys Asp Ile Lys
   1 5 10 15
   Asn Asp Asn Ser Lys Asp Ile Lys Asn Asp Asn Ser Lys Asp Ile Lys
   20 25 30
<210> 429
   <211> 29
   <212> PRT
   <213> Plasmodium falciparum
<400> 429
   His Lys Asn Asn Glu Asp Ile Lys Asn Asp Asn Ser Lys Asp Ile Lys
   1 5 10 15
   Asn Asp Asn Ser Lys Asp Ile Lys Asn Asp Asn Ser Lys
   20 25
<210> 430
   <211> 24
   <212> PRT
   <213> Plasmodium falciparum
<400> 430
   His Lys Asn Asn Glu Asp Ile Lys Asn Asp Asn Ser Lys Asp Ile Lys
   1 5 10 15
   Asn Asp Asn Ser Lys Asp Ile Lys
   20
<210> 431
   <211> 21
   <212> PRT
   <213> Plasmodium falciparum
<400> 431
   His Lys Asn Asn Glu Asp Ile Lys Asn Asp Asn Ser Lys Asp Ile Lys
   1 5 10 15
   Asn Asp Asn Ser Lys
   20
<210> 432
   <211> 16
   <212> PRT
   <213> Plasmodium falciparum
<400> 432
   His Lys Asn Asn Glu Asp Ile Lys Asn Asp Asn Ser Lys Asp Ile Lys
   1 5 10 15
<210> 433
   <211> 8
   <212> PRT
   <213> Plasmodium falciparum
<400> 433
   His Lys Asn Asn Glu Asp Ile Lys
   1 5
<210> 434
   <211> 31
   <212> PRT
   <213> Plasmodium falciparum
<400> 434
   Lys Lys Tyr Asp Asp Leu Gln Asn Lys Tyr Asn Ile Leu Asn Lys Leu
   1 5 10 15
   Lys Asn Ser Leu Glu Glu Lys Asn Glu Glu Leu Lys Lys Tyr His
   20 25 30
<210> 435
   <211> 30
   <212> PRT
   <213> Plasmodium falciparum
<400> 435
   Lys Tyr Asp Asp Leu Gln Asn Lys Tyr Asn Ile Leu Asn Lys Leu Lys
   1 5 10 15
   Asn Ser Leu Glu Glu Lys Asn Glu Glu Leu Lys Lys Tyr His
   20 25 30
<210> 436
   <211> 23
   <212> PRT
   <213> Plasmodium falciparum
<400> 436
   Lys Tyr Asn Ile Leu Asn Lys Leu Lys Asn Ser Leu Glu Glu Lys Asn
    1               5                  10                  15
   Glu Glu Leu Lys Lys Tyr His
               20
<210> 437
   <211> 17
   <212> PRT
   <213> Plasmodium falciparum
<400> 437
   Lys Leu Lys Asn Ser Leu Glu Glu Lys Asn Glu Glu Leu Lys Lys Tyr
    1               5                  10                  15
   His
<210> 438
   <211> 15
   <212> PRT
   <213> Plasmodium falciparum
<400> 438
   Lys Asn Ser Leu Glu Glu Lys Asn Glu Glu Leu Lys Lys Tyr His
    1               5                  10                  15
<210> 439
   <211> 9
   <212> PRT
   <213> Plasmodium falciparum
<400> 439
   Lys Asn Glu Glu Leu Lys Lys Tyr His
    1               5
<210> 440
   <211> 35
   <212> PRT
   <213> Plasmodium falciparum
<400> 440
   His Met Gly Asn Asn Gln Asp Ile Asn Glu Asn Val Tyr Asn Ile Lys
   1 5 10 15
   Pro Gln Glu Phe Lys Glu Glu Glu Glu Glu Asp Ile Ser Met Val Asn
   20 25 30
   Thr Lys Lys
   35
<210> 441
   <211> 17
   <212> PRT
   <213> Plasmodium falciparum
<400> 441
   Lys Asn Ser Asn Glu Leu Lys Arg Ile Asn Asp Asn Phe Phe Lys Leu
   1 5 10 15
   His
<210> 442
   <211> 55
   <212> PRT
   <213> Plasmodium falciparum
<400> 442
   Lys Pro Cys Leu Tyr Lys Lys Cys Lys Ile Ser Gln Cys Leu Tyr Lys
   1 5 10 15
   Lys Cys Lys Ile Ser Gln Val Trp Trp Cys Met Pro Val Lys Asp Thr
   20 25 30
   Phe Asn Thr Tyr Glu Arg Asn Asn Val Leu Asn Ser Lys Ile Glu Asn
   35 40 45
   Asn Ile Glu Lys Ile Pro His
   50 55
<210> 443
   <211> 40
   <212> PRT
   <213> Plasmodium falciparum
<400> 443
   His Ile Asn Asn Glu Tyr Thr Asn Lys Asn Pro Lys Asn Cys Leu Leu
   1 5 10 15
   Tyr Lys Asn Glu Glu Arg Asn Tyr Asn Asp Asn Asn Ile Lys Asp Tyr
   20 25 30
   Ile Asn Ser Met Asn Phe Lys Lys
   35 40
<210> 444
   <211> 39
   <212> PRT
   <213> Plasmodium falciparum
<400> 444
   His Ile Asn Asn Glu Tyr Thr Asn Lys Asn Pro Lys Asn Cys Leu Leu
   1 5 10 15
   Tyr Lys Asn Glu Glu Arg Asn Tyr Asn Asp Asn Asn Ile Lys Asp Tyr
   20 25 30
   Ile Asn Ser Met Asn Phe Lys
   35
<210> 445
   <211> 18
   <212> PRT
   <213> Plasmodium falciparum
<400> 445
   His Ile Asn Asn Glu Tyr Thr Asn Lys Asn Pro Lys Asn Cys Leu Leu
   1 5 10 15
   Tyr Lys
<210> 446
   <211> 23
   <212> PRT
   <213> Plasmodium falciparum
<400> 446
   Lys Asn Lys Thr Asn Gln Ser Lys Gly Val Lys Gly Glu Tyr Glu Lys
   1 5 10 15
   Lys Lys Glu Thr Asn Gly His
   20
<210> 447
   <211> 21
   <212> PRT
   <213> Plasmodium falciparum
<400> 447
   Lys Thr Asn Gln Ser Lys Gly Val Lys Gly Glu Tyr Glu Lys Lys Lys
   1 5 10 15
   Glu Thr Asn Gly His
   20
<210> 448
   <211> 16
   <212> PRT
   <213> Plasmodium falciparum
<400> 448
   Lys Gly Val Lys Gly Glu Tyr Glu Lys Lys Lys Glu Thr Asn Gly His
   1 5 10 15
<210> 449
   <211> 13
   <212> PRT
   <213> Plasmodium falciparum
<400> 449
   Lys Gly Glu Tyr Glu Lys Lys Lys Glu Thr Asn Gly His
   1 5 10
<210> 450
   <211> 28
   <212> PRT
   <213> Plasmodium falciparum
<400> 450
   Lys Ser Gly Met Tyr Thr Asn Glu Gly Asn Lys Ser Cys Glu Cys Ser
   1 5 10 15
   Tyr Lys Lys Lys Ser Ser Ser Ser Asn Lys Val His
   20 25
<210> 451
   <211> 18
   <212> PRT
   <213> Plasmodium falciparum
<400> 451
   Lys Ser Cys Glu Cys Ser Tyr Lys Lys Lys Ser Ser Ser Ser Asn Lys
   1 5 10 15
   Val His
<210> 452
   <211> 11
   <212> PRT
   <213> Plasmodium falciparum
<400> 452
   Lys Lys Lys Ser Ser Ser Ser Asn Lys Val His
   1 5 10
<210> 453
   <211> 10
   <212> PRT
   <213> Plasmodium falciparum
<400> 453
   Lys Lys Ser Ser Ser Ser Asn Lys Val His
   1 5 10
<210> 454
   <211> 9
   <212> PRT
   <213> Plasmodium falciparum
<400> 454
   Lys Ser Ser Ser Ser Asn Lys Val His
   1 5
<210> 455
   <211> 30
   <212> PRT
   <213> Plasmodium falciparum
<400> 455
   His Ile Met Leu Lys Ser Gly Met Tyr Thr Asn Glu Gly Asn Lys Ser
   1 5 10 15
   Cys Glu Cys Ser Tyr Lys Lys Lys Ser Ser Ser Ser Asn Lys
   20 25 30
<210> 456
   <211> 24
   <212> PRT
   <213> Plasmodium falciparum
<400> 456
   His Ile Met Leu Lys Ser Gly Met Tyr Thr Asn Glu Gly Asn Lys Ser
   1 5 10 15
   Cys Glu Cys Ser Tyr Lys Lys Lys
   20
<210> 457
   <211> 23
   <212> PRT
   <213> Plasmodium falciparum
<400> 457
   His Ile Met Leu Lys Ser Gly Met Tyr Thr Asn Glu Gly Asn Lys Ser
   1 5 10 15
   Cys Glu Cys Ser Tyr Lys Lys
   20
<210> 458
   <211> 22
   <212> PRT
   <213> Plasmodium falciparum
<400> 458
   His Ile Met Leu Lys Ser Gly Met Tyr Thr Asn Glu Gly Asn Lys Ser
   1 5 10 15
   Cys Glu Cys Ser Tyr Lys
   20
<210> 459
   <211> 50
   <212> PRT
   <213> Plasmodium falciparum
<400> 459
   Lys Pro Leu Ala Lys Leu Arg Lys Arg Glu Lys Thr Gln Ile Asn Lys
   1 5 10 15
   Thr Lys Tyr Glu Arg Gly Asp Val Ile Ile Asp Asn Thr Glu Ile Gln
   20 25 30
   Lys Ile Ile Ile Arg Asp Tyr His Glu Thr Leu Asn Val His Lys Leu
   35 40 45
   Asp His
   50
<210> 460
   <211> 43
   <212> PRT
   <213> Plasmodium falciparum
<400> 460
   Lys Arg Glu Lys Thr Gln Ile Asn Lys Thr Lys Tyr Glu Arg Gly Asp
   1 5 10 15
   Val Ile Ile Asp Asn Thr Glu Ile Gln Lys Ile Ile Ile Arg Asp Tyr
   20 25 30
   His Glu Thr Leu Asn Val His Lys Leu Asp His
   35 40
<210> 461
   <211> 40
   <212> PRT
   <213> Plasmodium falciparum
<400> 461
   Lys Thr Gln Ile Asn Lys Thr Lys Tyr Glu Arg Gly Asp Val Ile Ile
   1               5                   10                  15
   Asp Asn Thr Glu Ile Gln Lys Ile Ile Ile Arg Asp Tyr His Glu Thr
               20                  25                  30
   Leu Asn Val His Lys Leu Asp His
           35                  40
<210> 462
   <211> 46
   <212> PRT
   <213> Plasmodium falciparum
<400> 462
   Lys Pro Leu Ala Lys Leu Arg Lys Arg Glu Lys Thr Gln Ile Asn Lys
   1               5                   10                  15
   Thr Lys Tyr Glu Arg Gly Asp Val Ile Ile Asp Asn Thr Glu Ile Gln
               20                  25                  30
   Lys Ile Ile Ile Arg Asp Tyr His Glu Thr Leu Asn Val His
           35                  40                  45
<210> 463
   <211> 40
   <212> PRT
   <213> Plasmodium falciparum
<400> 463
   Lys Pro Leu Ala Lys Leu Arg Lys Arg Glu Lys Thr Gln Ile Asn Lys
   1               5                   10                  15
   Thr Lys Tyr Glu Arg Gly Asp Val Ile Ile Asp Asn Thr Glu Ile Gln
               20                  25                  30
   Lys Ile Ile Ile Arg Asp Tyr His
           35                  40
<210> 464
   <211> 36
   <212> PRT
   <213> Plasmodium falciparum
<400> 464
   Lys Leu Arg Lys Arg Glu Lys Thr Gln Ile Asn Lys Thr Lys Tyr Glu
   1               5                   10                  15
   Arg Gly Asp Val Ile Ile Asp Asn Thr Glu Ile Gln Lys Ile Ile Ile
               20                  25                  30
   Arg Asp Tyr His
           35
<210> 465
   <211> 33
   <212> PRT
   <213> Plasmodium falciparum
<400> 465
   Lys Arg Glu Lys Thr Gln Ile Asn Lys Thr Lys Tyr Glu Arg Gly Asp
   1               5                   10                  15
   Val Ile Ile Asp Asn Thr Glu Ile Gln Lys Ile Ile Ile Arg Asp Tyr
               20                  25                  30
   His
<210> 466
   <211> 30
   <212> PRT
   <213> Plasmodium falciparum
<400> 466
   Lys Thr Gln Ile Asn Lys Thr Lys Tyr Glu Arg Gly Asp Val Ile Ile
   1               5                   10                  15
   Asp Asn Thr Glu Ile Gln Lys Ile Ile Ile Arg Asp Tyr His
               20                  25                  30
<210> 467
   <211> 41
   <212> PRT
   <213> Plasmodium falciparum
<400> 467
   Lys Lys Asp Lys Glu Lys Lys Lys Asp Ser Asn Glu Asn Arg Lys Lys
   1               5                   10                  15
   Lys Gln Lys Glu Asp Lys Lys Asn Pro Asn Asp Asn Lys Leu Lys Lys
               20                  25                  30
   Ile Glu Tyr Thr Asn Lys Ile Thr His
           35                  40
<210> 468
   <211> 40
   <212> PRT
   <213> Plasmodium falciparum
<400> 468
   Lys Asp Lys Glu Lys Lys Lys Asp Ser Asn Glu Asn Arg Lys Lys Lys
   1 5 10 15
   Gln Lys Glu Asp Lys Lys Asn Pro Asn Asp Asn Lys Leu Lys Lys Ile
   20 25 30
   Glu Tyr Thr Asn Lys Ile Thr His
   35 40
<210> 469
   <211> 38
   <212> PRT
   <213> Plasmodium falciparum
<400> 469
   Lys Glu Lys Lys Lys Asp Ser Asn Glu Asn Arg Lys Lys Lys Gln Lys
   1 5 10 15
   Glu Asp Lys Lys Asn Pro Asn Asp Asn Lys Leu Lys Lys Ile Glu Tyr
   20 25 30
   Thr Asn Lys Ile Thr His
   35
<210> 470
   <211> 36
   <212> PRT
   <213> Plasmodium falciparum
<400> 470
   Lys Lys Lys Asp Ser Asn Glu Asn Arg Lys Lys Lys Gln Lys Glu Asp
   1 5 10 15
   Lys Lys Asn Pro Asn Asp Asn Lys Leu Lys Lys Ile Glu Tyr Thr Asn
   20 25 30
   Lys Ile Thr His
   35
<210> 471
   <211> 35
   <212> PRT
   <213> Plasmodium falciparum
<400> 471
   Lys Lys Asp Ser Asn Glu Asn Arg Lys Lys Lys Gln Lys Glu Asp Lys
   1 5 10 15
   Lys Asn Pro Asn Asp Asn Lys Leu Lys Lys Ile Glu Tyr Thr Asn Lys
   20 25 30
   Ile Thr His
   35
<210> 472
   <211> 34
   <212> PRT
   <213> Plasmodium falciparum
<400> 472
   Lys Asp Ser Asn Glu Asn Arg Lys Lys Lys Gln Lys Glu Asp Lys Lys
   1 5 10 15
   Asn Pro Asn Asp Asn Lys Leu Lys Lys Ile Glu Tyr Thr Asn Lys Ile
   20 25 30
   Thr His
<210> 473
   <211> 27
   <212> PRT
   <213> Plasmodium falciparum
<400> 473
   Lys Lys Lys Gln Lys Glu Asp Lys Lys Asn Pro Asn Asp Asn Lys Leu
   1 5 10 15
   Lys Lys Ile Glu Tyr Thr Asn Lys Ile Thr His
   20 25
<210> 474
   <211> 26
   <212> PRT
   <213> Plasmodium falciparum
<400> 474
   Lys Lys Gln Lys Glu Asp Lys Lys Asn Pro Asn Asp Asn Lys Leu Lys
   1 5 10 15
   Lys Ile Glu Tyr Thr Asn Lys Ile Thr His
   20 25
<210> 475
   <211> 25
   <212> PRT
   <213> Plasmodium falciparum
<400> 475
   Lys Gln Lys Glu Asp Lys Lys Asn Pro Asn Asp Asn Lys Leu Lys Lys
   1 5 10 15
   Ile Glu Tyr Thr Asn Lys Ile Thr His
   20 25
<210> 476
   <211> 23
   <212> PRT
   <213> Plasmodium falciparum
<400> 476
   Lys Glu Asp Lys Lys Asn Pro Asn Asp Asn Lys Leu Lys Lys Ile Glu
   1 5 10 15
   Tyr Thr Asn Lys Ile Thr His
   20
<210> 477
   <211> 20
   <212> PRT
   <213> Plasmodium falciparum
<400> 477
   Lys Lys Asn Pro Asn Asp Asn Lys Leu Lys Lys Ile Glu Tyr Thr Asn
   1 5 10 15
   Lys Ile Thr His
   20
<210> 478
   <211> 19
   <212> PRT
   <213> Plasmodium falciparum
<400> 478
   Lys Asn Pro Asn Asp Asn Lys Leu Lys Lys Ile Glu Tyr Thr Asn Lys
   1 5 10 15
   Ile Thr His
<210> 479
   <211> 13
   <212> PRT
   <213> Plasmodium falciparum
<400> 479
   Lys Leu Lys Lys Ile Glu Tyr Thr Asn Lys Ile Thr His
   1 5 10
<210> 480
   <211> 11
   <212> PRT
   <213> Plasmodium falciparum
<400> 480
   Lys Lys Ile Glu Tyr Thr Asn Lys Ile Thr His
   1 5 10
<210> 481
   <211> 10
   <212> PRT
   <213> Plasmodium falciparum
<400> 481
   Lys Ile Glu Tyr Thr Asn Lys Ile Thr His
   1 5 10
<210> 482
   <211> 44
   <212> PRT
   <213> Plasmodium falciparum
<400> 482
   His Gly Gln Ile Lys Ile Glu Asp Val Asn Asn Glu Asn Phe Asn Asn
   1 5 10 15
   Glu Gln Met Lys Asn Lys Tyr Asn Asp Glu Glu Lys Met Asp Ile Ser
   20 25 30
   Lys Ser Lys Ser Leu Lys Ser Asp Phe Leu Glu Lys
   35 40
<210> 483
   <211> 38
   <212> PRT
   <213> Plasmodium falciparum
<400> 483
   His Gly Gln Ile Lys Ile Glu Asp Val Asn Asn Glu Asn Phe Asn Asn
   1 5 10 15
   Glu Gln Met Lys Asn Lys Tyr Asn Asp Glu Glu Lys Met Asp Ile Ser
   20 25 30
   Lys Ser Lys Ser Leu Lys
   35
<210> 484
   <211> 35
   <212> PRT
   <213> Plasmodium falciparum
<400> 484
   His Gly Gln Ile Lys Ile Glu Asp Val Asn Asn Glu Asn Phe Asn Asn
   1 5 10 15
   Glu Gln Met Lys Asn Lys Tyr Asn Asp Glu Glu Lys Met Asp Ile Ser
   20 25 30
   Lys Ser Lys
   35
<210> 485
   <211> 33
   <212> PRT
   <213> Plasmodium falciparum
<400> 485
   His Gly Gln Ile Lys Ile Glu Asp Val Asn Asn Glu Asn Phe Asn Asn
   1 5 10 15
   Glu Gln Met Lys Asn Lys Tyr Asn Asp Glu Glu Lys Met Asp Ile Ser
   20 25 30
   Lys
<210> 486
   <211> 31
   <212> PRT
   <213> Plasmodium falciparum
<400> 486
   Lys Lys Tyr Asp Asp Leu Gln Asn Lys Tyr Asn Ile Leu Asn Lys Leu
   1 5 10 15
   Lys Asn Ser Leu Glu Glu Lys Asn Glu Glu Leu Lys Lys Tyr His
   20 25 30
<210> 487
   <211> 30
   <212> PRT
   <213> Plasmodium falciparum
<400> 487
   Lys Tyr Asp Asp Leu Gln Asn Lys Tyr Asn Ile Leu Asn Lys Leu Lys
   1 5 10 15
   Asn Ser Leu Glu Glu Lys Asn Glu Glu Leu Lys Lys Tyr His
   20 25 30
<210> 488
   <211> 23
   <212> PRT
   <213> Plasmodium falciparum
<400> 488
   Lys Tyr Asn Ile Leu Asn Lys Leu Lys Asn Ser Leu Glu Glu Lys Asn
   1               5                   10                  15
   Glu Glu Leu Lys Lys Tyr His
               20
<210> 489
   <211> 17
   <212> PRT
   <213> Plasmodium falciparum
<400> 489
   Lys Leu Lys Asn Ser Leu Glu Glu Lys Asn Glu Glu Leu Lys Lys Tyr
   1               5                   10                  15
   His
<210> 490
   <211> 15
   <212> PRT
   <213> Plasmodium falciparum
<400> 490
   Lys Asn Ser Leu Glu Glu Lys Asn Glu Glu Leu Lys Lys Tyr His
   1               5                   10                  15
<210> 491
   <211> 9
   <212> PRT
   <213> Plasmodium falciparum
<400> 491
   Lys Asn Glu Glu Leu Lys Lys Tyr His
   1               5
<210> 492
   <211> 44
   <212> PRT
   <213> Plasmodium falciparum
<400> 492
   His Met Gly Asn Asn Gln Asp Ile Asn Glu Asn Val Tyr Asn Ile Lys
   1 5 10 15
   Pro Gln Glu Phe Lys Glu Glu Glu Glu Glu Asp Ile Ser Met Val Asn
   20 25 30
   Thr Lys Lys Cys Asp Asp Ile Gln Glu Asn Ile Lys
   35 40
<210> 493
   <211> 50
   <212> PRT
   <213> Plasmodium falciparum
<400> 493
   Lys Thr Asn Leu Tyr Asn Ile Tyr Asn Asn Lys Asn Asp Asp Lys Asp
   1 5 10 15
   Asn Ile Leu Asp Asn Glu Asn Arg Glu Gly Leu Tyr Leu Cys Asp Val
   20 25 30
   Met Lys Asn Ser Asn Glu Leu Lys Arg Ile Asn Asp Asn Phe Phe Lys
   35 40 45
   Leu His
   50
<210> 494
   <211> 17
   <212> PRT
   <213> Plasmodium falciparum
<400> 494
   Lys Asn Ser Asn Glu Leu Lys Arg Ile Asn Asp Asn Phe Phe Lys Leu
   1 5 10 15
   His
<210> 495
   <211> 11
   <212> PRT
   <213> Plasmodium falciparum
<400> 495
   Lys Arg Ile Asn Asp Asn Phe Phe Lys Leu His
   1 5 10
<210> 496
   <211> 40
   <212> PRT
   <213> Plasmodium falciparum
<400> 496
   His Ile Asn Asn Glu Tyr Thr Asn Lys Asn Pro Lys Asn Cys Leu Leu
   1 5 10 15
   Tyr Lys Asn Glu Glu Arg Asn Tyr Asn Asp Asn Asn Ile Lys Asp Tyr
   20 25 30
   Ile Asn Ser Met Asn Phe Lys Lys
   35 40
<210> 497
   <211> 39
   <212> PRT
   <213> Plasmodium falciparum
<400> 497
   His Ile Asn Asn Glu Tyr Thr Asn Lys Asn Pro Lys Asn Cys Leu Leu
   1 5 10 15
   Tyr Lys Asn Glu Glu Arg Asn Tyr Asn Asp Asn Asn Ile Lys Asp Tyr
   20 25 30
   Ile Asn Ser Met Asn Phe Lys
   35
<210> 498
   <211> 18
   <212> PRT
   <213> Plasmodium falciparum
<400> 498
   His Ile Asn Asn Glu Tyr Thr Asn Lys Asn Pro Lys Asn Cys Leu Leu
   1 5 10 15
   Tyr Lys
<210> 499
   <211> 45
   <212> PRT
   <213> Plasmodium falciparum
<400> 499
   Lys Pro Cys Leu Tyr Lys Lys Cys Lys Ile Ser Gln Val Trp Trp Cys
   1 5 10 15
   Met Pro Val Lys Asp Thr Phe Asn Thr Tyr Glu Arg Asn Asn Val Leu
   20 25 30
   Asn Ser Lys Ile Glu Asn Asn Ile Glu Lys Ile Pro His
   35 40 45
<210> 500
   <211> 39
   <212> PRT
   <213> Plasmodium falciparum
<400> 500
   Lys Cys Lys Ile Ser Gln Val Trp Trp Cys Met Pro Val Lys Asp Thr
   1 5 10 15
   Phe Asn Thr Tyr Glu Arg Asn Asn Val Leu Asn Ser Lys Ile Glu Asn
   20 25 30
   Asn Ile Glu Lys Ile Pro His
   35
<210> 501
   <211> 11
   <212> PRT
   <213> Plasmodium falciparum
<400> 501
   Lys Ile Glu Asn Asn Ile Glu Lys Ile Pro His
   1 5 10
<210> 502
   <211> 23
   <212> PRT
   <213> Plasmodium falciparum
<400> 502
   Lys Asn Lys Thr Asn Gly Ser Lys Gly Val Lys Gly Glu Tyr Glu Lys
   1 5 10 15
   Lys Lys Glu Thr Asn Gly His
   20
<210> 503
   <211> 21
   <212> PRT
   <213> Plasmodium falciparum
<400> 503
   Lys Thr Asn Gly Ser Lys Gly Val Lys Gly Glu Tyr Glu Lys Lys Lys
   1 5 10 15
   Glu Thr Asn Gly His
   20
<210> 504
   <211> 16
   <212> PRT
   <213> Plasmodium falciparum
<400> 504
   Lys Gly Val Lys Gly Glu Tyr Glu Lys Lys Lys Glu Thr Asn Gly His
   1 5 10 15
<210> 505
   <211> 13
   <212> PRT
   <213> Plasmodium falciparum
<400> 505
   Lys Gly Glu Tyr Glu Lys Lys Lys Glu Thr Asn Gly His
   1               5                   10
<210> 506
   <211> 60
   <212> PRT
   <213> Plasmodium falciparum
<400> 506
   Lys Thr Ile Glu Lys Ile Asn Lys Ser Lys Ser Trp Phe Phe Glu Glu
   1               5                   10                  15
   Leu Asp Glu Ile Asp Lys Pro Leu Ala Lys Leu Arg Lys Arg Glu Lys
               20                  25                  30
   Thr Gln Ile Asn Lys Thr Lys Tyr Glu Arg Gly Asp Val Ile Ile Asp
           35                  40                  45
   Asn Thr Glu Ile Gln Lys Ile Ile Arg Asp Tyr His
       50                  55                  60
<210> 507
   <211> 56
   <212> PRT
   <213> Plasmodium falciparum
<400> 507
   Lys Ile Asn Lys Ser Lys Ser Trp Phe Phe Glu Glu Leu Asp Glu Ile
   1               5                   10                  15
   Asp Lys Pro Leu Ala Lys Leu Arg Lys Arg Glu Lys Thr Gln Ile Asn
               20                  25                  30
   Lys Thr Lys Tyr Glu Arg Gly Asp Val Ile Ile Asp Asn Thr Glu Ile
           35                  40                  45
   Gln Lys Ile Ile Arg Asp Tyr His
       50                  55
<210> 508
   <211> 39
   <212> PRT
   <213> Plasmodium falciparum
<400> 508
   Lys Pro Leu Ala Lys Leu Arg Lys Arg Glu Lys Thr Gln Ile Asn Lys
   1               5                   10                  15
   Thr Lys Tyr Glu Arg Gly Asp Val Ile Ile Asp Asn Thr Glu Ile Gln
               20                  25                  30
   Lys Ile Ile Arg Asp Tyr His
           35
<210> 509
   <211> 32
   <212> PRT
   <213> Plasmodium falciparum
<400> 509
   His Ile Met Leu Lys Ser Gln Met Tyr Thr Asn Glu Gly Asn Lys Ser
   1               5                   10                  15
   Cys Glu Cys Ser Tyr Lys Lys Lys Ser Ser Ser Ser Asn Lys Val His
               20                  25                  30
<210> 510
   <211> 35
   <212> PRT
   <213> Plasmodium falciparum
<400> 510
   Lys Leu Arg Lys Arg Glu Lys Thr Gln Ile Asn Lys Thr Lys Tyr Glu
   1               5                   10                  15
   Arg Gly Asp Val Ile Ile Asp Asn Thr Glu Ile Gln Lys Ile Ile Arg
               20                  25                  30
   Asp Tyr His
           35
<210> 511
   <211> 32
   <212> PRT
   <213> Plasmodium falciparum

<400> 511
   Lys Arg Glu Lys Thr Gln Ile Asn Lys Thr Lys Tyr Glu Arg Gly Asp
   1               5                   10                  15
   Val Ile Ile Asp Asn Thr Glu Ile Gln Lys Ile Ile Arg Asp Tyr His
               20                  25                  30
<210> 512
   <211> 29
   <212> PRT
   <213> Plasmodium falciparum
<400> 512
   Lys Thr Gln Ile Asn Lys Thr Lys Tyr Glu Arg Gly Asp Val Ile Ile
   1 5 10 15
   Asp Asn Thr Glu Ile Gln Lys Ile Ile Arg Asp Tyr His
   20 25
<210> 513
   <211> 48
   <212> PRT
   <213> Plasmodium falciparum
<400> 513
   Lys Pro Leu Ala Lys Leu Arg Lys Arg Glu Lys Thr Gln Ile Asn Lys
   1 5 10 15
   Thr Lys Tyr Glu Arg Gly Asp Val Ile Ile Asp Asn Thr Glu Ile Gln
   20 25 30
   Lys Ile Ile Arg Asp Tyr His Thr Leu Asn Val His Lys Leu Asp His
   35 40 45
<210> 514
   <211> 44
   <212> PRT
   <213> Plasmodium falciparum
<400> 514
   Lys Leu Arg Lys Arg Glu Lys Thr Gln Ile Asn Lys Thr Lys Tyr Glu
   1 5 10 15
   Arg Gly Asp Val Ile Ile Asp Asn Thr Glu Ile Gln Lys Ile Ile Arg
   20 25 30
   Asp Tyr His Thr Leu Asn Val His Lys Leu Asp His
   35 40
<210> 515
   <211> 41
   <212> PRT
   <213> Plasmodium falciparum
<400> 515
   Lys Arg Glu Lys Thr Gln Ile Asn Lys Thr Lys Tyr Glu Arg Gly Asp
   1 5 10 15
   Val Ile Ile Asp Asn Thr Glu Ile Gln Lys Ile Ile Arg Asp Tyr His
   20 25 30
   Thr Leu Asn Val His Lys Leu Asp His
   35 40
<210> 516
   <211> 38
   <212> PRT
   <213> Plasmodium falciparum
<400> 516
   Lys Thr Gln Ile Asn Lys Thr Lys Tyr Glu Arg Gly Asp Val Ile Ile
   1               5                   10                  15
   Asp Asn Thr Glu Ile Gln Lys Ile Ile Arg Asp Tyr His Thr Leu Asn
               20                  25                  30
   Val His Lys Leu Asp His
           35
<210> 517
   <211> 44
   <212> PRT
   <213> Plasmodium falciparum
<400> 517
   Lys Pro Leu Ala Lys Leu Arg Lys Arg Glu Lys Thr Gln Ile Asn Lys
   1               5                   10                  15
   Thr Lys Tyr Glu Arg Gly Asp Val Ile Ile Asp Asn Thr Glu Ile Gln
               20                  25                  30
   Lys Ile Ile Arg Asp Tyr His Thr Leu Asn Val His
           35                  40
<210> 518
   <211> 40
   <212> PRT
   <213> Plasmodium falciparum
<400> 518
   Lys Leu Arg Lys Arg Glu Lys Thr Gln Ile Asn Lys Thr Lys Tyr Glu
   1               5                   10                  15
   Arg Gly Asp Val Ile Ile Asp Asn Thr Glu Ile Gln Lys Ile Ile Arg
               20                  25                  30
   Asp Tyr His Thr Leu Asn Val His
           35                  40
<210> 519
   <211> 37
   <212> PRT
   <213> Plasmodium falciparum
<400> 519
   Lys Arg Glu Lys Thr Gln Ile Asn Lys Thr Lys Tyr Glu Arg Gly Asp
   1 5 10 15
   Val Ile Ile Asp Asn Thr Glu Ile Gln Lys Ile Ile Arg Asp Tyr His
   20 25 30
   Thr Leu Asn Val His
   35
<210> 520
   <211> 34
   <212> PRT
   <213> Plasmodium falciparum
<400> 520
   Lys Thr Gln Ile Asn Lys Thr Lys Tyr Glu Arg Gly Asp Val Ile Ile
   1 5 10 15
   Asp Asn Thr Glu Ile Gln Lys Ile Ile Arg Asp Tyr His Thr Leu Asn
   20 25 30
   Val His
<210> 521
   <211> 32
   <212> PRT
   <213> Plasmodium falciparum
<400> 521
   His Ile Met Leu Lys Ser Gln Met Tyr Thr Asn Glu Gly Asn Lys Ser
   1 5 10 15
   Cys Glu Cys Ser Tyr Lys Lys Lys Ser Ser Ser Ser Asn Lys Val His
   20 25 30
<210> 522
   <211> 28
   <212> PRT
   <213> Plasmodium falciparum
<400> 522
   Lys Ser Gln Met Tyr Thr Asn Glu Gly Asn Lys Ser Cys Glu Cys Ser
   1 5 10 15
   Tyr Lys Lys Lys Ser Ser Ser Ser Asn Lys Val His
   20 25
<210> 523
   <211> 18
   <212> PRT
   <213> Plasmodium falciparum
<400> 523
   Lys Ser Cys Glu Cys Ser Tyr Lys Lys Lys Ser Ser Ser Ser Asn Lys
   1 5 10 15
   Val His
<210> 524
   <211> 11
   <212> PRT
   <213> Plasmodium falciparum
<400> 524
   Lys Lys Lys Ser Ser Ser Ser Asn Lys Val His
   1 5 10
<210> 525
   <211> 10
   <212> PRT
   <213> Plasmodium falciparum
<400> 525
   Lys Lys Ser Ser Ser Ser Asn Lys Val His
   1 5 10
<210> 526
   <211> 9
   <212> PRT
   <213> Plasmodium falciparum
<400> 526
   Lys Ser Ser Ser Ser Asn Lys Val His
   1 5
<210> 527
   <211> 30
   <212> PRT
   <213> Plasmodium falciparum
<400> 527
   His Ile Met Leu Lys Ser Gln Met Tyr Thr Asn Glu Gly Asn Lys Ser
   1 5 10 15
   Cys Glu Cys Ser Tyr Lys Lys Lys Ser Ser Ser Ser Asn Lys
   20 25 30
<210> 528
   <211> 24
   <212> PRT
   <213> Plasmodium falciparum
<400> 528
   His Ile Met Leu Lys Ser Gln Met Tyr Thr Asn Glu Gly Asn Lys Ser
   1 5 10 15
   Cys Glu Cys Ser Tyr Lys Lys Lys
   20
<210> 529
   <211> 23
   <212> PRT
   <213> Plasmodium falciparum
<400> 529
   His Ile Met Leu Lys Ser Gln Met Tyr Thr Asn Glu Gly Asn Lys Ser
   1 5 10 15
   Cys Glu Cys Ser Tyr Lys Lys
   20
<210> 530
   <211> 22
   <212> PRT
   <213> Plasmodium falciparum
<400> 530
   His Ile Met Leu Lys Ser Gln Met Tyr Thr Asn Glu Gly Asn Lys Ser
   1 5 10 15
   Cys Glu Cys Ser Tyr Lys
   20
<210> 531
   <211> 36
   <212> PRT
   <213> Plasmodium falciparum
<400> 531
   His Asn Asn His Asn Ile Gln Ile Tyr Lys Asp Lys Arg Ile Asn Phe
   1 5 10 15
   Met Asn Pro His Lys Val Met Tyr His Asp Asn Met Ser Lys Asn Glu
   20 25 30
   Arg Thr Glu Lys
   35
<210> 532
   <211> 30
   <212> PRT
   <213> Plasmodium falciparum
<400> 532
   His Asn Asn His Asn Ile Gln Ile Tyr Lys Asp Lys Arg Ile Asn Phe
   1 5 10 15
   Met Asn Pro His Lys Val Met Tyr His Asp Asn Met Ser Lys
   20 25 30
<210> 533
   <211> 21
   <212> PRT
   <213> Plasmodium falciparum
<400> 533
   His Asn Asn His Asn Ile Gln Ile Tyr Lys Asp Lys Arg Ile Asn Phe
   1 5 10 15
   Met Asn Pro His Lys
   20
<210> 534
   <211> 17
   <212> PRT
   <213> Plasmodium falciparum
<400> 534
   His Lys Val Met Tyr His Asp Asn Met Ser Lys Asn Glu Arg Thr Glu
   1 5 10 15
   Lys
<210> 535
   <211> 11
   <212> PRT
   <213> Plasmodium falciparum
<400> 535
   His Lys Val Met Tyr His Asp Asn Met Ser Lys
   1 5 10

## Description

### FIELD OF THE INVENTION

This invention relates to the identification and use of Replikins, a class of peptides that share structural characteristics. In particular, this invention relates to Replikins which have been identified in H₁N₁ influenza viruses and their use in designing influenza virus vaccines.

### BACKGROUND OF THE INVENTION

Influenza is an acute respiratory illness of global importance. Despite international attempts to control influenza virus outbreaks through vaccination influenza infections remain an important cause of morbidity and mortality. Worldwide influenza pandemics have occurred at irregular and previously unpredictable intervals throughout history and it is expected that they will continue to occur in the future. The impact of pandemic influenza is substantial in terms of morbidity, mortality and economic cost.

Influenza vaccines remain the most effective defense against influenza virus, but because of the ability of the virus to mutate and the availability of non-human host reservoirs it is expected that influenza will remain an emergent or re-emergent infection. Global influenza surveillance indicates that influenza viruses may vary within a country and between countries and continents during an influenza season. Virologic surveillance is of importance in monitoring antigenic shift and drift. Disease surveillance is also important in assessing the impact of epidemics. Both types of information have provided the basis of vaccine composition and the correct use of antivirals. However, to date there has been only annual *post hoc* hematological classification of the increasing number of emerging influenza virus strains, and no specific chemical structure of the viruses has been identified as an indicator of approaching influenza epidemic or pandemic. Currently, the only basis for annual classification of influenza virus as active, inactive or prevalent in a given year is the activities of the virus hemagglutinin and neuraminidase proteins. No influenza viral chemical structure has been identified that can be used for quantitative warning of epidemics or pandemics or to design more effective and safer vaccines.

Because of the annual administration of influenza vaccines and the short period of time when a vaccine can be administered, strategies directed at improving vaccine coverage are of critical importance.

### SUMMARY OF THE INVENTION

The present invention is defined in the appended claims.
The present invention provides an isolated or synthesized H1N1 influenza virus Replikin peptide from a hemagglutinin protein wherein said peptide consists of 7 to 50 amino acids comprising (1) at least one lysine residue located six to ten residues from a second lysine residue; (2) at least one histidine residue; and (3) at least 6% lysine residues.
The invention also provides an antibody that specifically binds to H1N1 influenza virus Replikin peptides.
In one embodiment, the invention includes a composition comprising said antibody and a pharmaceutically acceptable carrier and/or adjuvant.
In another embodiment, the invention includes a therapeutic composition comprising an isolated or synthesized H1N1 influenza virus Replikin peptide and a pharmaceutically acceptable carrier and/or adjuvant.
In another aspect of the invention there is provided an antisense nucleic acid molecule complementary to an influenza virus hemagglutinin Replikin mRNA sequence encoding an H1N1 influenza virus Replikin peptide.
In yet another aspect of the invention there is provided a method of making an influenza virus vaccine comprising (1) identifying a strain of influenza virus as an emerging strain, (2) selecting at least one H1N1 influenza virus Replikin peptide present in the emerging strain as a peptide template for influenza virus vaccine manufacture, (3) synthesizing peptides having the amino acid sequence of the at least one H1N1 influenza virus Replikin peptide selected in step (2), and (4) combining a therapeutically effective amount of the peptides of step (3) with a pharmaceutically acceptable carrier and/or adjuvant.
In another embodiment, the invention includes an influenza virus vaccine comprising at least one isolated or synthesized H1N1 influenza virus Replikin peptide and a pharmaceutically acceptable carrier and/or adjuvant.

As used herein, the term "peptide" refers to a compound of two or more amino acids in which the carboxyl group of one is united with an amino group of another, forming a peptide bond. The term peptide is also used to denote the amino acid sequence encoding such a compound. Thus, a peptide sequence may be a subsequence of a larger polypeptide sequence. As used herein, a Replikin peptide is a peptide having 7 to about 50 amino acids comprising (1) at least one lysine residue located six to ten amino acid residues from a second lysine residue; (2) at least one histidine residue; and (3) at least 6% lysine residues. Similarly, a replikin sequence is the amino acid sequence encoding such a peptide.

The phrase "emerging strain" as used herein refers to a strain of influenza virus identified as having an increasing concentration of Replikin sequences in its hemagglutinin and/or neuraminidase protein sequence, relative to the concentration of replikins in other strains of influenza virus. The increase in concentration occurs over a period of at least about six months, and preferably over a period of at least about one year, most preferably over a period of at least about three years or more.

### BRIEF DESCRIPTION OF THE DRAWINGS

Figure 1 is a bar graph depicting the frequency of occurrence of replikins in various protein groups.

Figure 2 is a graph depicting the percentage of malignin per milligram total membrane protein during anaerobic replication of glioblastoma cells.

Figure 3 is a bar graph showing amount of antimalignin antibody produced in response to exposure to the recognin 16-mer.

Figure 4A is a photograph of a blood smear taken with ordinary and fluorescent light. Figure 4B is a photograph of a blood smear taken with ordinary and fluorescent light illustrating the presence of two leukemic cells. Figure 4C is a photograph of a dense layer of glioma cells in the presence of antimalignin antibody. Figure 4D and Figure 4E are photographs of the layer of cells in Figure 4C taken at 30 and 45 minutes following addition of antimalignin antibody.

Figure 4F is a bar graph showing the inhibition of growth of small cell lung carcinoma cells *in vitro* by antimalignin antibody.

Figure 5 is a plot of the amount of antimalignin antibody present in the serum of patients with benign or malignant breast disease pre-and post surgery.

Figure 6 is a box diagram depicting an embodiment of the invention wherein a computer is used to carry out the 3-point-recognition method of identifying replikin sequences.

Figure 7 is a graph showing the concentration of Replikins observed in hemagglutinin of influenza B and influenza A strain, H1N1, on a year by year basis from 1918 through 2001.

Figure 8 is a graph of the replikin concentration observed in hemagglutinin of influenza A strains, H2N2 and H3N2, as well as an emerging strain defined by its constituent Replikins, designated H3N2(R), on a year by year basis from 1950 to 2001.

### DETAILED DESCRIPTION OF THE INVENTION

The present invention provides methods for predicting future epidemics or pandemics of influenza virus, and vaccines and methods of designing effective vaccines against influenza virus. Identification of a new family of small peptides related to the phenomenon of rapid replication, referred to herein as Replikins, provides new targets for detection of pathogens in a sample and vaccine development, such as for example, influenza virus detection and influenza vaccine development.

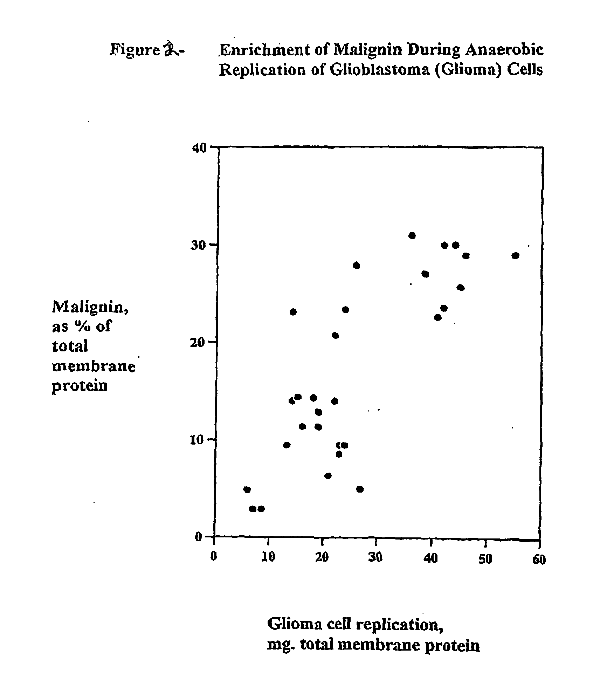

The first Replikin sequence to be identified was the cancer cell Replikin found in a brain cancer protein, malignin, which was demonstrated to be enriched ten-fold during rapid anaerobic replication of glioblastoma multiforme (glioma) cells. **(****Figure 2****)** Malignin is a 10KDa portion of the 250 KDa glycoprotein 10B, which was isolated *in vivo* and *in vitro* from membranes of glioblastoma multiforme (glioma) cells. Hydrolysis and mass spectroscopy of malignin revealed a16-mer peptide sequence, ykagvaflhkkndide (**SEQ ID NO.:4**), which includes the shorter peptide, kagvaflhkk (**SEQ ID NO.: 1**), SEQ ID NO.: 1 is referred to herein as the glioma Replikin, both of which apparently are absent in the normal human genome.

Table 1 illustrates how the sequence of the 16-mer peptide sequence, ykagvaflhkkndide (**SEQ ID NO.: 4**) was determined.

**Table 1**

| **16-mer peptide sequence ykagvaflhkkndide obtained from malignin by hydrolysis and mass spectrometry** | | | | | | | |
|---|---|---|---|---|---|---|---|
| Seq | Fragment | MH+ | Sequence | Method By Which Fragment Obtained | | | |
| ID NO. | Identified | (mass) | | Auto-hydrolysis of malignin free in solution | Auto-hydrolysis of malignin immobilized on bromoacetyl cellulose | Microwaved 5 seconds | Microwaved 30 seconds |
| 19 | 1-3 | 381.21 | Oyka(g) | | | | + |
| 20 | 1-5 | 537.30 | Oykagv(a) | | + | | |
| 21 | 2-6 | 445.28 | (y)kagva(f) | | + | | |
| 22 | 2-7 | 592.35 | (Y)kagvaf(1) | | | + | |
| 23 | 4-11 | 899.55 | (a)gvaflhkk(n) | | | | + |
| 24 | 5-7 | 336.19 | (g)vaf(1) | | | | + |
| 25 | 6-7 | 237.12 | (v)af(1) | + | | | |
| 26 | 6-10 | 615.36 | (v)aflhk(k) | | | | + |
| 27 | 6-10 | 615.36 | (v)aflhk(k) | + | | | |
| 28 | 6-12 | 857.50 | (v)aflhkkn(d) | | + | | |
| 29 | 6-12 | 857.50 | (v)afhkkn(d) | + | | | |
| 30 | 7-8 | 279.17 | (a)fl(h) | | | + | |
| 31 | 10-16 | 861.43 | (h)kkndide() | | + | | |
| 32 | 11-14 | 489.27 | (k)kndi(d) | | + | | |
| 33 | 12-15 | 476.2- | (k)ndid(e) | + | | | |

When the 16-mer peptide was synthesized and injected as a synthetic vaccine into rabbits, abundant antimalignin antibody was produced. (Bogoch et al., Cancer Detection and Prevention, 26(Supp. 1): 402 (2002). The concentration of antimalignin antibody in serum *in vivo* has been shown to relate quantitatively to the survival of cancer patients. (Bogoch et al., Protides of Biological Fluids, 31:739-747 (1984). *In vitro* antimalignin antibodies have been shown to be cytotoxic to cancer cells at a concentration of picograms (femtomolar) per cancer cell. (Bogoch et al., Cancer Detection and Prevention, 26(Supp. 1): 402 (2002).

Studies carried out by the inventors showed that the 16-mer peptide is not represented in the normal healthy human genome. Consequently, a search for the origin and possible homologues of the Replikin sequence was undertaken by analysis of published sequences of various organisms.

By using the 16-mer sequence as a template and constructing a recognition proteomic system to visually scan the amino acid sequences of proteins of several different organisms, a new class of peptides, the Replikins, was identified. By use of the "3-point recognition" method, described herein below, a new class of peptides was revealed in algae, yeast, fungi, amoebae, bacteria, plant and virus proteins having replication, transformation, or redox functions. Surprisingly, the Replikin peptides were found to be concentrated in larger 'replicating' and 'transforming' proteins (so designated by their investigators, See Table 2). No sequences were found to be identical to the malignin 16-mer peptide.

Table 2 illustrates several Replikin sequences that were identified by the 3-point recognition method of the invention.

**Table 2:**

| **Examples of Replikins in various organisms -** prototype: Glioma Replikin* kagvaflhkk (SEQ ID No.: 1) | | | |
|---|---|---|---|
| | | | |
| **Algae:** | SEQ ID NO. | | |
| | 34 | Caldophera prolifera | kaskftkh |
| | 35 | Isolepisprolifera | kaqaetgeikgh |
| **Yeast:** | 36 | Schizosaccharomyces pombe | ksfkypkkhk |
| | 37 | Oryza sativa | kkaygnelhk |
| | 2 | Sacch. cerevisiae replication binding protein | hsikrelgiifdk |
| **Fungi:** | | Isocitrate lyase ICI I,Penicillium marneffei | kvdivthqk |
| | 38 | DNA-dependent RNA polymerase 11, Diseula destructiva | kleedaayhrkk |
| | 39 | Ophiostoma novo-ulm 1,RNA in Dutch elm disease | kvilplrgnikgiffkh |
| | 40 | fungus | |
| **Amoeba:** | 41 | Entamoeba invadens, histone H2B | klilkgdlnkh |
| **Bacteria:** | 42 | Pribosomal protein replication factor, Helicobacter pylori Replication-associated protein Staph. aureus | ksvhaflk |
| | 10 | Mycoplasma pulmonic, chromosome replication | kkektthnk |
| | 43 | Macrophage infectivity potentiator, L. legionella | kvhffqlkk |
| | 90 | Bacillus anthracis | kihlisvkk |
| | 91 | Bacillus anthricis | hvkkekeknk |
| | 92 | Bacillus anthracis | khivkievk |
| | 93 | Bacillus anthracis | kkkkikdiygkdallh |
| | 94 | Bacillus anthracis | kwekikqh |
| | 95 | Bacillus anthracis | kklqipppiepkkddiih |
| | 96 | Bacillus anthracis | hnryasniesayllilnewknniqsdlikk |
| | 97 | Bacillus anthracis | havddyagylldkaqsdlvtnskk |
| | 98 | Bacillus anthracis | haerlkqknapk |
| **Plants:** | 44 | Arabidopsis thaliana, prolifera | kdhdfdgdk |
| | 45 | Arabidopsis thaliana, cytoplasmic ribosomal | kmkglkqkkah |
| | 46 | Arabidopsis thaliana, DNA binding protein | kelssttqeksh |
| **Viruses:** | 9 | Replication associated protein A [Maize streak virus] | kekkpskdeimrdiish |
| | 11 | Bovine herpes virus 4, DNA replication protein | hkinitngqk |
| | 12 | Meleagrid herpesvirus 1, replication binding protein | hkdlyrllmk |
| | 47 | Feline immunodeficiency | hlkdyklvk |
| | 3 | Foot and Mouth Disease (O) | hkqkivapvk |
| | 5 | HIV Type 1 | kcfncgkegh |
| | 7 | HIV Type 2 | kcwncgkegh |
| | 99 | Small Pox Virus (Variola) | khynnitwyk |
| | 100 | Small Pox Virus (Variola) | kysqtgkeliih |
| | 101 | Small Pox Virus (Variola) | hyddvrikndivvsrck |
| | 102 | Small Pox Virus (Variola | hrfldildski |
| | 103 | Small Pox Virus (Variola) | kerghnyyfek |
| **Tumor** | 48 | Rous sarcoma virus tyrosine-protein kinase | kklrhek |
| **Viruses:** | 49 | v-yes, avian sarcoma | kklrhdk |
| | 50 | c-yes, colon cancer, malignant melanoma | kklrhdk |
| | 51 | v-srcC, avian sarcoma | kklrhek |
| | 52 | c-src, colon, mammary, panrcreatic cancer | kklrhek |
| | 53 | Neuroblastoma RAS viral (v-ras) oncogene | kqahelak |
| | 54 | VP1 (major capsid protein) [Polyamavicus sp .] | kthrfskh |
| | 55 | Sindbis | knlhekik |
| | 56 | El [Human papilloamavirus type 71] | khrpllqlk |
| | 57 | v-erbB from AEV and c-erb | kspnhvk |
| | 58 | v-fms (feline sarcoma) | knihlekk |
| | 59 | c-fms (acute and chronic myelomonocytic tumors) | knihlekk |
| | 60 | large t-antigen I [Polyomavirus sp.1 | kphlaqslek |
| | 61 | middle t-antigen [Polyomavirus sp,1- | kqhrelkdk |
| | 62 | small t-antigen [Polyomavirus sp], | kqhrelkdk |
| | 63 | v-abl, murine acute leukemia | kvpvlisptlkh |
| | 64 | Human T-cell lymphotropic virus typo 2 | kslllevdkdish |
| | 65 | c-kit, GI tumors, small cell lung carcinoma | kagitimvkreyh |
| | 18 | Hepatitis C | hyppkpgcivpak |
| **Trans-** | 66 | Transforming protein myb | ksgkhlgk |
| **forming** | 67 | Transforming protein myc, Burkitt lymphoma | krreqlkhk |
| **Proteins:** | 68 | Ras-related GTP-binding protein | ksfevikvih |
| | 69 | Transforming protein ras (teratocarcinoma) | kkkhtvkk |
| | 70 | TRAF-associated NF·kB activator TANK | kaqkdhlsk |
| | 71 | RFP transforming protein | hlkrvkdlkk |
| | 72 | Transforming protein D (S.C.) | kygspkhrlik |
| | 73 | Papilloma virus type 11, transforming protein | klkhilgkarfik |
| | 74 | Protein tryosine kinase (EC 27.1.112slk | kgdhvkhykirk |
| | 75 | Transforming protein (axl(-)) | kelklrdvmvdrhk |
| | 76 | Transforming protein (N-myc) | klqarqqqllkkieh |
| | 77 | Fibroblast growth factor 4 (Kaposi sarcoma) | kkgnsptmkvth |
| **Cancer** | 78 | Matrix metaloproteinase 7 (uterine) | keiplhfrk |
| **Cell** | 79 | Transcription factor 7-like | kkkphikk |
| **Proteins:** | 80 | Breast cancer antigen NY-BR-87 | ktrhdplak |
| | 81 | BRCA-1-Associated Ring Domain Protein (breast) | khhpkdnlik |
| | 82 | 'Autoantigen from a breast tumor' | khkrkkfrqk |
| | 83 | Glioma Replikin (this study) | kagvaflhkk |
| | 84 | Ovarian cancer antigen | khkrkkfrqk |
| | 85 | EE L leukemia | kkkskkhkdk |
| | 86 | Proto-oncogene tyrosine-protein kinase C-ABLE | hksekpalprk |
| | 87 | Adenomatosis polyposis coli | kkkkpsrlkgdnek |
| | 88 | Gastric cancer transforming protein | ktkkgnivsptmkvth |
| | 89 | Transforming protein (K-RAS 2B), lung | khkekmskdgkkkkkksk |

Identification of an amino acid sequence as a Replikin or as containing a Replikin, i. e., a homologue of the glioma Replikin, kagvaflhkk, requires that the three following requirements be met. The peptide sequence must have (1) at least one lysine residue located six to ten residues from another lysine residue; (2) at least one histidine residue; and (3) a composition of at least 6% lysine within an amino acid sequence of 7 to about 50 residues.

Databases were searched using the National Library of Medicine keyword "PubMed" descriptor for protein sequences containing Replikin sequences. Over 4,000 protein sequences were visually examined for homologues. Sequences of all individual proteins within each group of PubMed-classified proteins were visually scanned for peptides meeting the three above-listed requirements. An infrequent occurrence of homologues was observed in "virus peptides" as a whole (1.5%) (N=953), and in other peptides not designated as associated with malignant transformation or replication such as "brain peptides" and "neuropeptides" (together 8.5%) (N=845). However, surprisingly, homologues were significantly more frequently identified in large "replicating proteins," which were identified as having an established function in replication in bacteria, algae, and viruses. Even more surprising was the finding that Replikin homologues occurred in 100% of "tumor viruses" (N=250), in 97% of "cancer proteins" (N=401), and in 85% of "transforming viruses" (N=248). These results suggest that there are shared properties of cancer pathogenesis regardless of cell type and suggest a role of viruses in carcinogenesis, i.e., conversion of cells from a transformed albeit dormant state to a more virulent actively replicating state.

To permit classification of subtypes of Replikins, additional or "auxiliary specifications" to the basic "3-point-recognition" requirements may be added: (a) on a structural basis, such as the common occurrence of adjacent di- and polylysines in cancer cell proteins (*e.g*., transforming protein P21B(K-RAS 2B), lung, Table 2, **SEQ ID NO.: 89**), and other adjacent di-amino acids in TOLL-like receptors, or b) on a functional basis, such as exhibiting ATPase, tyrosine kinase or redox activity as seen in Table 2.

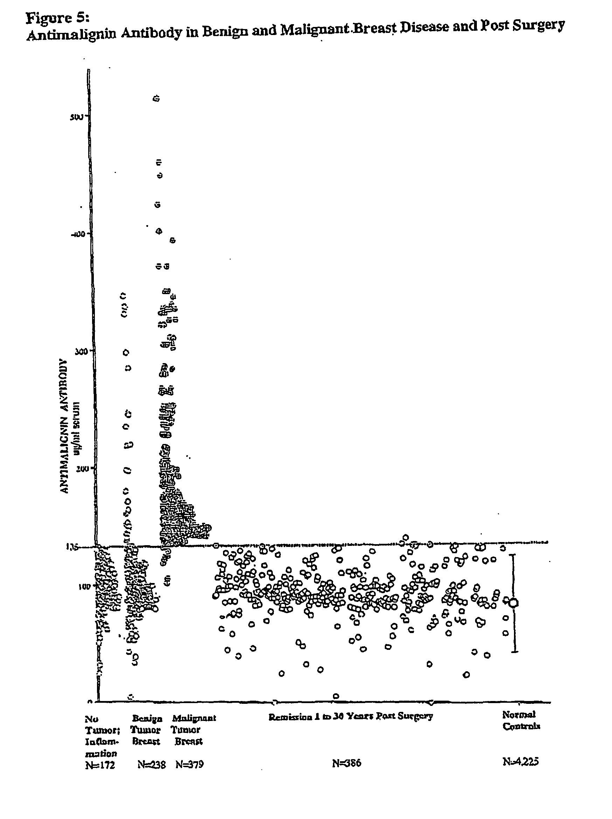

Whether Replikin structures are conserved or are subject to extensive natural mutation was examined by scanning the protein sequences of various isolates of foot and mouth disease virus (FMDV), where mutations in proteins of these viruses have been well documented worldwide for decades. Protein sequences of FMDV isolates were visually examined for the presence of both the entire Replikin and each of the component Replikin amino acid residues observed in a particular Replikin. For example, in the protein VP1 of FMDV type O, the Replikin (**SEQ ID NO.: 3**) "hkqkivapvk" was found to be conserved in 78% of the 236 isolates reported in PubMed, and each amino acid was found to be conserved in individual isolates as follows: his, 95.6%; lys, 91.8%; gln 92.3%; lys, 84.1%; ile, 90.7%; val, 91.8%; ala, 97.3%; pro, 96.2%; ala, 75.4%; and lys, 88.4%. The high rate of conservation suggests structural and functional stability of the Replikin structure. Similarly, sequence conservation was observed in different isolates of HIV for its Replikins, such as (**SEQ ID NO.: 5**) **"kcfncgkegh"** or (**SEQ ID NO.: 6**) **"kvylawvpahk"** in HIV Type 1 and (**SEQ ID NO.: 7**) "kcwncgkegh" in HIV Type 2 (Table 2). Other examples of conservation are seen in the constant presence of malignin in successive generations, over ten years of tissue culture of glioma cells, and by the constancy of affinity of the glioma Replikin for antimalignin antibody isolated by immunoadsorption from 8,090 human sera from the U.S., U.K., Europe and Asia (e.g., **Figure 5** and U.S. Patent 6,242,578 B1).

As seen in **Figure 2****,** during anaerobic respiration when the rate of cell replication is increased, malignin is enriched. That is, malignin is found to increase not simply in proportion to the increase in cell number and total membrane proteins, but is enriched as much as tenfold in concentration, starting with 3% at rest and reaching 30% of total membrane protein. This clear demonstration of a marked increase in Replikin concentration with glioma cell replication points to and is consistent with the presence of Replikins here sought by the 3-point recognition method and found in the proteins of various organisms which were found by mutation studies and other previous studies to be critical to replication. For example, Replikins were identified in such proteins as *"Saccharomyces cerevisiae* replication binding protein" (**SEQ ID NO.: 2**) (**hsikrelgiifdk**); the "replication associated protein A of maize streak virus" (**SEQ ID NO.: 8**) (**kyivcareahk**) and (**SEQ ID NO.: 9**) (**kekkpskdeimrdiish**); the "replication-associated protein of *Staphylococcus aureus"* (**SEQ ID NO.:10**) ( **kkektthnk**); the "DNA replication protein of bovine herpes virus 4" (**SEQ ID NO**.: **11**) (**hkinitngqk**); and the "Mealigrid herpes virus 1 replication binding protein" (**SEQ ID NO.: 12**) (hkdlyrllmk). Previous studies of tomato leaf curl gemini virus show that the regulation of virus accumulation appears to involve binding of amino acids 1-160 of the "replicating protein" of that virus to leaf DNA and to other replication protein molecules during virus replication. Analysis of this sequence showed that amino acids 1-163 of this "replicating protein" contain five Replikins, namely: (**SEQ ID NO.: 13**) **kfrinaknyfltyph**, (**SEQ ID NO.: 14**) **knletpvnklfiricrefh**, (**SEQ ID NO.: 15**) **hpniqaaksstdvk**, (**SEQ ID NO.: 16**) **ksstdvkaymdkdgdvldh**, and (**SEQ ID NO.: 17**) **kasalnilrekapkdfvlqfh**.

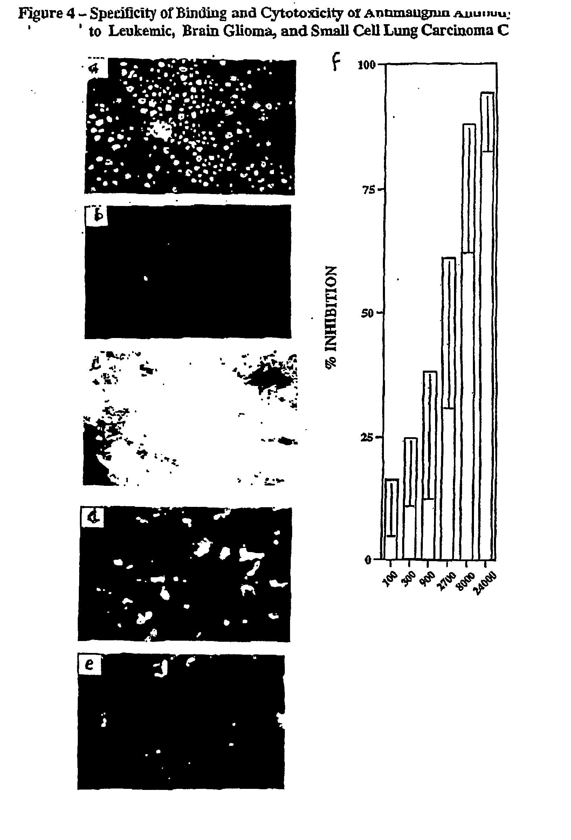

Table 2 shows that Replikin-containing proteins also are associated frequently with redox functions, and protein synthesis or elongation, as well as with cell replication. The association with metal-based redox functions, the enrichment of the Replikin-containing glioma malignin concentration during anaerobic replication, and the cytotoxicity of antimaligain at low concentrations (picograms/cell) (Figure 4c-f), all suggest that the Replikins are related to central respiratory functions, which are perhaps less often subjected to the mutations characteristic of proteins of more superficial location or less central survival function.

Of particular interest, it was observed that at least one Replikin per 100 amino acids was found to be present in the hemagglutinin proteins of almost all of the individual strains of influenza viruses examined. The replikin sequences that were observed to occur in the hemagglutinin proteins of isolates of each of the four prevalent strains of influenza virus, influenza B, H1N1, H2N2, and H3N2, for each year that amino acid sequence data are available (1902-2001) are shown in Tables 3, 4, 5 and 6, below.

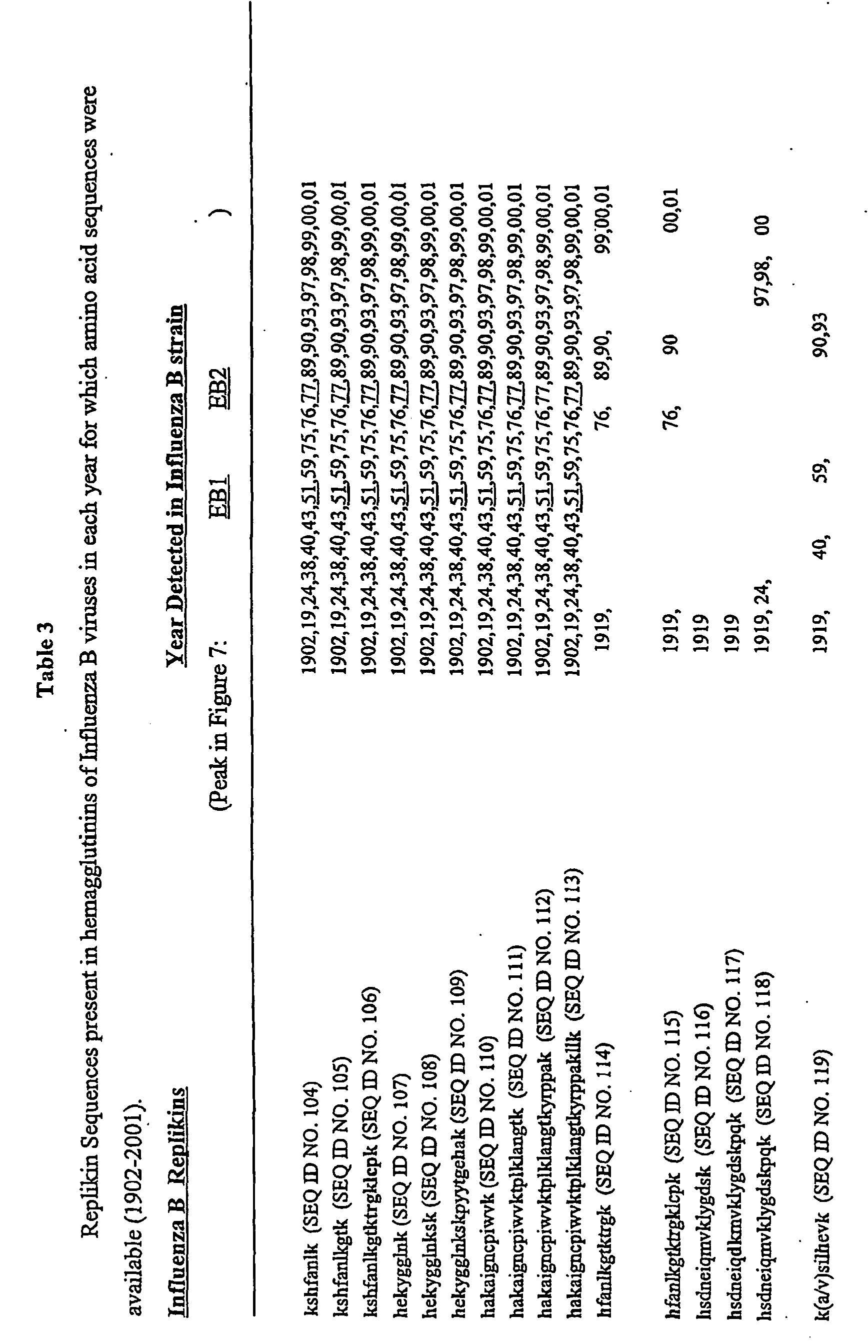

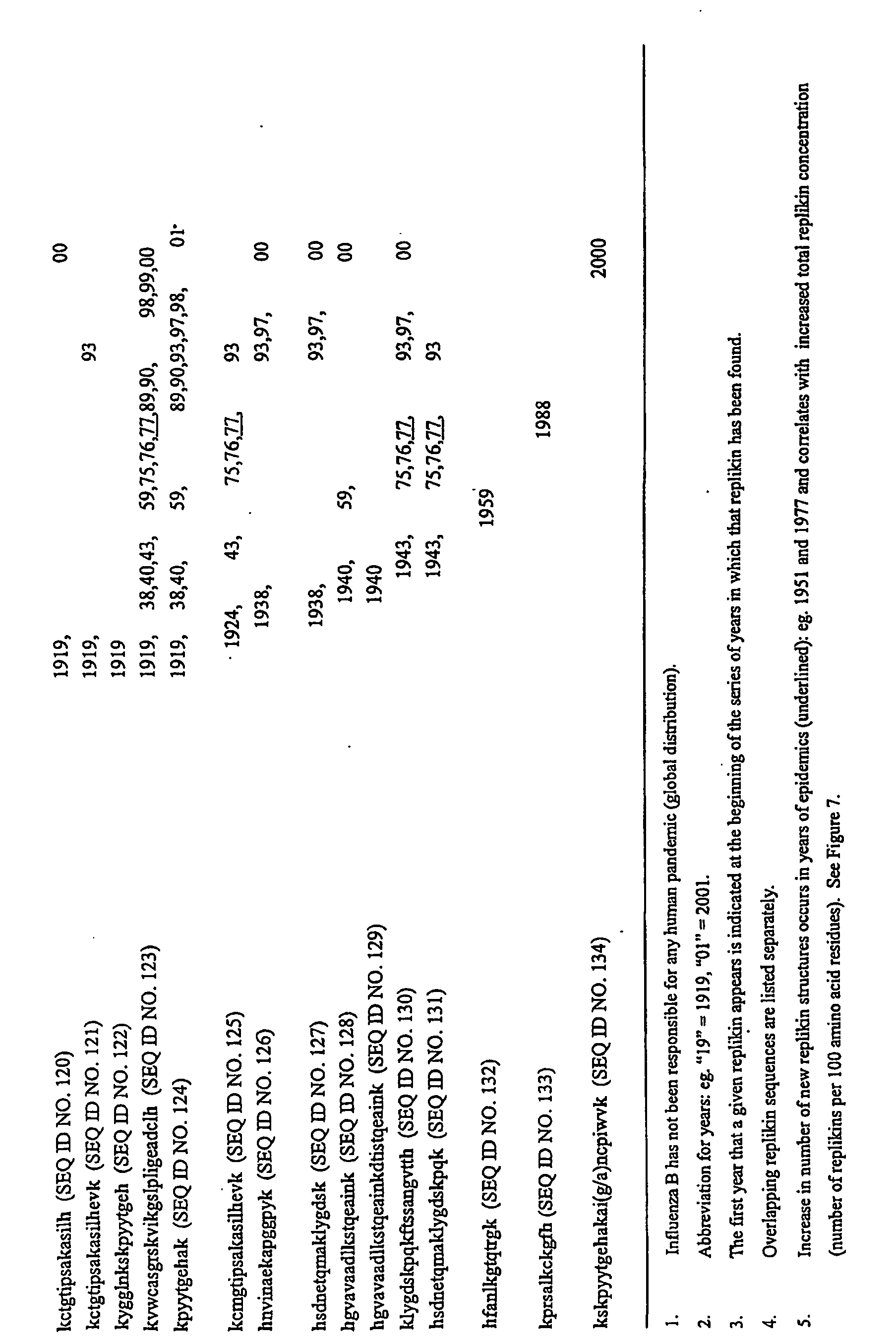

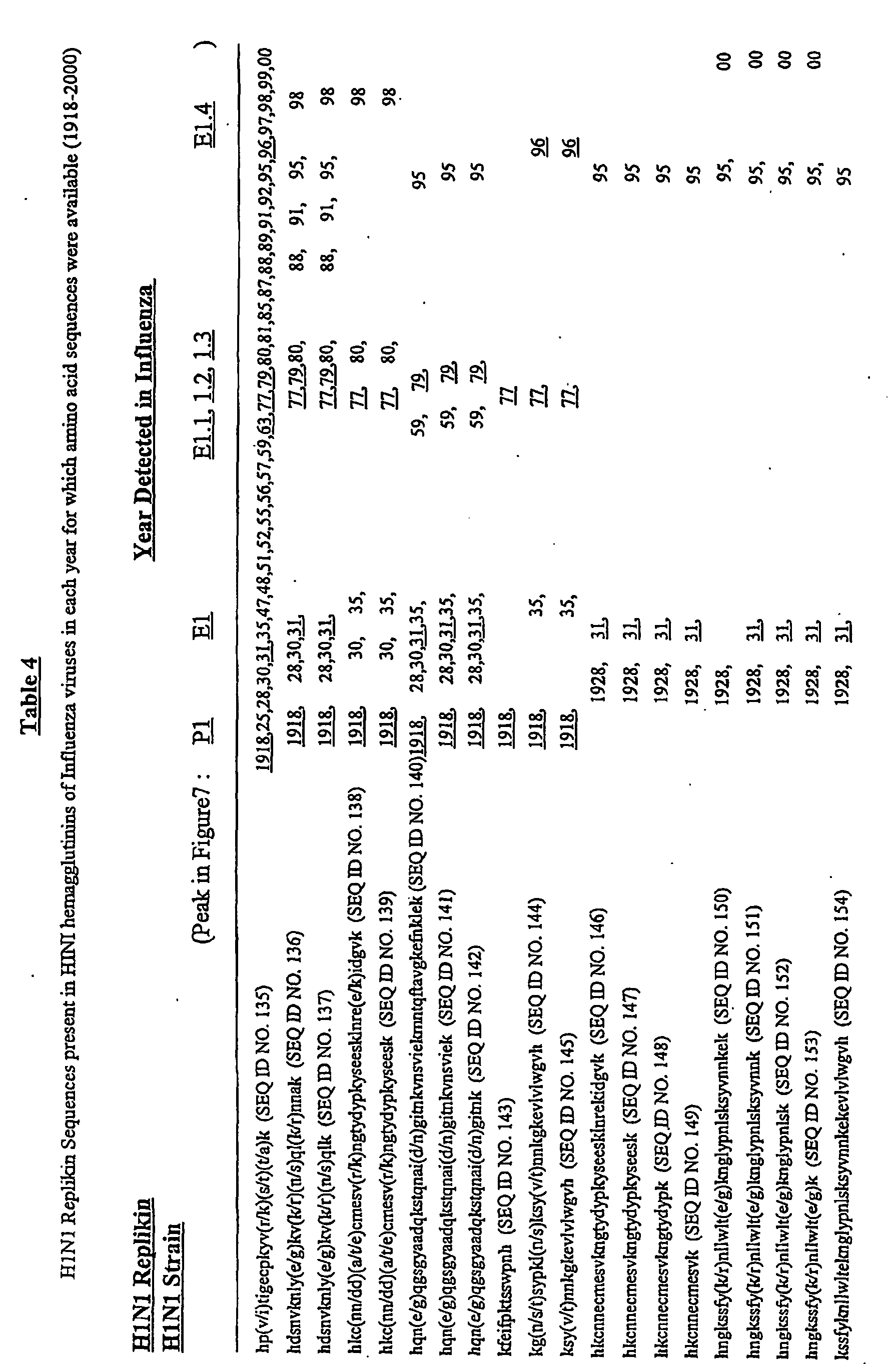

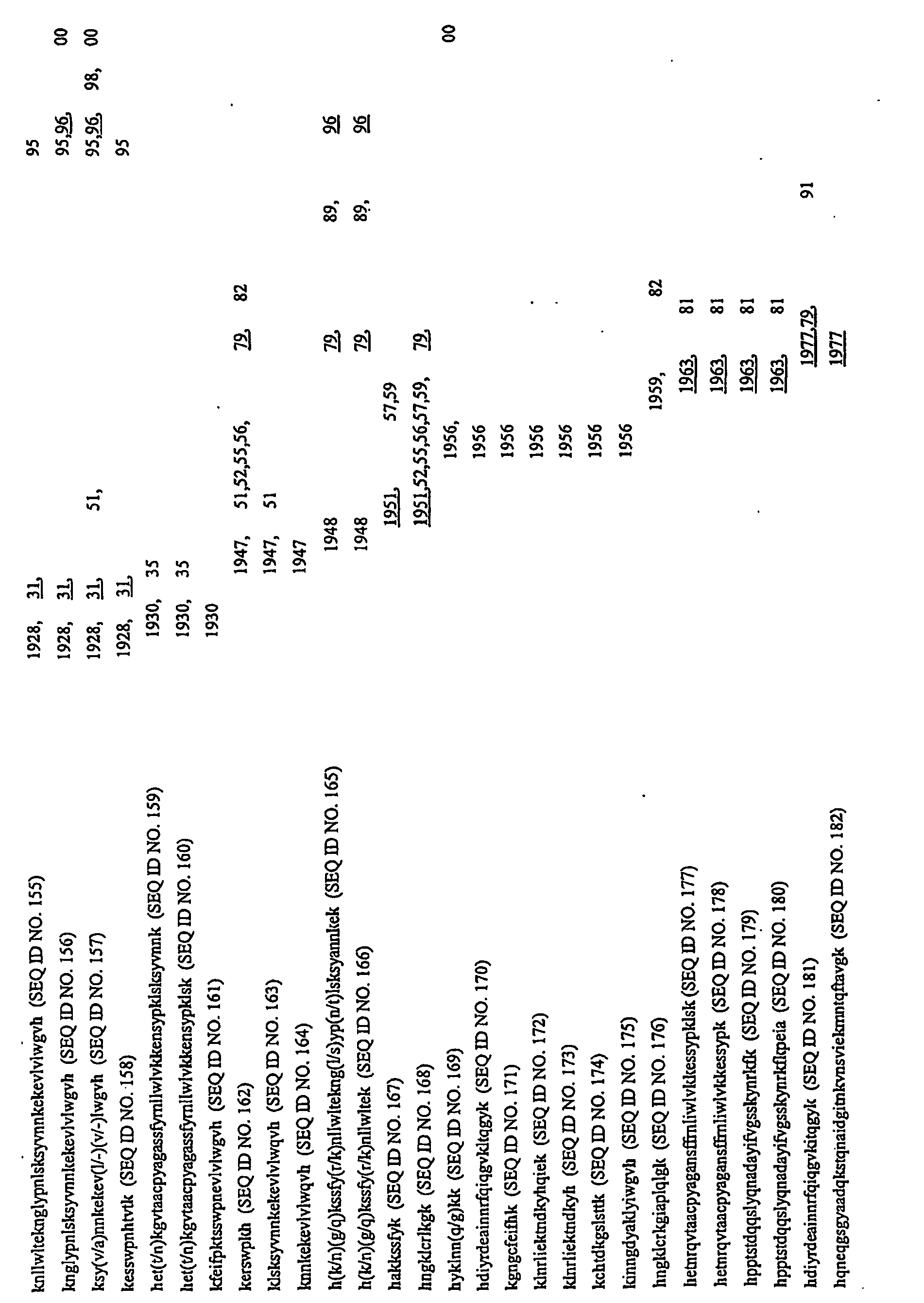

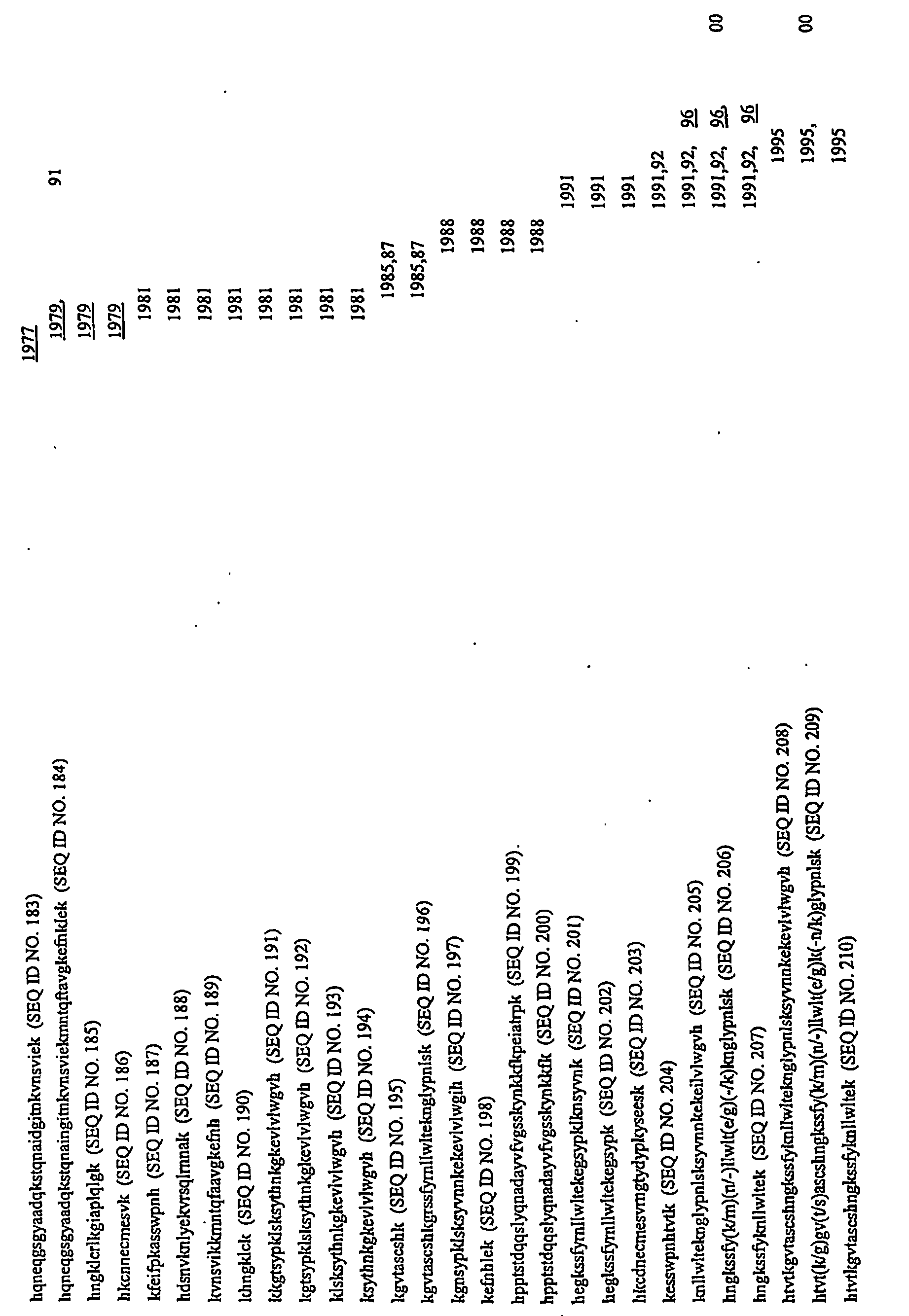

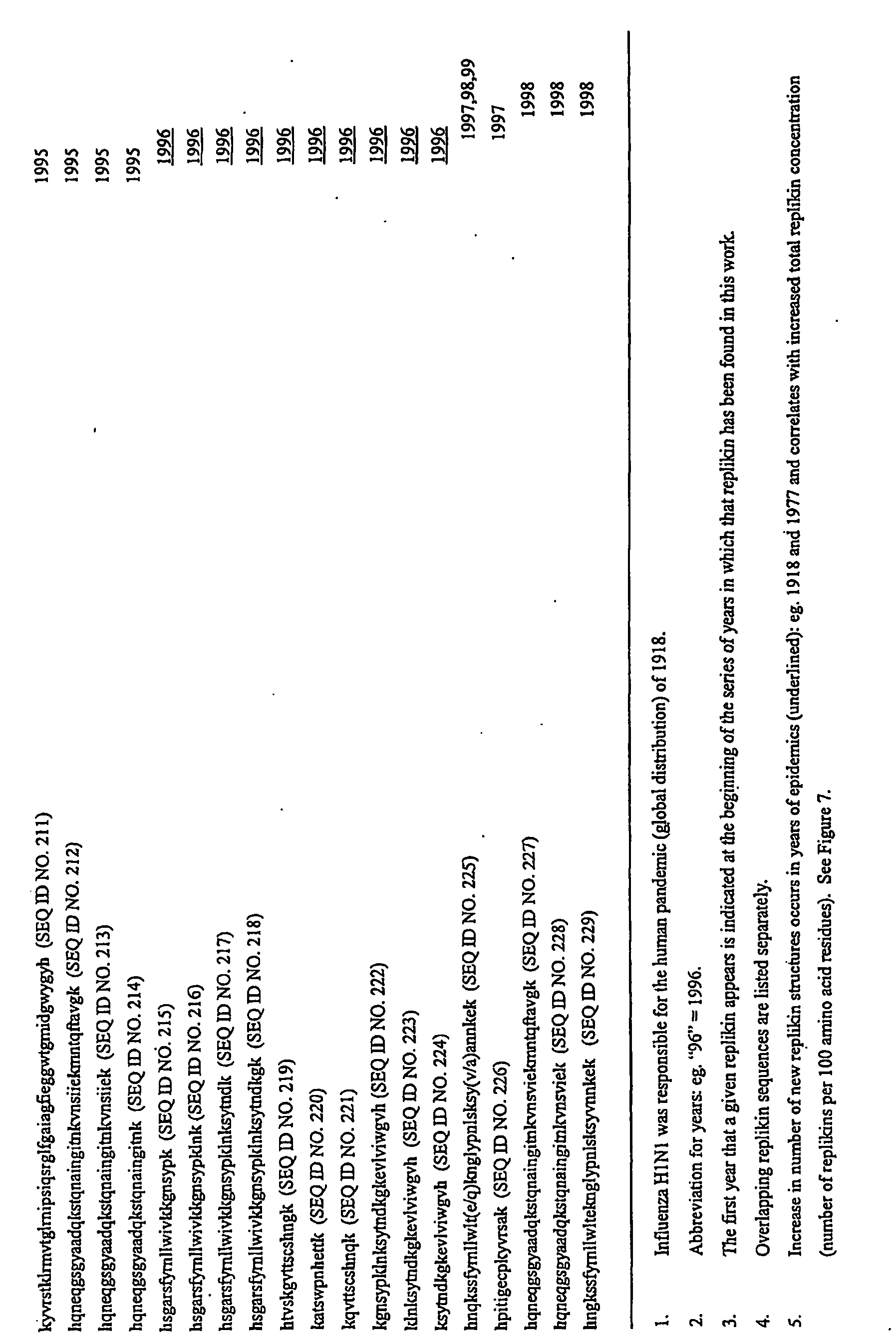

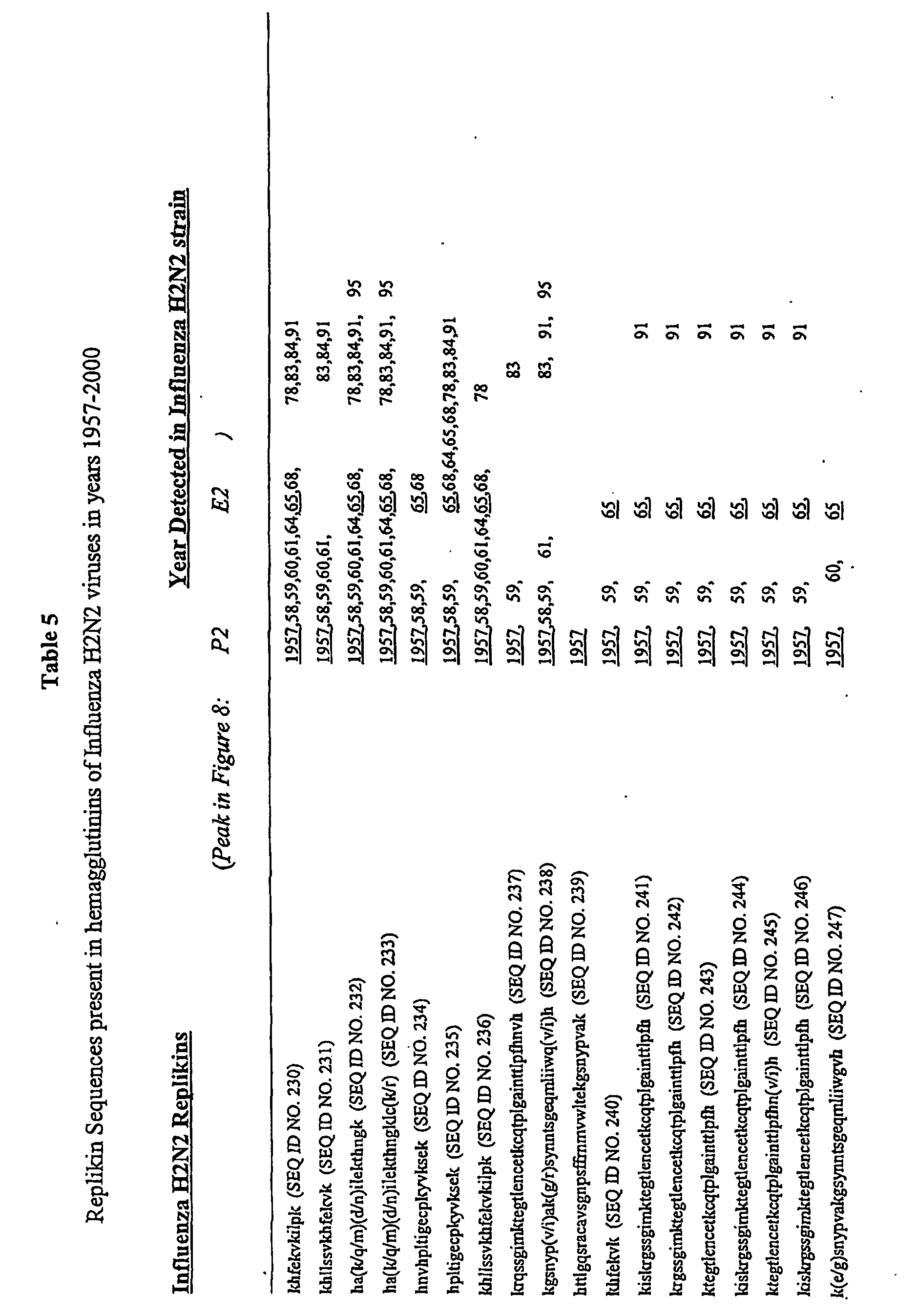

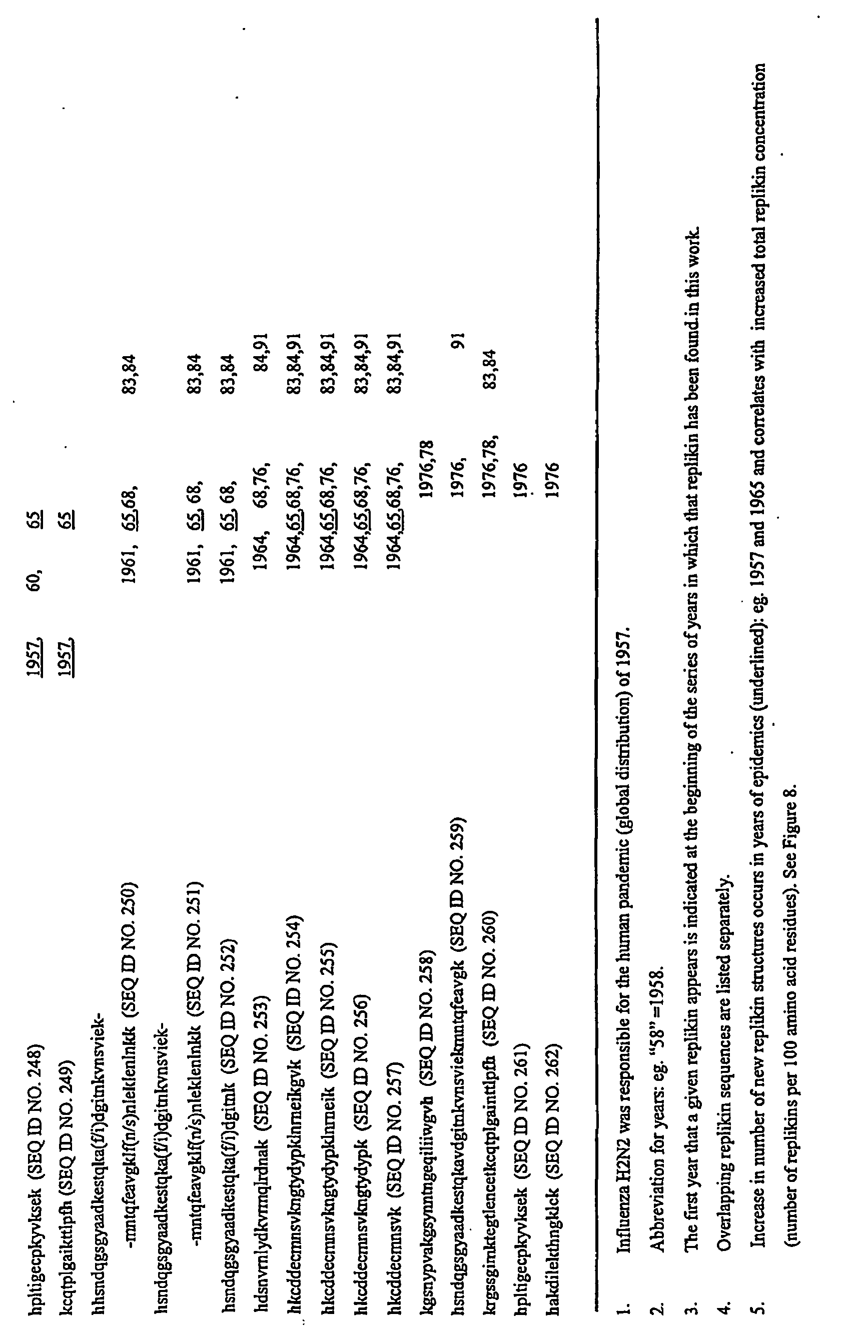

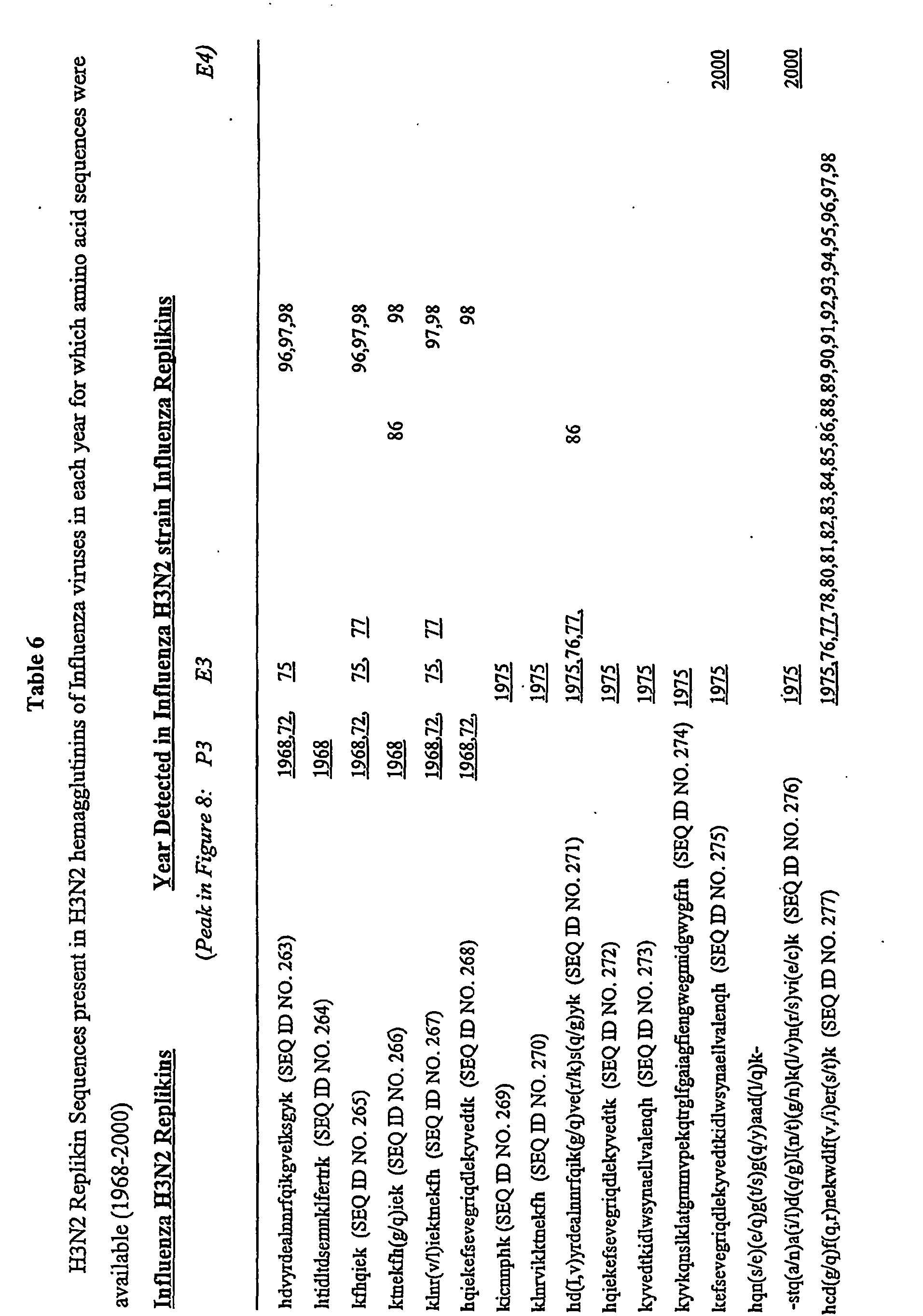

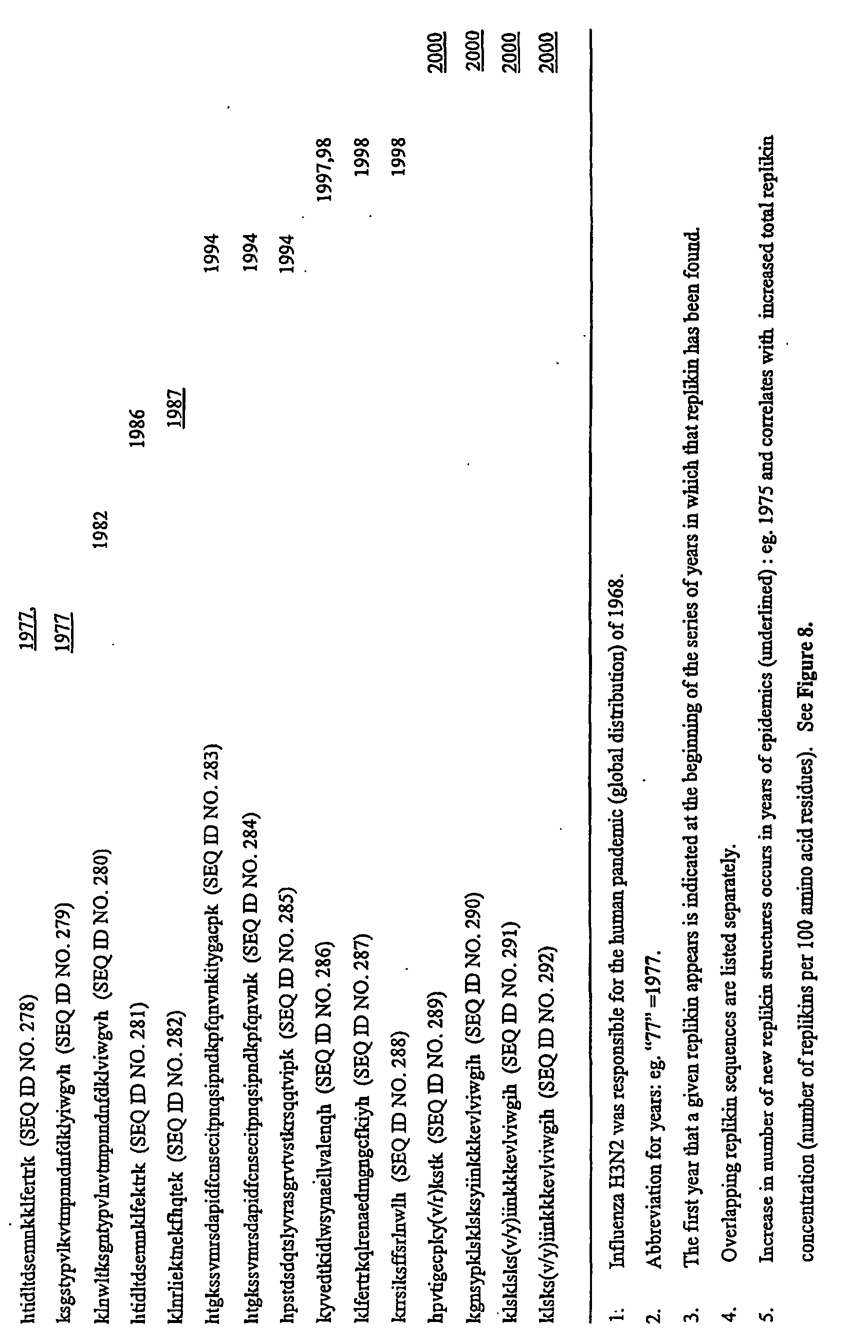

Both the concentration and type, i.e., composition of Replikins observed were found to relate to the occurrence of influenza pandemics and epidemics. The concentration of Replikins in influenza viruses was examined by visually scanning the hemagglutinin amino acid sequences published in the National Library of Medicine "PubMed" data base for influenza strains isolated world wide from human and animal reservoirs year by year over the past century, i.e., 1900 to 2001. These Replikin concentrations (number of Replikins per 100 amino acids, mean +/- SD) were then plotted for each strain.

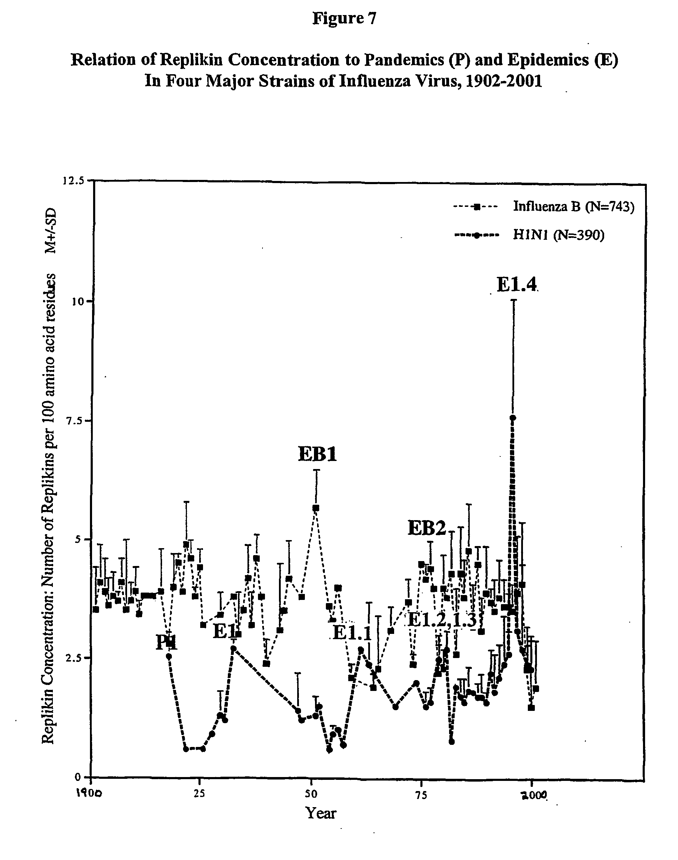

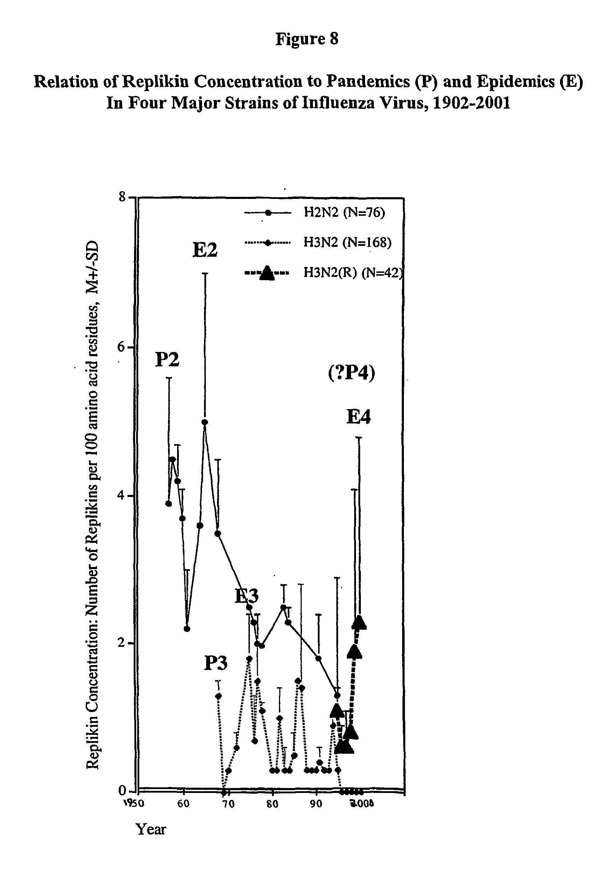

The concentration of Replikins was found to directly relate to the occurrence of influenza pandemics and epidemics. The concentration of Replikins found in influenza B hemagglutinin and influenza A strain, H1N1, is shown in **Figure 7****,** and the concentration of Replikins found in the two other common influenza virus A strains, H2N2 and H3N2 is shown in **Figure 8** (H2N2, H3N2). The data in **Figure 8** also demonstrate an emerging new strain of influenza virus as defined by its constituent Replikins (H3N2(R)).

Each influenza A strain has been responsible for one pandemic: in 1918, 1957, and 1968, respectively. The data in **Figures 7** and **8** show that at least one replikin per 100 amino acids is present in each of the influenza hemagglutinin proteins of all isolates of the four common influenza viruses examined, suggesting a function for Replikins in the maintenance of survival levels of replication. In the 1990s, during the decline of the H3N2 strain there were no Replikins present in many isolates of H3N2, but a high concentration of new replikins appeared in H3N2 isolates, which define the emergence of the H3N2(R) strain.

Several properties of Replikin concentration are seen in **Figure 7** and **Figure 8** to be common to all four influenza virus strains: (1) Concentration is cyclic over the years, with a single cycle of rise and fall occurring over a period of two to thirty years. This rise and fall is consistent with the known waxing and waning of individual influenza virus strain predominance by hemagglutinin and neuraminidase classification. (2) Peak Replikin concentrations of each influenza virus strain previously shown to be responsible for a pandemic were observed to relate specifically and individually to each of the three years of the pandemics. For example, for the pandemic of 1918, where the influenza virus strain, H1N1, was shown to be responsible, a peak concentration of the Replikins in H1N1 independently occurred (P1); for the pandemic of 1957, where H2N2 emerged and was shown to be responsible, a peak concentration of the Replikins in H2N2 occurred (P2); and for the pandemic of 1968, where H3N2 emerged and was shown to be the cause of the pandemic, a peak concentration of the Replikins in H3N2 occurred (P3). (3) In the years immediately following each of the above three pandemics, the specific Replikin concentration decreased markedly, perhaps reflecting the broadly distributed immunity generated in each case. Thus, this post-pandemic decline is specific for H1N1 immediately following the pandemic (P1) for which it was responsible, and is not a general property of all strains at the time. An increase of Replikin concentration in influenza B repeatedly occurred simultaneously with the decrease in Replikin concentration in H1N1, e.g., EB1 in 1951 and EB2 in 1976, both associated with influenza B epidemics having the highest mortality. (Stuart-Harris, et al., Edward Arnold Ltd. (1985). (4) A secondary peak concentration, which exceeded the primary peak increase in concentration, occurred 15 years after each of the three pandemics, and this secondary peak was accompanied by an epidemic: 15 years after the 1918 pandemic in an H1N1 'epidemic' year (E1); eight years after the 1957 pandemic in an H2N2 'epidemic' year (E2); and occurred seven years after the 1968 pandemic in an H3N2 'epidemic' year (E3). These secondary peak concentrations of specific Replikins may reflect recovery of the strain. (5) Peaks of each strain's specific Replikin concentration frequently appear to be associated with declines in Replikin concentration of one or both other strains, suggesting competition between strains for host sites. (6) There is an apparent overall tendency for the Replikin concentration of each strain to decline over a period of 35 years (H2N2) to 60 years (influenza B). This decline cannot be ascribed to the influence of vaccines because it was evident in the case of influenza B from 1901 to 1964, prior to common use of influenza vaccines. In the case of influenza B, Replikin recovery from the decline is seen to occur after 1965, but Replikin concentration declined again between 1997 and 2000 (Figure 7), and this correlates with the low occurrence of influenza B in recent case isolates. H1N1 Replikin concentration peaked in 1978-1979 (**Figure 7**) together with the reappearance and prevalence of the H1N1 strain, and then peaked in 1996 coincident with an H1N1 epidemic. (**Figure 7**). H1N1 Replikin concentration also declined between 1997 and 2000, and the presence of H1N1 strains decreased in isolates obtained during these years. For H2N2 Replikins, recovery from a 35 year decline has not occurred (**Figure 8**), and this correlates with the absence of H2N2 from recent isolates. For H3N2, the Replikin concentration of many isolates fell to zero during the period from 1996 to 2000, but other H3N2 isolates showed a significant, sharp increase in Replikin concentration. This indicates the emergence of a sub-strain of H3N2, which is designated herein as H3N2(R).

**Figures 7** and **8** demonstrate that frequently a one to three year stepwise increase is observed before Replikin concentration reaches a peak. This stepwise increase proceeds the occurrence of an epidemic, which occurs concurrently with the Replikin peak. Thus, the stepwise increase in concentration of a particular strain is a signal that that particular strain is the most likely candidate to cause an epidemic or pandemic.

Currently, Replikin concentration in the H3N2(R) strain of influenza virus is increasing (**Figure 8**, 1997 to 2000). Three similar previous peak increases in H3N2 Replikin concentration are seen to have occurred in the H3N2-based pandemic of 1968 (**Figure 8**), when the strain first emerged, and in the H3N2-based epidemics of 1972 and 1975 (**Figure 8**). Each of these pandemic and epidemics was associated with excess mortality. (Ailing, et al., Am J. Epidemiol., 113(1):30-43 (1981). The rapid ascent in concentration of the H3N2(R) subspecies of the H3N2 Replikins in 1997-2000, therefore, statistically represents an early warning of an approaching severe epidemic or pandemic. An H3N2 epidemic occurred in Russia in 2000 (**Figure 8**, E4); and the CDC report of December 2001 states that currently, H3N2 is the most frequently isolated strain of influenza virus world wide. (Morbidity and Mortality Weekly Reports (MMWR), Center for Disease Control; 50(48):1084-68 (Dec.7, 2001).

In each case of influenza virus pandemic or epidemic new Replikins emerge. There has been no observation of two of the same Replikins in a given hemagglutinin in a given isolate. To what degree the emergence of a new Replikin represents mutations versus transfer from another animal or avian pool is unknown. In some cases, each year one or more of the original Replikin structures is conserved, while at the same time, new Replikins emerge. For example, in influenza virus B hemagglutinin, five Replikins were constantly conserved between 1919 and 2001, whereas 26 Replikins came and went during the same period (some recurred after several years absence). The disappearance and re-emergence years later of a particular Replikin structure suggests that the Replikins return from another virus host pool rather than through *de novo* mutation.

In the case of H1N1 Replikins, the two Replikins present in the P1 peak associated with the 1918 pandemic were not present in the recovery E1 peak of 1933, which contains 12 new Replikins. Constantly conserved Replikins, therefore, are the best choice for vaccines, either alone or in combination. However, even recently appearing Replikins accompanying one year's increase in concentration frequently persist and increase further for an additional one or more years, culminating in a concentration peak and an epidemic, thus providing both an early warning and time to vaccinate with synthetic Replikins (see for example, H1N1 in the early 1990's, **Figure 7**).

The data in **FIGURES 7** and **8** demonstrate a direct relationship between the presence and concentration of a particular Replikin in influenza protein sequences and the occurrence of pandemics and epidemics of influenza. Thus, analysis of the influenza virus hemagglutinin protein sequence for the presence and concentration of Replikins provides a predictor of influenza pandemics and/or epidemics, as well as a target for influenza vaccine formulation.

Composition of Replikins in Strains of Influenza **Virus B**: Of a total of 26 Replikins identified in this strain (**Table 3**), the following ten Replikins are present in every influenza B isolate examined from 1902-2001. Overlapping Replikin sequences are listed separately. Lysines and histidines are in bold type to demonstrate homology consistent with the "3-point recognition."
kshfanlk (SEQ ID NO. 104)
kshfanlkgtk (SEQ ID NO. 105)
kshfanlkgtktrgklcpk (SEQ ID NO. 106)
hekygglnk (SEQ ID NO. 107)
hekygglnksk (SEQ ID NO. 108)
hekygglnkskpyytgehak (SEQ ID NO. 109)
hakaigncpiwvk (SEQ ID NO. 110)
hakaigncpiwvktplklangtk (SEQ ID NO. 111)
hakaigncpiwvktplklangtkyrppak (SEQ ID NO. 112)
hakaigncpiwvktplklangtkyrppakllk (SEQ ID NO. 113)

**Tables 3 and 4** indicate that there appears to be much greater stability of the Replikin structures in influenza B hemagglutinins compared with H1N1 Replikins. Influenza B has not been responsible for any pandemic, and it appears not to have an animal or avian reservoirs. (Stuart-Harris et al., Edward Arnold Ltd., London (1985)).

**Influenza H1N1 Replikins: Only one replikin** "hp(v/i)tigecpkyv(r/k)(s/t)(t/a)k" is present in every H1N1 isolate for which sequences are available from 1918, when the strain first appeared and caused the pandemic of that year, through 2000. (**Table 4**). ("(v/i)" indicates that the amino acid v or i is present in the same position in different years.) Although H1N1 contains only one persistent replikin, H1N1 appears to be more prolific than influenza B. There are 95 different replikin structures in 82 years on H1N1 versus only 31 different Replikins in 100 years of influenza B isolates (Table 4). An increase in the number of new Replikin structures occurs in years of epidemics (**Tables 3, 4, 5 and 6**) and correlates with increased total Replikin concentration (Figures 7 and 8).

**Influenza H2N2 Replikins:** Influenza H2N2 was responsible for the human pandemic of 1957. Three of the 20 Replikins identified in that strain for 1957 were conserved in each of the H2N2 isolates available for examination on PubMed until 1995 (**Table 5**).
ha(klq/m)(d/n)ilekthngk (SEQ ID NO. 232)
ha(k/q/m)(d/n)ilekthngklc(k/r) (SEQ ID NO. 233)
kgsnyp(v/i)ak(g/r)synntsgeqmliiwq(v/i)h (SEQ ID No. 238)

However, in contrast to H1N1, only 13 additional Replikins have been found in H2N2 beginning in 1961. This paucity of appearance of new Replikins correlates with the decline in the concentration of the H2N2 Replikins and the appearance of H2N2 in isolates over the years. (**Figure 8**).

**Influenza H3N2 Replikins:** Influenza H3N2 was responsible for the human pandemic of 1968. Five Replikins which appeared in 1968 disappeared after 1977, but reappeared in the 1990s (**Table 6**). The only Replikin structure which persisted for 22 years was hcd(g/q)f(q/r)nekwdlf(v/i)er(s/t)k, which appeared first in 1977 and persisted through 1998. The emergence of twelve new H3N2 replikins in the mid 1990s (Table 6) correlates with the increase in Replikin concentration at the same time (Figure 8), and with the prevalence of the H3N2 strain in recent isolates. together with the concurrent disappearance of all Replikins from some of these isolates (**Figure 8**), this suggests the emergence of the new substrain H3N2(R).

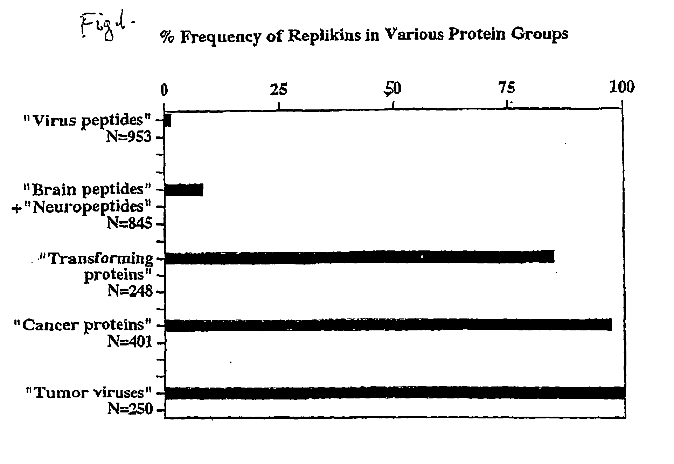

Figures 1 and 2 show that influenza epidemics and pandemics correlate with the increased concentration of replikins in influenza virus, which is due to the reappearance of at least one replikin from one to 59 years after its disappearance. Also, in the A strain only, there is an emergence of new strain-specific Replikin compositions (**Tables 4-6**). Increase in Replikin concentration by repetition of individual replikins within a single protein appears not to occur in influenza virus, but is seen in other organisms.

It has been believed that changes in the activity of different influenza strains are related to sequence changes in influenza hemagglutinins, which in turn are the products of substitutions effected by one of two poorly understood processes: i) antigenic drift, thought to be due to the accumulation of a series of point mutations in the hemagglutinin molecule, or ii) antigenic shift, in which the changes are so great that genetic reassortment is postulated to occur between the viruses of human and non-human hosts. First, the present data suggests that the change in activity of different influenza strains, rather than being related to non-specific sequence changes, are based upon, or relate to the increased concentration of strain-specific replikins and strain-specific increases in the replication associated with epidemics. In addition, the data were examined for a possible insight into which sequence changes are due to "drift" or "shift", and which due to conservation, storage in reservoirs, then reappearance. The data show that the epidemic-related increase in replikin concentration is not due to the duplication of existing replikins per hemagglutinin, but is due to the reappearance of at least one replikin composition from 1 to up to 59 years after its disappearance, plus in the A strains only, the emergence of new strain-specific replikin compositions (Tables 3-6). Thus the increase in replikin concentration in the influenza B epidemics of 1951 and 1977 are not associated with the emergence of new replikin compositions in the year of the epidemic but only with the reappearance of replikin compositions which had appeared in previous years then disappeared (Table 3). In contrast, for the A strains, in addition to the reappearance of previously disappeared virus replikins, new compositions appear (e.g. in H1N1 in the year of the epidemic of 1996, in addition to the reappearance of 6 earlier replikins, 10 new compositions emerged). Since the A strains only, not influenza B, have access to non-human animal and avian reservoirs, totally new compositions probably derive from non-human host reservoirs rather than from mutations of existing human replikins which appear to bear no resemblance to the new compositions other than the basic requirements of "3-point recognition" (Tables 2-5). The more prolific nature of HIN1 compared with B, and the fact that pandemics have been produced by the three A strains only, but not by the B strain, both may also be a function of the ability of the human A strains to receive new replikin compositions from non-human viral reservoirs.

Some replikins have appeared in only one year, disappeared, and not reappeared to date (Tables 3-6). Other replikins disappear for from one to up to 81 years, when the identical replikin sequence reappears. Key replikin 'k' and 'h' amino acids, and the spaces between them, are conserved during the constant presence of particular replikins over many years, as shown in Tables 23-6 for the following strain-specific replikins: ten of influenza B, the single replikin of HIN1, and the single replikin of H2N3, as well as for the reappearance of identical replikins after an absence. Despite the marked replacement or substitution activity of other amino acids both inside the replikin structure and outside it in the rest of the hemagglutinin sequences, influenza replikin histidine (h) appears never to be, and lysine (k) is rarely replaced. Examples of this conservation are seen in the H1N1 replikin"hp(v/i)tigecpkyv(r/k)(s/t)(t/a)k," (SEQ ID NO. 135) constant between 1918 and 2000, in the H3N2 replikin "hcd(g/q)f(q,r)nekwdlf(v/i)er(s/t)k" (SEQ ID NO. 277) constant between 1975 and 1998 and in the H3N2 replikin "hqn(s/e)(e/q)g(t/s)g(q/y)aad(l/q)kstq(a/n)a(i/1)d(q/g)I(n/t)(g/n)k,(l/v)n(r/s)vi(e/c)k" (SEQ ID NO. 276) which first appeared in 1975, disappeared for 25 years, and then reappeared in 2000. While many amino acids were substituted, the basic replikin structure of 2 Lysines, 6 to 10 residues apart, one histidine, a minimum of 6% lysine in not more than approximately 50 amino acids, was conserved.

Totally random substitution would not permit the persistence of these H1N1 and H3N2 replikins, nor from 1902 to 2001 in influenza B the persistence of 10 replikin structures, nor the reappearance in 1993 of a 1919 18mer replikin after an absence of 74 years. Rather than a random type of substitution, the constancy suggests an orderly controlled process, or in the least, protection of the key replikin residues so that they are fixed or bound in some way: lysines, perhaps bound to nucleic acids, and histidines, perhaps bound to respiratory redox enzymes. The mechanisms which control this conservation are at present unknown.

Whether the conservation of replikin structures is unique to influenza or occurs in other virus replikins was examined in foot and mouth disease virus (FMDV) isolates, where extensive mutations in proteins of this virus have been well-documented worldwide over decades. In the protein VP1 of FMDV type 0, the replikin "hkqkivapvk" (SEQ ID NO. 3) was found to be conserved in 78% of the 236 isolates reported in PubMed, and each amino acid was found to be conserved in individual isolates as follows: h,95.6%; k,91.8%; q,92.3%; k,84.1%; i,90.7%; v,91.8%; a..97.3%; p,96.2%; a,75.4%; k,88.4%. Similarly, conservation was observed in different isolates of HIV for its replikins such as "kcfncgkegh" (SEQ ID NO. 5) or "kvylawvpahk" (SEQ ID NO. 6) in HIV Type 1 and "kcwncglcegh" (SEQ ID NO. 7) in HIV Type 2¹⁶. The high rate of conservation observed in FMVD and HIV replikins suggests that conservation observed in influenza replikins is a general property of viral replikins.

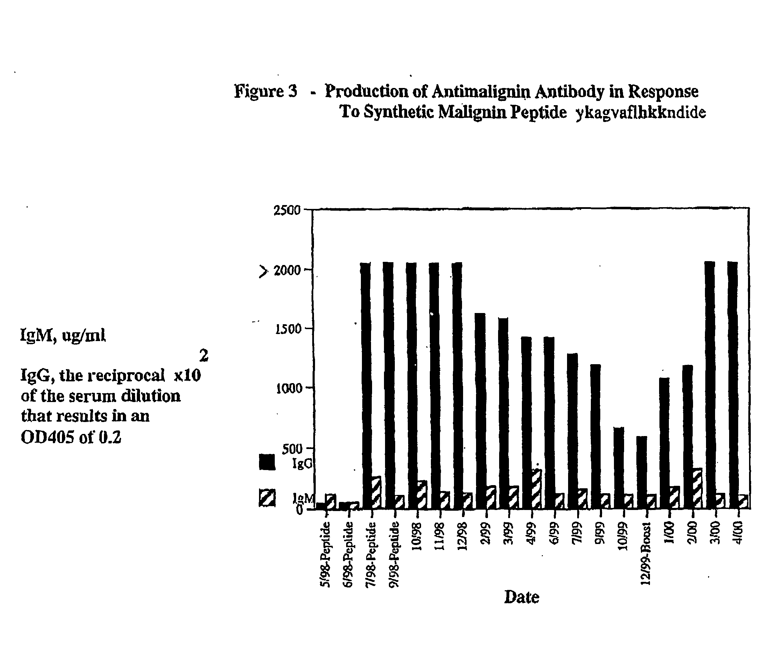

Data on anti-Replikin antibodies also support Replikin class unity. An anti-Replikin antibody response has been quantified by immunoadsorption of serum antimalignin antibody to immobilized malignin (see Methods in U.S. Patent #5,866,690). The abundant production of antimalignin antibody by administration to rabbits of the synthetic version of the 16-mer peptide whose sequence was derived from malignin, absent carbohydrate or other groups, has established rigorously that this peptide alone is an epitope, that is, it is a sufficient basis for this immune response (**Figure 3**). The 16-mer peptide produced both IgM and IgG forms of the antibody. Antimalignin antibody was found to be increased in concentration in serum in 37% of 79 cases in the U.S. and Asia of hepatitis B and C, early, in the first five years of infection, long before the usual observance of liver cancer, which develops about fifteen to twenty-five years after infection. Relevant to both infectious hepatitis and HIV infections, transformed cells may be one form of safe haven for the virus: prolonging cell life and avoiding virus eviction, so that the virus remains inaccessible to anti-viral treatment.

Because administration of Replikins stimulates the immune system to produce antibodies having a cytotoxic effect, peptide vaccines based on the particular influenza virus Replikin or group of Replikins observed to be most concentrated over a given time period provide protection against the particular strain of influenza most likely to cause an outbreak in a given influenza season., e.g., an emerging strain or re-emerging strain For example, analysis of the influenza virus hemagglutinin amino acid sequence on a yearly or bi-yearly basis, provides data which are useful in formulating a specifically targeted influenza vaccine for that year. It is understood that such analysis may be conducted on a region-by-region basis or at any desired time period, so that strains emerging in different areas throughout the world can be detected and specifically targeted vaccines for each region can be formulated.

Currently, vaccine formulations are changed twice yearly at international WHO and CDC meetings. Vaccine formulations are based on serological evidence of the most current preponderance of influenza virus strain in a given region of the world. However, prior to the present invention there has been no correlation of influenza virus strain specific amino acid sequence changes with occurrence of influenza epidemics or pandemics.

The observations of specific Replikins and their concentration in influenza virus proteins provides the first specific quantitative early chemical correlates of influenza pandemics and epidemics and provides for production and timely administration of influenza vaccines tailored specifically to treat the prevalent emerging or re-emerging strain of influenza virus in a particular region of the world. By analyzing the protein sequences of isolates of strains of H₁N₁ influenza virus, such as the hemagglutinin protein sequence, for the presence, concentration and/or conservation of Replikins, influenza virus pandemics and epidemics can be predicted. Furthermore, the severity of such outbreaks of influenza can be significantly lessened by administering an H₁N₁ influenza peptide vaccine based on the Replikin sequences found to be most abundant or shown to be on the rise in virus isolates over a given time period, such as about one to about three years.

An influenza peptide vaccine of the invention may include a single Replikin peptide sequence or may include a plurality of Replikin sequences observed in H₁N₁ influenza virus strains. Preferably, the peptide vaccine is base on H₁N₁ influenza Replikin sequence(s) shown to be increasing in concentration over a given time period and conserved for at least that period of time. However, a vaccine may include a conserved Replikin peptide(s) in combination with a new Replikin(s) peptide or may be based on new Replikin peptide sequences. The Replikin peptides can be synthesized by any method, including chemical synthesis or recombinant gene technology, and may include non-Replikin sequences, although vaccines based on peptides containing only Replikin sequences are preferred. Preferably, vaccine compositions of the invention also contain a pharmaceutically acceptable carrier and/or adjuvant.

The influenza vaccines of the present invention can be administered alone or in combination with antiviral drugs, such as gancyclovir; interferon; interleukin; M2 inhibitors, such as, amantadine, rimantadine; neuraminidase inhibitors, such as zanamivir and oseltamivir; and the like, as well as with combinations of antiviral drugs.

Analysis of the primary structure of a *Plasmodium farciparum* malaria antigen located at the merozoite surface and/or within the parasitophorous vacuole revealed that this organism, like influenza virus, also contains numerous Replikins. However, there are several differences between the observation of Replikins in *Plasmodium falciparum* and influenza virus isolates. For example, *Plasmodium falciparum* contains several partial Replikins, referred to herein as "Replikin decoys." These decoy structures contain an abundance of lysine residues, but lack the histidine required of Replikin structures. It is believed that the decoy structure maximizes the chances that an anti-malarial antibody or other agent will bind to the relatively less important structure containing the lysines, i.e., the Replikin decoys, rather than binding to histidine, which is present in Replikin structure, such as replikins in respiratory enzymes, which could result in destruction of the trypanosome.

Another difference seen in *Plasmodium falciparum* is a frequent repetition of individual Replikin structures within a single protein, which was not observed with influenza virus. Repetition may occur by (a) sharing of lysine residues between Replikins, and (b) by repetition of a portion of a Replikin sequence within another Replikin sequence.

A third significant difference between Replikin structures observed in influenza virus isolates and *Plasmodium falciparum* is a marked overlapping of Replikin structures throughout malarial proteins, e.g., there are nine overlapping replikins in the 39 amino acid sequence of SEQ ID NO. 393 (Replikin concentration = 23.1/100 amino acids); and 15 overlapping replikins in the 41 amino acids of SEQ ID NO. 467 (Replikin concentration = 36.6/100 amino acids). Both of these overlapping Replikin structures occur in blood stage trophozoites and schizonts. In contrast, influenza virus Replikins are more scattered throughout the protein and the maximum Replikin concentration is about 7.5/100 amino acids (**Figure 7**); and tomato leaf curl gemini virus, which was also observed to have overlapping replikins has only about 3.1/100 amino acids.

This mechanism of lysine multiples is also seen in the Replikins of cancer proteins such as in gastric cancer transforming protein, ktkkgnrvsptmkvth (SEQ ID NO. 88), and in transforming protein P21B (K-RAS 2B) of lung,
khkekmskdgkkkkkks (SEQ ID NO. 89).

The relationship of higher Replikin concentration to rapid replication is also confirmed by analysis of HIV isolates. It was found that the slow-growing low titer strain of HIV (NSI, "Bru", which is prevalent in early stage HIV infection has a Replikin concentration of 1.1 (+/-1.6) Replikins per 100 amino acids, whereas the rapidly-growing high titer strain of HIV (S1, "Lai"), which is prevalent in late stage HIV infection has a Replikin concentration of 6.8 (+/- 2.7) Replikins per 100 amino acid residues.

The high concentration of overlapping Replikins in malaria, influenza virus and cancer cells is consistent with the legendary high and rapid replicating ability of malaria organisms. The multitude of overlapping Replikins in malaria also provides an opportunity for the organism to flood and confuse the immune system of its host and thereby maximize the chance that the wrong antibody will be made and perpetuated, leaving key malaria antigens unharmed.

Table 7 provides a list of several *Plasmodium falciparum* Replikin sequences.

**Table 7**

| **Malaria replikins** |
|---|
| **a) Primary structure of a Plasmodium falciparum malaria antigen located at the merozoite surface and within the parasitophorous vacuole** |
| **a) i) DECOYS:** |
| (C-Terminal) |
| keeeekekekekekeekekeekekeekekekeekekekeekeeekk (SEQ ID NO. 293), or |
| keeeekekekekekeekekeekekeekekekeekekekeekeeekkek (SEQ ID NO. 294), or |
| keeeekekekekekeekekeekekekeekekeekekeekeekeeekk (SEQ ID NO. 295), or |
| keeeekekek (SEQ ID NO. 296) |

| **ii) REPLIKINS:** |
|---|
| Hkklikalkkniesiqnkk (SEQ ID NO. 297) |
| hkklikalkkniesiqnkm (SEQ ID NO. 298) |
| hkklikalkk (SEQ ID NO. 299) |
| hkklikalk (SEQ ID NO. 300) |
| katysfvntkkkiislksqghkk (SEQ ID NO. 301) |
| katysfvntkkkiislksqghk (SEQ ID NO. 302) |
| katysfvntkkkiislksqgh (SEQ ID NO. 303) |
| htyvkgkkapsdpqca dikeeckellkek (SEQ ID NO. 304) |
| kiislksqghk (SEQ ID NO. 305) |
| kkkkfeplkngnvsetiklih (SEQ ID NO. 306) |
| kkkfeplkngnvsetiklih (SEQ ID NO. 307) |
| kkfeplkngnvsetiklih (SEQ ID NO. 308) |
| kngnvsetiklih (SEQ ID NO. 309) |
| klihlgnkdkk (SEQ ID NO. 310) |
| kvkkigvtlkkfeplkngnvsetiklihlgnkdkkh (SEQ ID NO. 311) |
| hliyknksynplllscvkkmnmlkenvdyiqnqrufkelmnqkatysfvntkkkiislk (SEQ ID NO. 312) |
| hliyknksynplllscvkkmnmlkenvdyiqnqnlfkelmnqkatysfvntk (SEQ ID NO. 313) |
| hliyknksynplllscvkmnmlkenvdyiqnqnlfkelmnqk (SEQ ID NO. 314) |
| hliyknksynplllscvkkmnmlkenvdyiqknqnlfk (SEQ ID NO. 315) |
| hliyknksynplllscvkkmnmlk (SEQ ID NO. 316) |
| ksannsanngklamaeemlmlvnflqshkklikalkkniesiqnkkh (SEQ ID NO. 317) |
| kknnaeemknlvnflqshkklikalkkniesiqnkkh (SEQ ID NO. 318) |
| knlvnflqshkklikalkkniesiqnkkh (SEQ ID NO. 319) |
| kklikalklaniesiqnkkh (SEQ ID NO. 320) |
| klikalkkniesiqnkkh (SEQ ID NO. 321) |
| kkniesiqnkkh (SEQ ID NO. 322) |
| kniesiqnkkh (SEQ ID NO. 323) |
| knnaeemknlvnflqsh (SEQ ID NO. 324) |
| kklikalkkniesiqnkkqghkk (SEQ ID NO. 325) |
| kknnaeemknlvnflqshk (SEQ ID NO. 326) |
| knnaeemknlvnflqsh (SEQ ID NO. 327) |
| klikalkkniesiqnkkqghkk (SEQ ID NO. 328) |
| kvkkigvtlkkfeplkngnvsetiklih (SEQ ID NO. 329) |
| kngnvsetiklih (SEQ ID NO. 330) |
| klihlgnkdkk (SEQ ID NO. 331) |
| ksannsanngkknnaeemknlvnflqsh (SEQ ID NO. 332) |
| kknnaeernknlvnflqsh (SEQ ID NO. 333) |
| kklikalkkniesiqnkkh (SEQ ID NO. 334) |
| kalkkniesiqnkkh (SEQ ID NO. 335) |
| kkniesiqnkkh (SEQ ID NO. 336) |
| kelmnqkatysfvntkkkiislksqgh (SEQ ID NO. 337) |
| ksqghkk (SEQ ID NO. 338) |
| kkkiislksqgh (SEQ ID NO. 339) |
| kkiislksqgh (SEQ ID NO. 340) |
| kkniesiqnkkh (SEQ ID NO. 341) |
| kniesiqnkkh (SEQ ID NO. 342) |
| htyvkgkkapsdpqcadikeeckellkek (SEQ ID NO. 343) |
| htyvkgkkapsdpqcadikeeckellk (SEQ ID NO. 344 ) |

| **b)"liver stage antigen-3" gene="LSA-3" Replikins** |
|---|
| henvlsaalentqseeekkevidvieevk (SEQ ID NO. 345) |
| kenvvttilekveettaesvttfsnileeiqentitndtieekleelh (SEQ ID NO. 346) |
| hylqqmkekfskek (SEQ ID NO. 347) |
| hylqqmkekfskeknnnvievtnkaekkgnvqvtnktekttk (SEQ ID NO. 348) |
| hylqqmkekfskeknnnvievtnkaekkgnvqvtnkttkvdknnk (SEQ ID NO. 349) |
| hylqqmkekfskeknnnvievtnkaekkgnvqvtnktekttkvdknnkvpkkrrtqk (SEQ ID NO. 350) |
| hylqqmkekfskeknnnvievtnkaekkgnvqvtnktekttkvdnnkvpkkrrtqksk (SEQ ID NO. 351) |
| hvdevmkyvqkidkevdkevskaleskndvtnvllcqnqdffskvknfvkkyk (SEQ ID NO. 352) |
| hvdevmkyvqkidkevdkevskaleskndvtnvlkqnqdffskvknfvkk (SEQ ID NO. 353) |
| hvdevmkyvqkidkevdkevskaleskndvtnvlkqnqdffsk (SEQ ID NO. 354) |
| hvdevmkyvqkidkevdkevskaleskndvtnvlk (SEQ ID NO. 355) |
| hvdevmkyvqkidkevdkevskalesk (SEQ ID NO. 356) |
| hvdevmkyvqkidkevdkevsk (SEQ ID NO. 357) |
| hvdevmkyvqkidkevdk (SEQ ID NO. 358) |
| hvdevmkyvqkidk (SEQ ID NO. 359) |
| kdevidlivqkekriekvkakkkklekkveegvsglkkh (SEQ ID NO. 360) |
| kvkakkkklekkveegvsglkkh (SEQ ID NO. 361) |
| kakkkklekkveegvsglkkh (SEQ ID NO. 362) |
| kkkklekkveegvsglkkh (SEQ ID NO. 363) |
| kkklekkveegvsglkkh (SEQ ID NO. 364) |
| kklekkveegvsglkkh (SEQ ID NO. 365) |
| klekkveegvsglkkh (SEQ ID NO. 366) |
| kkveegvsglkkh (SEQ ID NO. 367) |
| kveegvsglkkh (SEQ ID NO.368) |
| hveqnvyvdvdvpamkdqflgilneagglkemffnledvfksesdvitveeikdepvqk (SEQ ID NO. 369) |
| hikgleeddleevddlkgsildmlkgdmelgdmdkesledvttklgerveslk (SEQ ID NO. 370) |
| hikgleeddleevddlkgsildmlkgdmelgdmdkesledvttk (SEQ ID NO. 371) |
| hikgleeddleevddlkgsildmlkgdmelgdmdk (SEQ ID NO. 372) |
| hikgleeddleevddlkgsildmlk (SEQ ID NO. 373) |
| hiisgdadvlssalgmdeeqmktrkkaqrpk (SEQ ID NO. 374) |
| hditttldevvelkdveedkiek (SEQ ID NO. 375) |
| kkleevhelk (SEQ ID NO. 376) |
| kleevhelk (SEQ ID NO. 377) |
| ktietdileekkkeiekdh (SEQ ID NO. 378) |
| kkeiekdhfek (SEQ ID NO. 379) |
| kdhfek (SEQ ID NO. 380) |
| kfeeeaeeikh (SEQ ID NO. 381) |
| |

| **c) 28 KDA ookinete surface antigen precursor Replikins:** |
|---|
| kdgdtkctlecaqgkkcikhksdhnhksdhnhksdpnhkkknnnnnk (SEQ ID NO. 382) |
| kdgdtkctlecaqgkkcikhksdhnhksdhnhksdpnhkk (SEQ.ID NO. 383) |
| kdgdtkctlecaqgkkcikhksdhnhksdhnhksdpnhk (SEQ ID NO. 384) |
| kdgdtkctlecaqgkkcikhksdhnhksdhnhk (SEQ ID NO. 385) |
| kdgdtkctlecaqgkkcikhksdhnhk (SEQ ID NO. 386) |
| kdgdtkctlecaqgkkcikhk (SEQ ID NO. 387) |
| kdgdtkctlecaqgkk (SEQ ID NO. 388) |
| kdgdtkctlecaqgk (SEQ ID NO. 389) |
| kciqaecnykecgeqkcvwdgih (SEQ ID NO. 390) |
| kecgeqkcvwdgih (SEQ ID NO. 391) |
| hieckcnndyvltnryecepknkctsledtnk (SEQ ID NO. 392) |
| |

| **d) Blood stage trophozoites and schizonts Replikins:** |
|---|
| ksdhnhksdhnhksdhnhksdhnhksdpnhkknnnnnk (SEQ ID NO. 393) |
| ksdhnhksdhnhksdhnhksdpnhkkknnnnnk (SEQ ID NO. 394) |
| ksdhnhksdhnhksdpnhkkknnnnnk (SEQ ID NO. 395) |
| ksdhnhksdpnhkkknnnnnk (SEQ ID NO. 396) |
| kkknnnnnkdnksdpnhk (SEQ ID NO. 397) |
| kknnnnnkdnksdpnhk (SEQ ID NO. 398) |
| knnnnnkdnksdpnhk (SEQ ID NO. 399) |
| kdnksdpnhk (SEQ ID NO. 400) |
| ksdpnhk (SEQ ID NO. 401) |
| hslyalqqneeyqkvknekdqneikkikqlieknk (SEQ ID NO. 402) |
| hslyalqqneeyqkvknekdqneikkik (SEQ ID NO. 403) |
| hslyalqqneeyqkvknekdqneikk (SEQ ID NO. 404) |
| hslyalqqneeyqkvknekdqneik (SEQ ID NO. 405) |
| hklenleemdk (SEQ ID NO. 406) |
| khfddntneqk (SEQ ID NO. 407) |
| kkeddekh (SEQ ID NO. 408) |
| keennkkeddekh (SEQ ID NO. 409) |
| ktssgilnkeennkkeddekh (SEQ ID NO. 410) |
| knihikk (SEQ ID NO. 411) |
| hikkkegidigyk (SEQ ID NO. 412) |
| kkmwtcklwdnkgneitknih (SEQ ID NO. 413) |
| kkgiqwnllkkmwtcklwdnkgneitknih (SEQ ID NO. 414) |
| kekkdsnenrkkkqkedkknpnklkkieytnkithffkaknnkqqnnvth (SEQ ID NO. 415) |
| kkdsnenrkkkqkedkknpnklkkieytnkithffkaknnkqqnnvth (SEQ ID NO. 416) |
| kdsnenrkkkqkedkknpnklkkieytnkithffkaknnkqqnnvth (SEQ ID NO. 417) |
| kkqkedkknpnklkkieytnkithffkaknnkqqnnvth (SEQ ID NO. 418) |
| kqkedkknpnklkkieytnkithffkaknnkqqnnvth (SEQ ID NO. 419) |
| kedkknpnklkkieytnkithffkaknnkqqnnvth (SEQ ID NO. 420) |
| knpnklkkieytnkithffaknnkqqnnvth (SEQ ID NO. 421) |
| kkieytnkithffkaknnkqqnnvth (SEQ ID NO. 422) |
| kieytnkithffkaknnkqqnnvth (SEQ ID NO. 423) |
| kithffkaknnkqqnnvth (SEQ ID NO. 424) |
| hknnedikndnskdikndnskdikndnskdikndnnedikndnskdik (SEQ ID NO. 425) |
| hknnedikndnskdikndnskdikndnskdikndnnedikndnsk (SEQ ID NO. 426) |
| hknnedikndnskdikndnskdikndnskdikndnnedik (SEQ ID NO. 427) |
| hknnedikndnskdikndnskdikndnskdik (SEQ ID NO. 428) |
| hknnedikndnskdikndnskdikndnsk (SEQ ID NO. 429) |
| hknnedikndnskdikndnskdik (SEQ ID NO. 430) |
| hknnedikndnskdikndnsk (SEQ ID NO. 431) |
| hknnedikndnskdik (SEQ ID NO. 432) |
| hknnedik (SEQ ID NO. 433) |
| kkyddlqnkynilnklknsleekneelkkyh (SEQ ID NO. 434) |
| kyddlqnkymlnklknsleekneelkkyh (SEQ ID NO. 435) |
| kynilnklknsleekneelkkyh (SEQ ID NO. 436) |
| klknsleekneelkkyh (SEQ ID NO. 437) |
| knsleekneelkkyh (SEQ ID NO. 438) |
| kneelkkyh (SEQ ID NO. 439) |
| hmgnnqdinenvynikpqefkeeeeedismvntkk (SEQ ID NO. 440) |
| knsnelkrindnffklh (SEQ ID NO. 441) |
| kpclykkckisqclykkckisqvwwcmpvkdtfntyernnvlnskienniekiph (SEQ ID NO. 442) |
| hinneytnknpkncllykneernyndnnikdyinsmnfkk (SEQ ID NO. 443) |
| hinneytnknplmcllykneenyndnnikdyinsmnfk (SEQ ID NO. 444) |
| hinneytnknpkncllyk (SEQ ID NO. 445) |
| knktnqskgvkgeyekkketngh (SEQ ID NO. 446) |
| ktnqskgvkgeyekkketngh (SEQ ID NO. 447) |
| kgvkgeyekkketngh (SEQ ID NO. 448) |
| kgeyekkketngh (SEQ ID NO. 449) |
| ksgmytnegnkscecsykkkssssnkvh (SEQ ID NO. 450) |
| kscecsykkkssssnkvh (SEQ ID NO. 451) |
| kkkssssnkvh (SEQ ID NO. 452) |
| kkssssnkvh (SEQ ID NO. 453) |
| kssssnkvh (SEQ ID NO. 454) |
| himlksgmytnegnkscecsykkkssssnk (SEQ ID NO. 455) |
| himlksgmytnegnkscecsykkk (SEQ ID NO. 456) |
| himlksgmytnegnkscecsykk (SEQ ID NO. 457) |
| himlksgmytnegnkscecsyk (SEQ ID NO. 458) |
| kplaklrkrektqinktkyergdviidnteiqkiiirdyhetlnvhkldh (SEQ ID NO. 459) |
| krektqinktkyergdviidnteiqkiiirdyhetlnvhkldh (SEQ ID NO. 460) |
| ktqinktkyergdviidnteiqkiiirdyhetlnvhkldh (SEQ ID NO. 461) |
| kplaklrkrektqinktkyergdviidnteiqkiiirdyhetlnvh (SEQ ID NO. 462) |
| kplaklrkrektqinktkyergdviidnteiqkiiirdyh (SEQ ID NO. 463) |
| klrkrektqinktkyergdviidnteiqkiiirdyh (SEQ ID NO. 464) |
| krektqinktkyergdviidnteiqkiiirdyh (SEQ ID NO. 465) |
| ktqinktkyergdviidnteiqkiiirdyh (SEQ ID NO. 466) |
| kkdkekkkdsnenrlkkkqkedkknpndnklkkieytnkith (SEQ ID NO. 467) |
| kdkekkkdsnenrkkkqkedkknpndnklkkieytnkith (SEQ ID NO. 468) |
| kekkkdsnenrkkkqkedkknpndnklkkieytnkith (SEQ ID NO. 469) |
| kkkdsnenrkkkqkedkknpndnklkkieytnkith (SEQ ID NO. 470) |
| kkdsnenrkkkqkedkknpndnklkkieytnkith (SEQ ID NO. 471) |
| kdsnenrkkkqkedkknpndnklkkieytnkith (SEQ ID NO. 472) |
| kkkqkedkknpndnklkkieytnkith (SEQ ID NO. 473) |
| kkqkedkknpndnklkkieytnkith (SEQ ID NO. 474) |
| kqkedkknpndnklkkieytnkith (SEQ ID NO. 475) |
| kedkknpndnklkkieytnkith (SEQ ID NO. 476) |
| kknpndnklkkieytnkith (SEQ ID NO. 477) |
| knpndnklkkieytnkith (SEQ ID NO. 478) |
| klkkieytnkith (SEQ ID NO. 479) |
| kkieytnkith (SEQ ID NO. 480) |
| kieytnkith (SEQ ID NO. 481) |
| hgqikiedvnnenfnneqmknkyndeekmdiskskslksdflek (SEQ ID NO. 482) |
| hgqikiedvnnenfnneqmknkyndeekmdiskskslk (SEQ ID NO. 483) |
| hgqikiedvnnenfnneqmknkyndeekmdisksk (SEQ ID NO. 484) |
| hgqikiedvnnenfnneqmknkyndeekmdisk (SEQ ID NO. 485) |
| kkyddlqnkynilnklknsleekneelkkyh (SEQ ID NO. 486) |
| kyddlqnkynilnkhmsleekneelkkyh (SEQ ID NO. 487) |
| kynilnklknsleekneelkkyh (SEQ ID NO. 488) |
| klknsleekneelkkyh (SEQ ID NO. 489) |
| knsleekneelkkyh (SEQ ID NO. 490) |
| kneelkkyh (SEQ ID NO. 491) |
| hmgnnqdinenvynikpqefkeeeeedismvntkkcddiqenik (SEQ ID NO. 492) |
| ktnlyniynnknddkdnildnenreglylcdvmknsnelkrindnffklh (SEQ ID NO. 493) |
| knsnelkrindnffklh (SEQ ID NO. 494) |
| krindnffklh (SEQ ID NO. 495) |
| hinneytnknpkncllykneernyndnnikdyinsmnfkk (SEQ ID NO. 496) |
| hinneytnknpkncllykneernyndnnikdyinsmnfk (SEQ ID NO. 497) |
| hinneytnknpkncllyk (SEQ ID NO. 498) |
| kpclykkckisqvwwcmpvkdtfntyernnvlnskienniekiph (SEQ ID NO. 499) |
| kckisqvwwcmpvkdtfntyernnvlnskienniekiph (SEQ ID NO. 500) |
| kienniekiph (SEQ ID NO. 501) |
| knktngskgvkgeyekkketngh (SEQ ID NO. 502) |
| ktngskgvkgeyekkketngh (SEQ ID NO. 503) |
| kgvkgeyekkketngh (SEQ ID NO. 504) |
| kgeyekkketngh (SEQ ID NO. 505) |
| ktiekinkskswffeeldeidkplaklrkrektqinktkyergdviidnteiqkiirdyh (SEQ ID NO. 506) |
| kinkskswffeeldeidkplaklrkrektqinktkyergdviidnteiqkiirdyh (SEQ ID NO. 507) |
| kplaklrkrektqinktkyergdviidnteiqkiirdyh (SEQ ID NO. 508) |
| himlksqmytnegnkscecsykkkssssnkvh (SEQ ID NO. 509) |
| klrkrektqinktkyergdviidnteiqkiirdyh (SEQ ID NO. 510) |
| krektqihktkyergdviidnteiqkiirdyh (SEQ ID NO. 511) |
| ktqinktkyergdviidnteiqkiirdyh (SEQ ID NO. 512) |
| kplaklrkrektqinktkyergdviidnteiqviirdnteiqkiidyhtlnvhkldh (SEQ ID NO. 513) |
| klrkrektqinktkyergdviidnteiqkiirdyhtlnvhkldh (SEQ ID NO. 514) |
| krektqinktkyergdviidnteiqkiirdyhtlnvhkldh(SEQ ID NO. 515) |
| ktqinktkyergdviidnteiqkiirdyhtlnvhldh (SEQ ID NO. 516) |
| kplaklrkrektqinktkyergdviidnteiqkiirdyhtlnvh (SEQ ID NO. 517) |
| klrkrektqinktkyergdviidnteiqkiirdyhtlnvh (SEQ ID NO. 518) |
| krektqinktkyergdviidnteiqkiirdyhtlnvh (SEQ ID NO. 519) |
| ktqinktkyergdviidnteiqkiirdyhtlnvh (SEQ ID NO. 520) |
| himlksqmytnegnkscecsykkkssssnkvh (SEQ ID NO. 521) |
| ksqmytnegnkscecsykkkssssnkvh (SEQ ID NO. 522) |
| kscecsykkkssssnkvh (SEQ ID NO. 523) |
| kkkssssnkvh (SEQ ID NO. 524) |
| kkssssnkvh (SEQ ID NO. 525) |
| kssssnkvh (SEQ ID NO. 526) |
| himlksqmytnegnkscecsykkkssssnk (SEQ ID NO. 527) |
| himlksqmytnegnkscecsykkk (SEQ ID NO. 528) |
| himlksqmytnegnkscecsykk (SEQ ID NO. 529) |
| himlksqmytnegnkscecsyk (SEQ ID NO. 530) |
| hnnhniqiykdkrinfmnphkvmyhdnmsknertek (SEQ ID NO. 531) |
| hnnhniqiykdkrinfmnphkvmyhdnmsk (SEQ ID NO. 532) |
| hnnhniqiykdkrinfmnphk (SEQ ID NO. 533) |
| hkvmyhdnmsknertek (SEQ ID NO. 534) |
| hkvmyhdnmsk (SEQ ID NO. 535) |

Other microorganisms found to contain numerous Replikin structures are *Bacillus anthracis,* the organism responsible for anthrax infections, in which eight different replikins were identified; and small pox virus, in which five different Replikins were identified. The eight *Bacillus anthracis* peptides are present in the Anthrax Toxin Lethal Factor Protein pX01-107 and have the amino acid sequence of SEQ ID NO. 91, SEQ ID NO.92, SEQ ID NO. 93, SEQ ID NO. 94, SEQ ID NO. 95, SEQ ID NO. 96, SEQ ID NO. 97 and SEQ ID NO. 98, respectively. The five small pox virus peptides are present in the Small Pox Virus Surface Antigen S Precursor Protein, which purportedly enhances Small Pox Virus replication. The five peptides have the amino acid sequence of SEQ ID NO. 99, SEQ ID NO. 100, SEQ ID NO.101, SEQ ID NO. 102 and SEQ ID NO. 103, respectively.

Synthetic H₁N₁ inflluenza Replikin vaccines, or preferably, an influenza vaccine based on conserved and/or emerging or re-emerging H₁N₁ influenza Replikin(s) over a given time period may be used to augment antibody concentration in order to lyse the virus infected cells and release virus extracellularly where chemical treatment can then be effective.

H₁N₁ influenza Replikin peptides may be administered to a subject to induce the immune system of the subject to produce anti-Replikin antibodies. Generally, a 0.5 to about 2 mg dosage, preferably a 1 mg dosage of each peptide is administered to the subject to induce an immune response. Subsequent dosages may be administered if desired.

In another embodiment of the invention, isolated H₁N₁ influenza Replikin peptides may be used to generate antibodies, which may be used, for example to provide passive immunity in an individual. Passive immunity to the strain of influenza identified by the method of the invention to be the most likely cause of future influenza infections may be obtained by administering antibodies to Replikin sequences of the identified strain of influenza virus to patients in need.

Various procedures known in the art may be used for the production of antibodies to Replikin sequences. Such antibodies include but are not limited to polyclonal, monoclonal, chimeric, humanized, single chain, Fab fragments and fragments produced by an Fab expression library. Antibodies that are linked to a cytotoxic agent may also be generated. Furthermore, combinations of antibodies to different Replikins may be administered as an antibody cocktail.

For the production of antibodies various host animals may be immunized by injection with a Replikin peptide or a combination of Replikin peptides, including but not limited to rabbits, mice, rats, and larger mammals. Various adjuvants may be used to enhance the immunological response, depending on the host species, including but not limited to Freund's (complete and incomplete), mineral gels, such as aluminum hydroxide, surface active substances such as lysolecithin, pluronic polyols, polyanions, peptides, oil emulsions, key limpet hemocyanin, dintrophenol, and potentially useful human adjuvants such as BCG and Corynebacterium *parvum.*

Monoclonal antibodies to H₁N₁ influenza Replikins may be prepared by using any technique that provides for the production of antibody molecules. These include but are not limited to the hybridoma technique originally described by Kohler and Milstein, (Nature,1975, 256:495-497), the human B-cell hybridoma technique (Kosbor et al., 1983, Immunology Today, 4:72), and the EBV hybridoma technique (Cole et al., Monoclonal Antibodies and Cancer Therapy, Alan R. Liss, Inc., pp. 77-96). In addition, techniques developed for the production of chimeric antibodies (Morrison et al., 1984, Proc. Nat. Acad. Sci USA, 81:6851-6855) or other techniques may be used. Alternatively, techniques described for the production of single chain antibodies (US 4,946,778) can be adapted to produce Replikin-specific single chain antibodies.

, Particularly useful antibodies of the invention are those that specifically bind to H₁N₁ influenza Replikin sequences contained in peptides and/or polypeptides of influenza virus. For example, antibodies to any of peptides observed to be present in an emerging or re-emerging strain of influenza virus and combinations of such antibodies are useful in the treatment and/or prevention of influenza.

Antibody fragments which contain binding sites for a Replikin may be generated by known techniques. For example, such fragments include but are not limited to F(ab')₂ fragments which can be produced by pepsin digestion of the antibody molecules and the Fab fragments that can be generated by reducing the disulfide bridges of the F(ab')₂ fragments. Alternatively, Fab expression libraries can be generated (Huse et al., 1989, Science, 246:1275-1281) to allow rapid and easy identification of monoclonal Fab fragments with the desired specificity.

The fact that antimalignin antibody is increased in concentration in human malignancy regardless of cancer cell type (Figure 5), and that this antibody binds to malignant cells regardless of cell type now may be explained by the presence of the Replikin structures herein found to be present in most malignancies - (**Figure 1** and Table 2). Population studies have shown that antimalignin antibody increases in concentration in healthy adults with age, and more so in high-risk families, as the frequency of cancer increases. An additional two-fold or greater antibody increase which occurs in early malignancy has been independently confirmed with a sensitivity of 97% in breast cancers 1-10 mm in size. Shown to localize preferentially in malignant cells *in vivo,* histochemically the antibody does not bind to normal cells but selectively binds to (**Figure 4a****,b**) and is highly cytotoxic to transformed cells *in vitro* (**Figure 4c****-f**). Since in these examples the same antibody is bound by several cell types, that is, brain glioma, hematopoietic cells (leukemia), and small cell carcinoma of lung, malignant Replikin class unity is again demonstrated.

Antimalignin does not increase with benign proliferation, but specifically increases only with malignant transformation and replication in breast *in vivo* and returns from elevated to normal values upon elimination of malignant cells (Figure 5). Antimalignin antibody concentration has been shown to relate quantitatively to the survival of cancer patients, that is, the more antibody, the longer the survival. Taken together, these results suggest that anti-Replikin antibodies may be a part of a mechanism of control of cell transformation and replication.

The Replikin sequence structure is associated with the function of replication. Thus, whether the H₁N₁ influenza Replikins of this invention are used for targeting sequences that contain Replikins for the purpose of diagnostic identification, promoting replication, or inhibiting or attacking replication, for example, the structure-function relationship of the Replikin is fundamental. Thus, while the structure of the Replikin may be a part of a larger protein sequence, which may have been previously identified, it is preferable to utilize only the specific Replikin structure when seeking to induce antibodies that will recognize and attach to the Replikin fragment and thereby cause destruction of the cell. Even though the larger protein sequence may be known in the art as having a "replication associated function," vaccines using the larger protein often have failed or proven ineffective, even though they contain one or more Replikin sequences.

Although the present inventors do not wish to be held to a single theory, the studies herein suggest that the prior art vaccines are ineffective because they are based on the use of the larger protein sequence. The larger protein sequence invariably has one or more epitopes (independent antigenic sequences that can induce specific antibody formation); Replikin structures usually comprise one of these potential epitopes. The presence of other epitopes within the larger protein may interfere with adequate formation of antibodies to the Replikin, by "flooding" the immune system with irrelevant antigenic stimuli which may preempt the Replikin antigens, *See*, e.g., Webster, R.G., J. Immunol., 97(2):177-183 (1966); and Webster et al., J. Infect. Dis., 134:48-58, 1976; Klenerman et al, Nature 394:421-422 (1998) for a discussion of the well-known phenomenon "original antigenic sin"). The fonnation of an antibody to a non-Replikin epitope may allow binding to the cell, but not necessarily lead to cell destruction. The presence of structural "decoys" on the C-termini of malaria proteins is another aspect of this ability of other epitopes to interfere with binding of effective anti-Replikin antibodies, since the decoy epitopes have many lysine residues, but no histidine residues. Thus, decoy epitopes may bind anti-Replikin antibodies, but keep the antibodies away from histidine - bound respiratory enzymes.

It is well known in the art that in the course of antibody production against a "foreign" protein, the protein is first hydrolyzed into smaller fragments. Usually fragments containing from about six to ten amino acids are selected for antibody formation. Thus, if hydrolysis of a protein does not result in Replikin-containing fragments, anti-Replikin antibodies will not be produced. In this regard, it is interesting that Replikins contain lysine residues located six to ten amino acids apart, since lysine residues are known to bind to membranes.

Furthermore, Replikin sequences contain at least one histidine residue. Histidine is frequently involved in binding to redox centers. Thus, an antibody that specifically recognizes a Replikin sequence has a better chance of inactivating or destroying the cell in which the Replikin is located, as seen with anti-malignin antibody, which is perhaps the most cytotoxic antibody yet described, being active at picograms per cell.

One of the reasons that vaccines directed towards a particular protein antigen of a disease causing agent have not been fully effective in providing protection against the disease (such as foot and mouth vaccine which has been developed against the VP1 protein or large segments of the VP1 protein) is that antibody to the Replikins have not been produced. That is, either epitopes other than Replikins present in the larger protein fragments may interfere according to the phenomenon of "original antigenic sin", and/or because the hydrolysis of larger protein sequences into smaller sequences for processing to produce antibodies results in loss of integrity of any Replikin structure that is present, e.g., the Replikin is cut in two and/or the histidine residue is lost in the hydrolytic processing. The present studies suggest that for an effective vaccine to be produced, the Replikin sequences, and no other epitope, should be used as the vaccine. For example, a H₁N₁ influenza vaccine of the invention can be generated using any one of the Replikin peptides identified by the three point recognition system. Particularly preferred peptides for an H₁N₁ influenza vaccine include peptides that have been demonstrated to be conserved over a period of one or more years, preferably about three years or more. Among the preferred Replikin peptides for use in an influenza virus vaccine are those replikins observed to "re-emerge" after an absence from the hemagglutinin amino acid sequence for one or more years.

The H₁N₁ influenza Replikin peptides of the invention, alone or in various combinations are administered to a subject, preferably by i.v. or intramuscular injection, in order to stimulate the immune system of the subject to produce antibodies to the peptide. Generally the dosage of peptides is in the range of from about 0.1 µg to about 10 mg, preferably about 10 *g to about 1 mg, and most preferably about 50 µg to about 500 ug. The skilled practitioner can readily determine the dosage and number of dosages needed to produce an effective immune response.

Replikin DNA or RNA may have a number of uses for the diagnosis of diseases resulting from infection with a H₁N₁ influenza virus. For example, H₁N₁ influenza Replikin nucleotide sequences may be used in hybridization assays of biopsied tissue or blood, e.g., Southern or Northern analysis, including *in situ* hybridization assays, to diagnose the presence of a particular organism in a tissue sample or an environmental sample, for example. The present invention also contemplates kits containing antibodies specific for particular H₁N₁ influenza Replikins, or containing nucleic acid molecules (sense or antisense) that hybridize specifically to a particular H₁N₁ influenza Replikin, and optionally, various buffers and/or reagents needed for diagnosis.

Also within the scope of the invention are oligoribonucleotide sequences, that include antisense RNA and DNA molecules and ribozymes that function to inhibit the translation of H₁N₁ influenza Replikin- containing mRNA Both antisense RNA and DNA molecules and ribozymes may be prepared by any method known in the art. The antisense molecules can be incorporated into a wide variety of vectors for delivery to a subject. The skilled practitioner can readily determine the best route of delivery, although generally i.v. or i.m. delivery is routine. The dosage amount is also readily ascertainable.

Particularly preferred antisense nucleic acid molecules are those that are complementary to a Replikins sequence contained in a mRNA encoding an H₁N₁ influenza virus polypeptide, wherein the Replikin sequence comprises from 7 to about 50 amino acids including (1) at least one lysine residue located six to ten residues from a second lysine residue; (2) at least one histidine residue; and (3) at least 6% lysine residues. More preferred are antisense nucleic acid molecules that are complementary to a Replikin present in the coding strand of the gene or to the mRNA encoding the influenza virus hemagglutinin protein, wherein the antisense nucleic acid molecule is complementary to a nucleotide sequence encoding a Replikin that has been demonstrated to be conserved over a period of six months to one or more years and/or which are present in a strain of H₁N₁ influenza virus shown to have an increase in concentration of Replikins relative to Replikin concentration in other H₁N₁ influenza virus strains. The increase in Replikin concentration preferably occurs over a period of at least six months, preferably about one year, most preferably about two or three years or more.

Immune serum containing antibodies to one or more H₁N₁ influenza Replikins obtained from an individual exposed to one or more H₁N₁ influenza Replikins may be used to induce passive immunity in another individual or animal. Immune serum may be administered *via* i.v. to a subject in need of treatment. Passive immunity also can be achieved by injecting a recipient with preformed antibodies to one or more H₁N₁ influenza Replikins. Passive immunization may be used to provide immediate protection to individuals who have been exposed to an infectious organism. Administration of immune serum or preformed antibodies is routine and the skilled practitioner can readily ascertain the amount of serum or antibodies needed to achieve the desired effect.

From a proteomic point of view the construction of a "3-point - recognition" template based on the new glioma peptide sequence led directly to identification of a biology-wide class of proteins having related structures and functions. The operation of the 3-point-recognition method resembles identification by the use of a "keyword"search; but instead of using the exact spelling of the keyword "kagvaflhkk" (**SEQ ID NO.: 1**) as in a typical sequence homology search, or in the nucleotide specification of an amino acid, an abstraction of the keyword delimited by the "3-point-recognition" parameters is used. This delimited abstraction, although derived from a single relatively short amino acid sequence leads to identification of a class of proteins with structures that are defined by the same specifications. That particular functions, in this case transformation and replication, in addition to structures, turn out also to be shared by members of the exposed class suggests that these structures and functions are related. Thus, from this newly identified short peptide sequence, a molecular recognition 'language' has been formulated, which previously has not been described. Further, the sharing of immunological specificity by diverse members of the class, as here demonstrated for the cancer Replikins, suggests that B cells and their product antibodies recognize Replikins by means of a similar recognition language.

Several embodiments of the present invention are specifically illustrated and described herein. However, it will be appreciated that modifications and variations of the present invention are encompassed by the above teachings and within the purview of the appended claims without departing from intended scope of the invention.

### EXAMPLE 1

### PROCESS FOR EXTRACTION, ISOLATION AND IDENTIFICATION OF REPLIKINS AND THE USE OF REPLIKINS TO TARGET, LABEL OR DESTROY REPLIKlN-CONTAINING ORGANISMS

### a) Algae

The following algae were collected from Bermuda water sites and either extracted on the same day or frozen at -20 degrees C and extracted the next day. The algae were homogenized in a cold room (at 0 to 5 degrees C) in 1 gram aliquots in neutral buffer, for example 100 cc. of 0.005M phosphate buffer solution, pH 7 ("phosphate buffer") for 15 minutes in a Waring blender, centrifuged at 3000 rpm, and the supernatant concentrated by perevaporation and dialyzed against phosphate buffer in the cold to produce a volume of approximately 15 ml. The volume of this extract solution was noted and an aliquot taken for protein analysis, and the remainder was fractionated to obtain the protein fraction having a pK range between 1 and 4. The preferred method of fractionation is chromatography as follows:

The extract solution is fractionated in the cold room (4 degrees C) on a DEAE cellulose (Cellex-D) column 2.5x11.0 cm, which has been equilibrated with 0.005M phosphate buffer. Stepwise eluting solvent changes are made with the following solutions:
Solution 1- 4.04 g. NaH2P04 and 0.5g NaH2P04 are dissolved in 15 litres of distilled water (0.005 molar, pH 7);
Solution 2 - 8.57 g. NaH2P04 is dissolved in 2,480 ml. of distilled water;
Solution 3 - 17.1 g. of NaH2P04 is dissolved in 2480 ml of distilled water (0.05 molar, pH 4.7);
Solution 4 - 59.65 g. of NaH2P04 is dissolved in 2470 ml distilled water (0.175 molar);
Solution 5 - 101.6 g. of NaH2PO4 is dissolved in 2455 ml distilled water (pH 4.3);
Solution 6 - 340.2 g. of NaH2PO4 is dissolved in 2465 of distilled water (1.0 molar, pX-i 4.1);
Solution 7 - 283.63 g. of 80% phosphoric acid (H3PO4) is made up in 2460 ml of distilled water (1.0 molar, pH 1.0).

The extract solution, in 6 to 10 ml volume, is passed onto the column and overlayed with Solution 1, and a reservoir of 300 ml of Solution 1 is attached and allowed to drip by gravity onto the column. Three ml aliquots of eluant are collected and analyzed for protein content at OD 280 until all of the protein to be removed with Solution 1 has been removed from the column. Solution 2 is then applied to the column, followed in succession by Solutions 3, 4, 5, 6 aid 7 until all of the protein which can, be removed with each Solution is removed from the column. The eluates from Solution 7 are combined, dialyzed against phosphate buffer, the protein content determined of both dialysand and dialyzate, and both analyzed by gel electrophoresis. One or two bands of peptide or protein of molecular weight between 3,000 and 25,000 Daltons are obtained in Solution 7. For example the algae *Caulerpa mexicana, Laurencia obtura, Cladophexa prolifera, Sargassum natans, Caulerpa verticillata, Halimeda tuna,* and *Penicillos capitatus,* after extraction and treatment as above, all demonstrated in Solution 7 eluates sharp peptide bands in this molecular weight region with no contaminants. These Solution 7 proteins or their eluted bands are hydrolyzed, and the amino acid composition determined. The peptides so obtained, which have a lysine composition of 6% or greater are Replikin precursors. These Replikin peptide precursors are then determined for amino acid sequence and the replikins are determined by hydrolysis and mass spectrometry as detailed in U.S. patent 6,242,578 B1. Those which fulfill the criteria defined by the " 3-point-recognition" method are identified as Replikins. This procedure can also be applied to obtain yeast, bacterial and any plant Replikins.

### b) Virus

Using the same extraction and column chromatography separation methods as above in a) for algae, Replikens in virus-infected cells are isolated and identified.

### c) Tumor cells in vivo and in vitro tissue culture

Using the same extraction and column chromatography separation methods as above in a) for algae, Replikins in tumor cells are isolated and identified. For example, Replikin precursors of Astrocytin isolated from malignant brain tumors, Malignin (Aglyco 1OB) isolated from glioblastoma tumor cells in tissue cu0lture, MCF7 mammary carcinoma cells in tissue culture, and P₃J Lymphoma cells in tissue culture each treated as above in a) yielded Replikin precursors with lysine content of 9.1%, 6.7%, 6.7%, and 6.5% respectively. Hydrolysis and mass spectrometry of Aglyco 1OB as described in Example 10 U.S. 6,242,578 B1 produced the amino acid sequence, ykagvaflhkkndiide the 16-mer Replikin.

### EXAMPLE 2:

As an example of diagnostic use of Replikins: Aglyco 1OB or the 16-mer Repliken may be used as antigen to capture and quantify the amount of its corresponding antibody present in serum for diagnostic purposes are as shown in Figures 2,3,4 and 7 of U.S. 6,242,578 B1.

As an example of the production of agents to attach to Replikins for labeling, nutritional or destructive purposes: Injection of the 16-mer Replikin into rabbits to produce the specific antibody to the 16-mer Replikin is shown in Example 6 and Figures 9A and 9B of U.S. 6,242,578 B1.

As an example of the use of agents to label Replikins: The use of antibodies to the 16-mer Replikin to label specific cells which contain this Replikin is shown in Figure 5 and Example 6 of U.S. 6,242,578 B1.

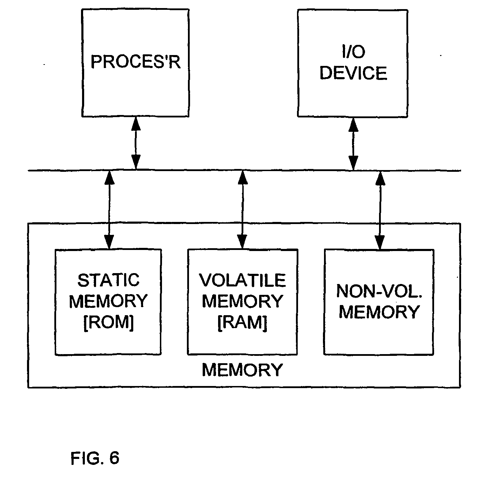

As an example of the use of agents to destroy Replikins: The use of antibodies to the 16-mer Replikin to inhibit or destroy specific cells which contain this Replikin is shown in Figure 6 of U.S. 6,242,578 B1.

### EXAMPLE 3

Analysis of sequence data of isolates of influenza virus hemagglutinin protein or neuraminidase protein for the presence and concentration of Replikins is carried out by visual scanning of sequences or through use of a computer program based on the 3-point recognition system described herein. Isolates of influenza virus are obtained and the amino acid sequence of the influenza hemagglutinin and/or neuraminidase protein is obtained by any art known method, such as by sequencing the hemagglutinin or neuraminidase gene and deriving the protein sequence therefrom. Sequences are scanned for the presence of new Replikins, conservation of Replikins over time and concentration of Replikins in each isolate. Comparison of the Replikin sequences and concentrations to the amino acid sequences obtained from isolates at an earlier time, such as about six months to about three years earlier, provides data that are used to predict the emergence of strains that are most likely to be the cause of influenza in upcoming flu seasons, and that form the basis for seasonal influenza peptide vaccines or nucleic acid based vaccines. Observation of an increase in concentration, particularly a stepwise increase in concentration of Replikins in a given strain of influenza virus for a period of about six months to about three years or more is a predictor of emergence of the strain as a likely cause of influenza epidemic or pandemic in the future.

Peptide vaccines or nucleic acid-based vaccines based on the Replikins observed in the emerging strain are generated. An emerging strain is identified as the strain of influenza virus having the highest increase in concentration of replikin sequences within the hemagglutinin and/or neuraminidase sequence during the time period. Preferably, the peptide or nucleic acid vaccine is based on or includes any Replikin sequences that are observed to be conserved in the emerging strain. Conserved replikins are preferably those Replikin sequences which are present in the hemagglutinin or neuraminidase protein sequence for about two years and preferably longer. The vaccines may include any combination of Replikin sequences identified in the emerging strain.

For vaccine production, the Replikin peptide or peptides identified as useful for an effective vaccine are synthesized by any method, including chemical synthesis and molecular biology techniques, including cloning, expression in a host cell and purification therefrom. The peptides are preferably admixed with a pharmaceutically acceptable carrier in an amount determined to induce a therapeutic antibody reaction thereto. Generally, the dosage is about 0.1 µg to about 10 mg.

The influenza vaccine is preferably administered to a patient in need thereof prior to the onset of "flu season." Influenza flu season generally occurs in late October and lasts through late April. However, the vaccine may be administered at any time during the year. Preferably, the influenza vaccine is administered once yearly, and is based on Replikin sequences observed to be present, and preferably conserved in the emerging strain of influenza virus. Another preferred Replikin for inclusion in an influenza vaccine is a Replikin demonstrated to have re-emerged in a strain of influenza after an absence of one or more years.

### EXAMPLE 4

Analysis of sequence data of isolates of Plasmodium falciparum antigens for the presence and concentration of Replikins is carried out by visual scanning of sequences or through use of a computer program based on the 3-point recognition system described herein. Isolates of Plasmodium falciparum are obtained and the amino acid sequence of the protein is obtained by any art known method, such as by sequencing the gene and deriving the protein sequence therefrom. Sequences are scanned for the presence of Replikins, conservation ofReplikins over time and concentration of Replikins in each isolate. This information provides data that are used to form the basis for anti-malarial peptide vaccines or nucleic acid based vaccines.

Peptide vaccines or nucleic acid-based vaccines based on the Replikins observed in the malaria causing organism are generated. Preferably, the peptide or nucleic acid vaccine is based on or includes any Replikin sequences that are observed to be present on a surface antigen of the organism. The vaccines may include any combination of Replikin sequences identified in the malaria causing strain.

For vaccine production, the Replikin peptide or peptides identified as useful for an effective vaccine are synthesized by any method, including chemical synthesis and molecular biology techniques, including cloning, expression in a host cell and purification therefrom. The peptides are preferably admixed with a pharmaceutically acceptable carrier in an amount determined to induce a therapeutic antibody reaction thereto. Generally, the dosage is about 0.1 µg to about 10 mg.

Then malaria vaccine is preferably administered to a patient in need thereof at any time during the year, and particularly prior to travel to a tropical environment.

## Claims

1. An isolated or synthesized H1N1 influenza virus Replikin peptide from a hemagglutinin protein wherein said peptide consists of 7 to 50 amino acids comprising (1) at least one lysine residue located six to ten residues from a second lysine residue; (2) at least one histidine residue; and (3) at least 6% lysine residues.

2. The isolated or synthesized H1N1 influenza virus Replikin peptide of claim 1 consisting of any one of the amino acid sequences of SEQ ID NO(s): 135-179, 181-207, and 209-229.

3. An antibody that specifically binds to the influenza virus peptide of claim 1 or claim 2.

4. A composition comprising the antibody of claim 3 and a pharmaceutically acceptable carrier and/or adjuvant.

5. A therapeutic composition comprising the isolated or synthesized H1N1 influenza virus Replikin peptide of claim 1 or claim 2 and a pharmaceutically acceptable carrier and/or adjuvant.

6. The therapeutic composition of claim 5 wherein the peptide is conserved in a strain of influenza virus for at least two consecutive years including the current year.

7. A therapeutic composition comprising a plurality of isolated or synthesized influenza virus peptides of claim 1 or claim 2 and a pharmaceutically acceptable carrier.

8. The therapeutic composition of claim 7 wherein at least one of the plurality of influenza virus peptides is conserved in the influenza virus hemagglutinin amino acid sequence for at least two consecutive years including the current year.

9. An antisense nucleic acid molecule complementary to an influenza virus hemagglutinin Replikin mRNA sequence encoding the influenza virus Replikin peptide of claim 1 or claim 2.

10. An antisense nucleic acid molecule complementary to the coding strand of the gene or the mRNA encoding influenza virus hemagglutinin, wherein said antisense nucleic acid molecule is complementary to a nucleotide sequence present in an emerging strain of influenza virus and wherein said nucleotide sequence encodes the influenza virus Replikin peptide of claim 1 or claim 2.

11. A method of making an influenza virus vaccine comprising (1) identifying a strain of influenza virus as an emerging strain, (2) selecting at least one H1N1 influenza virus Replikin peptide of claim 1 or claim 2 present in the emerging strain as a peptide template for influenza virus vaccine manufacture, (3) synthesizing peptides having the amino acid sequence of the at least one H1N1 influenza virus Replikin peptide selected in step (2), and (4) combining a therapeutically effective amount of the peptides of step (3) with a pharmaceutically acceptable carrier and/or adjuvant.

12. The method of claim 11 wherein the at least one Replikin peptide selected in step (2) is conserved in the influenza virus hemagglutinin sequence for at least two consecutive years, including the current year.

13. An influenza virus vaccine comprising at least one isolated or synthesized Replikin peptide of claim 1 or claim 2 and a pharmaceutically acceptable carrier and/or adjuvant.

14. The influenza virus vaccine of claim 13 wherein the vaccine comprises a plurality of isolated or synthesized Replikin peptides of claim 1 or claim 2.

15. The vaccine of claim 13 wherein the at least one isolated or synthesized Replikin peptide is conserved for at least two consecutive years in an emerging strain of influenza virus.

## Patentansprüche

1. Isoliertes oder synthetisiertes H1N1-Influenzavirus-Replikin-Peptid von einem Hämagglutininprotein, wobei das Peptid aus 7 bis 50 Aminosäuren besteht, umfassend (1) mindestens einen Lysinrest, der sechs bis zehn Reste von einem zweiten Lysinrest lokalisiert ist; (2) mindestens einen Histidinrest und (3) mindestens 6% Lysinreste.

2. Isoliertes oder synthetisiertes H1N1- Influenzavirus -Replikin-Peptid nach Anspruch 1, bestehend aus einer beliebigen der Aminosäuresequenzen von SEQ ID NO: 135-179, 181-207 und 209-229.

3. Antikörper, der sich spezifisch an das Influenzavirus-Peptid nach Anspruch 1 oder Anspruch 2 bindet.

4. Zusammensetzung, umfassend den Antikörper nach Anspruch 3 und einen pharmazeutisch verträglichen Träger und/oder Hilfsstoff.

5. Therapeutische Zusammensetzung, umfassend das isolierte oder synthetisierte H1N1-Influenzavirus-Replikin-Peptid nach Anspruch 1 oder Anspruch 2 und einen pharmazeutisch verträglichen Träger und/oder Hilfsstoff.

6. Therapeutische Zusammensetzung nach Anspruch 5, wobei das Peptid mindestens zwei aufeinander folgende Jahre lang, einschließlich des laufenden Jahres, in einem Influenzavirusstamm konserviert ist.

7. Therapeutische Zusammensetzung, umfassend eine Mehrzahl an isolierten oder synthetisierten Influenzavirus-Peptiden nach Anspruch 1 oder Anspruch 2 und einen pharmazeutisch verträglichen Träger.

8. Therapeutische Zusammensetzung nach Anspruch 7, wobei mindestens eines der Mehrzahl an Influenzavirus-Peptiden mindestens zwei aufeinander folgende Jahre lang, einschließlich des laufenden Jahres, in der Influenzavirus-Hämagglutinin-Aminosäuresequenz konserviert ist.

9. Antisense-Nukleinsäuremolekül, das zu einer das Influenzavirus-Replikin-Peptid nach Anspruch 1 oder Anspruch 2 kodierenden Influenzavirus-Hämagglutinin-Replikin-mRNA-Sequenz komplementär ist.

10. Antisense-Nukleinsäuremolekül, das zu dem kodierenden Strang des Gens oder der mRNA, kodierend Influenzavirus-Hämagglutinin, komplementär ist, wobei das Antisense-Nuldeinsäuremolekül zu einer Nukleotidsequenz komplementär ist, die in einem auftretenden Influenzavirusstamm vorliegt, und wobei die Nuldeotidsequenz das Influenzavirus-Replikin-Peptid nach Anspruch 1 oder Anspruch 2 kodiert.

11. Verfahren zur Herstellung eines Influenzavirusimpfstoffs, umfassend (1) Identifizieren eines Influenzavirusstamms als auftretenden Stamm, (2) Auswählen mindestens eines H1N1-Influenzavirus-Replikin-Peptids nach Anspruch 1 oder Anspruch 2, das in dem auftretenden Stamm vorliegt, als Peptidvorlage zur Herstellung eines Influenzavirusimpfstoffs, (3) Synthetisieren von Peptiden mit der Aminosäuresequenz des mindestens einen in Schritt (2) ausgewählten H1N1-Influenzavirus-Replikin-Peptids und (4) Vereinigen einer therapeutisch wirksamen Menge der Peptide von Schritt (3) mit einem pharmazeutisch verträglichen Träger und/oder Hilfsstoff.

12. Verfahren nach Anspruch 11, wobei das mindestens eine in Schritt (2) ausgewählte Replikin-Peptid mindestens zwei aufeinander folgende Jahre lang, einschließlich des laufenden Jahres, in der Influenzavirus-Hämagglutinin-Sequenz konserviert ist.

13. Influenzavirusimpfstoff, umfassend mindestens ein isoliertes oder synthetisiertes Replikin-Peptid nach Anspruch 1 oder Anspruch 2 und einen pharmazeutisch verträglichen Träger und/oder Hilfsstoff.

14. Influenzavirusimpfstoff nach Anspruch 13, wobei der Impfstoff eine Mehrzahl an isolierten oder synthetisierten Replikin-Peptiden nach Anspruch 1 oder Anspruch 2 umfasst.

15. Impfstoff nach Anspruch 13, wobei das mindestens eine isolierte oder synthetisierte Replikin-Peptid mindestens zwei aufeinander folgende Jahre lang in einem auftretenden Influenzavirusstamm konserviert ist.

## Revendications

1. Peptide réplikine du influenzavirus H1N1, isolé ou synthétisé, provenant d'une protéine hémagglutinine, où ledit peptide consiste en 7 à 50 acides aminés comprenant (1) au moins un résidu de lysine situé six à dix résidus à partir d'un deuxième résidu de lysine ; (2) au moins un résidu d'histidine ; et (3) au moins 6 % de résidus de lysine.

2. Peptide réplikine du influenzavirus H1N1, isolé ou synthétisé, selon la revendication 1, consistant en l'une quelconque des séquences d'acides aminés SEQ ID N° 135-179, 181-207 et 209-229.

3. Anticorps qui se lie d'une manière spécifique au peptide du influenzavirus selon la revendication 1 ou 2.

4. Composition comprenant l'anticorps de la revendication 3, et un support et/ou un adjuvant pharmaceutiquement acceptable.

5. Composition thérapeutique comprenant le peptide réplikine du influenzavirus H1N1, isolé ou synthétisé, de la revendication 1 ou de la revendication 2, et un support et/ou un adjuvant pharmaceutiquement acceptable.

6. Composition thérapeutique de la revendication 5, dans laquelle le peptide est conservé dans une souche du influenzavirus pendant au moins deux années consécutives, y compris l'année en cours.

7. Composition thérapeutique comprenant une pluralité de peptides du influenzavirus isolés ou synthétisés de la revendication 1 ou de la revendication 2 et un support pharmaceutiquement acceptable.

8. Composition thérapeutique de la revendication 7, dans laquelle au moins l'un de la pluralité de peptides du influenzavirus est conservé dans la séquence d'acides aminés de l'hémagglutinine du influenzavirus pendant au moins deux années consécutives, y compris l'année en cours.

9. Molécule d'acide nucléique antisens, complémentaire d'une séquence d'ARNm réplikine de l'hémagglutinine du influenzavirus, codant pour le peptide réplikine du influenzavirus de la revendication 1 ou de la revendication 2.

10. Molécule d'acide nucléique antisens, complémentaire du brin codant du gène de l'ARNm codant pour l'hémagglutinine du influenzavirus, la molécule d'acide nucléique antisens étant complémentaire d'une séquence nucléotidique présente dans une souche émergente du influenzavirus, ladite séquence nucléotidique codant pour le peptide réplikine du influenzavirus de la revendication 1 ou de la revendication 2.

11. Procédé de fabrication d'un vaccin contre le virus de la grippe, comprenant (1) l'identification d'une souche de influenzavirus comme étant une souche émergente, (2) la sélection d'au moins un peptide réplikine du influenzavirus H1N1 de la revendication 1 ou 2, présent dans la souche émergente en tant que matrice peptidique pour la fabrication d'un vaccin contre le influenzavirus, (3) la synthèse de peptides ayant la séquence d'acides aminés d'au moins un peptide réplikine du influenzavirus H1N1 choisi dans l'étape (2), et (4) la combinaison d'une quantité thérapeutiquement efficace de peptides de l'étape (3) avec un support et/ou un adjuvant pharmaceutiquement acceptables.

12. Procédé de la revendication 11, dans lequel l'au moins un peptide réplikine choisi dans l'étape (2) est conservé dans la séquence d'hémagglutinine du influenzavirus pendant au moins deux années consécutives, y compris l'année en cours.

13. Vaccin contre le influenzavirus, comprenant au moins un peptide réplikine isolé ou synthétisé de la revendication 1 ou 2 et un support et/ou un adjuvant pharmaceutiquement acceptables.

14. Vaccin contre le influenzavirus de la revendication 13, le vaccin comprenant une pluralité de peptides réplikine isolés ou synthétisés de la revendication 1 ou de la revendication 2.

15. Vaccin de la revendication 13, dans lequel l'au moins un peptide réplikine isolé ou synthétisé est conservé pendant au moins deux années consécutives dans une souche émergente du influenzavirus.
